# EUROPEAN PATENT SPECIFICATION

(11) **EP 2 970 260 B1**
(45) Date of publication and mention of the grant of the patent: **24.04.2019**
(21) Application number: 14711578.6
(22) Date of filing: 13.03.2014
(51) Int. Cl.: C07D 471/04, A61K 31/437, A61P 29/00, A61P 35/00, A61P 37/00

(54) **IMIDAZO[4,5-C]PYRIDINE AND PYRROLO[2,3-C]PYRIDINE DERIVATIVES AS SSAO INHIBITORS**
IMIDAZO-[4,5-C]PYRIDIN- UND PYRROLO-[2,3-C]PYRIDINDERIVATE ALS SSAO-HEMMER
DÉRIVÉS D'IMIDAZO[4,5-C]PYRIDINE ET DE PYRROLO[2,3-C]PYRIDINE EN TANT QU'INHIBITEURS SSAO

(30) Priority: 13.03.2013 GB 201304526
(43) Date of publication of application: 20.01.2016
(73) Proprietor: PROXIMAGEN, LLC, Plymouth, MN 55441 (US)
(72) Inventor: ESPENSEN, Max, Plymouth, MN 55441 (US); PATIENT, Lee, Plymouth, MN 55441 (US); EVANS, David, Plymouth, MN 55441 (US); SIMPSON, Iain, Plymouth, MN 55441 (US); SAVORY, Edward, Suite 850 (US)
(74) Representative: Gill Jennings & Every LLP
(86) International application number: PCT/GB2014/050765
(87) International publication number: WO 2014/140592

(56) References cited:
- WO-A1-2013/078254
- US-A1- 2005 054 631
- DATABASE REGISTRY [Online] CHEMICAL ABSTRACTS SERVICE, COLUMBUS, OHIO, US; 19 January 2011 (2011-01-19), XP002723294, Database accession no. 1259952-23-2
- FERDINAND S. MELKONYAN ET AL: "One-pot synthesis of substituted indoles via titanium(iv) alkoxide mediated imine formation - copper-catalyzed N-arylation", RSC ADVANCES, vol. 3, no. 22, 21 March 2013 (2013-03-21) , page 8388, XP055113497, ISSN: 2046-2069, DOI: 10.1039/c3ra40389k
- ROBERT J. WILSON ET AL: "Copper- and Palladium-Catalyzed Amidation Reactions for the Synthesis of Substituted Imidazo[4,5- c ]pyridines", THE JOURNAL OF ORGANIC CHEMISTRY, vol. 79, no. 5, 6 February 2014 (2014-02-06), pages 2203-2212, XP055113503, ISSN: 0022-3263, DOI: 10.1021/jo500064j

## Description

### FIELD OF THE INVENTION

The present invention relates to compounds which are inhibitors of SSAO activity. The invention also relates to pharmaceutical compositions comprising these compounds and to the use of these compounds in the treatment or prevention of medical conditions wherein inhibition of SSAO activity is beneficial, such as inflammatory diseases, immune disorders and the inhibition of tumour growth.

### BACKGROUND ART

Semicarbazide-sensitive amine oxidase (SSAO) activity is an enzyme activity expressed by Vascular Adhesion Protein-1 (VAP-1) or Amine Oxidase, Copper Containing 3 (AOC3), belongs to the copper-containing amine oxidase family of enzymes (EC.1.4.3.6). Therefore inhibitors of the SSAO enzyme may also modulate the biological functions of the VAP-1 protein. Members of this enzyme family are sensitive to inhibition by semicarbazide and utilize cupric ion and protein-derived topa quinone (TPQ) cofactor in the oxidative deamination of primary amines to aldehydes, hydrogen peroxide, and ammonia according to the following reaction:

R-CH₂-NH₂ + O₂ → R-CHO + H₂O₂ + NH₃

Known substrates for human SSAO include endogenous methylamine and aminoacetone as well as some xenobiotic amines such as benzylamine [Lyles, Int. J. Biochem. Cell Biol. 1996, 28, 259-274; Klinman, Biochim. Biophys. Acta 2003, 1647(1-2), 131-137; Mátyus et al., Curr. Med. Chem. 2004, 11(10), 1285-1298; O'Sullivan et al., Neurotoxicology 2004, 25(1-2), 303-315]. In analogy with other copper-containing amine oxidases, DNA-sequence analysis and structure determination suggest that the tissue-bound human SSAO is a homodimeric glycoprotein consisting of two 90-100 kDa subunits anchored to the plasma membrane by a single N-terminal membrane spanning domain [Morris et al., J. Biol. Chem. 1997, 272, 9388-9392; Smith et al., J. Exp. Med. 1998, 188, 17-27; Airenne et al., Protein Science 2005, 14, 1964-1974; Jakobsson et al., Acta Crystallogr. D Biol. Crystallogr. 2005, 61(Pt 11), 1550-1562].

SSAO activity has been found in a variety of tissues including vascular and non-vascular smooth muscle tissue, endothelium, and adipose tissue [Lewinsohn, Braz. J. Med. Biol. Res. 1984, 17, 223-256; Nakos & Gossrau, Folia Histochem. Cytobiol. 1994, 32, 3-10; Yu et al., Biochem. Pharmacol. 1994, 47, 1055-1059; Castillo et al., Neurochem. Int. 1998, 33, 415-423; Lyles & Pino, J. Neural. Transm. Suppl. 1998, 52, 239-250; Jaakkola et al., Am. J. Pathol. 1999, 155, 1953-1965; Morin et al., J. Pharmacol. Exp. Ther. 2001, 297, 563-572; Salmi & Jalkanen, Trends Immunol. 2001, 22, 211-216]. In addition, SSAO protein is found in blood plasma and this soluble form appears to have similar properties as the tissue-bound form [Yu et al., Biochem. Pharmacol. 1994, 47, 1055-1059; Kurkijärvi et al., J. Immunol. 1998, 161, 1549-1557]. It has recently been shown that circulating human and rodent SSAO originates from the tissue-bound form [Göktürk et al., Am. J. Pathol. 2003, 163(5), 1921-1928; Abella et al., Diabetologia 2004, 47(3), 429-438; Stolen et al., Circ. Res. 2004, 95(1), 50-57], whereas in other mammals the plasma/serum SSAO is also encoded by a separate gene called AOC4 [Schwelberger, J. Neural. Transm. 2007, 114(6), 757-762].

The precise physiological role of this abundant enzyme has yet to be fully determined, but it appears that SSAO and its reaction products may have several functions in cell signalling and regulation. For example, recent findings suggest that SSAO plays a role in both GLUT4-mediated glucose uptake [Enrique-Tarancon et al., J. Biol. Chem. 1998, 273, 8025-8032; Morin et al., J. Pharmacol. Exp. Ther. 2001, 297, 563-572] and adipocyte differentiation [Fontana et al., Biochem. J. 2001, 356, 769-777; Mercier et al., Biochem. J. 2001, 358, 335-342]. In addition, SSAO has been shown to be involved in inflammatory processes where it acts as an adhesion protein for leukocytes [Salmi & Jalkanen, Trends Immunol. 2001, 22, 211-216; Salmi & Jalkanen, in "Adhesion Molecules: Functions and Inhibition" K. Ley (Ed.), 2007, pp. 237-251], and might also play a role in connective tissue matrix development and maintenance [Langford et al., Cardiovasc. Toxicol. 2002, 2(2), 141-150; Göktürk et al., Am. J. Pathol. 2003, 163(5), 1921-1928]. Moreover, a link between SSAO and angiogenesis has recently been discovered [Noda et al., FASEB J. 2008, 22(8), 2928-2935], and based on this link it is expected that inhibitors of SSAO have an anti-angiogenic effect.

Several studies in humans have demonstrated that SSAO activity in blood plasma is elevated in conditions such as congestive heart failure, diabetes mellitus, Alzheimer's disease, and inflammation [Lewinsohn, Braz. J. Med. Biol. Res. 1984, 17, 223-256; Boomsma et al., Cardiovasc. Res. 1997, 33, 387-391; Ekblom, Pharmacol. Res. 1998, 37, 87-92; Kurkijärvi et al., J. Immunol. 1998, 161, 1549-1557; Boomsma et al., Diabetologia 1999, 42, 233-237; Meszaros et al., Eur. J. Drug Metab. Pharmacokinet. 1999, 24, 299-302; Yu et al., Biochim. Biophys. Acta 2003, 1647(1-2), 193-199; Mátyus et al., Curr. Med. Chem. 2004, 11(10), 1285-1298; O'Sullivan et al., Neurotoxicology 2004, 25(1-2), 303-315; del Mar Hernandez et al., Neurosci. Lett. 2005, 384(1-2), 183-187]. The mechanisms underlying these alterations of enzyme activity are not clear. It has been suggested that reactive aldehydes and hydrogen peroxide produced by endogenous amine oxidases contribute to the progression of cardiovascular diseases, diabetic complications and Alzheimer's disease [Callingham et al., Prog. Brain Res. 1995, 106, 305-321; Ekblom, Pharmacol. Res. 1998, 37, 87-92; Yu et al., Biochim. Biophys. Acta 2003, 1647(1-2), 193-199; Jiang et al., Neuropathol Appl Neurobiol. 2008, 34(2), 194-204]. Furthermore, the enzymatic activity of SSAO is involved in the leukocyte extravasation process at sites of inflammation where SSAO has been shown to be strongly expressed on the vascular endothelium [Salmi et al., Immunity 2001, 14(3), 265-276; Salmi & Jalkanen, in "Adhesion Molecules: Functions and Inhibition" K. Ley (Ed.), 2007, pp. 237-251]. Accordingly, inhibition of SSAO has been suggested to have a therapeutic value in the prevention of diabetic complications and in inflammatory diseases [Ekblom, Pharmacol. Res. 1998, 37, 87-92; Salmi et al., Immunity 2001, 14(3), 265-276; Salter-Cid et al., J. Pharmacol. Exp. Ther. 2005, 315(2), 553-562].

WO2007146188 teaches that blocking SSAO activity inhibits leucocyte recruitment, reduces the inflammatory response, and is expected to be beneficial in prevention and treatment of seizures, for example, in epilepsy.

O'Rourke et al (J Neural Transm. 2007;114(6):845-9) examined the potential of SSAO inhibitors in neurological diseases, having previously demonstrated the efficacy of SSAO inhibition in a rat model of stroke. An SSAO inhibitor is tested on relapsing-remitting experimental autoimmune encephalomyelitis (EAE), a mouse model that shares many characteristics with human multiple sclerosis. The data demonstrates the potential clinical benefit of small molecule anti-SSAO therapy in this model and therefore in treatment of human multiple sclerosis.

SSAO knockout animals are phenotypically overtly normal but exhibit a marked decrease in the inflammatory responses evoked in response to various inflammatory stimuli [Stolen et al., Immunity 2005, 22(1), 105-115]. In addition, antagonism of its function in wild type animals in multiple animal models of human disease (e.g. carrageenan-induced paw inflammation, oxazolone-induced colitis, lipopolysaccharide-induced lung inflammation, collagen-induced arthritis, endotoxin-induced uveitis) by the use of antibodies and/or small molecules has been shown to be protective in decreasing the leukocyte infiltration, reducing the severity of the disease phenotype and reducing levels of inflammatory cytokines and chemokines [Kirton et al., Eur. J. Immunol. 2005, 35(11), 3119-3130; Salter-Cid et al., J. Pharmacol. Exp. Ther. 2005, 315(2), 553-562; McDonald et al., Annual Reports in Medicinal Chemistry 2007, 42, 229-243; Salmi & Jalkanen, in "Adhesion Molecules: Functions and Inhibition" K. Ley (Ed.), 2007, pp. 237-251; Noda et al., FASEB J. 2008 22(4), 1094-1103; Noda et al., FASEB J. 2008, 22(8), 2928-2935]. This anti-inflammatory protection seems to be afforded across a wide range of inflammatory models all with independent causative mechanisms, rather than being restricted to one particular disease or disease model. This would suggest that SSAO may be a key nodal point for the regulation of the inflammatory response, and it is therefore likely that SSAO inhibitors will be effective anti-inflammatory drugs in a wide range of human diseases. VAP-1 has also been implicated in the progression and maintenance of fibrotic diseases including those of the liver and lung. Weston and Adams (J Neural Transm. 2011, 118(7), 1055-64) have summarised the experimental data implicating VAP-1 in liver fibrosis, and Weston et al (EASL Poster 2010) reported that blockade of VAP-1 accelerated the resolution of carbon tetrachloride induced fibrosis. In addition VAP-1 has been implicated in inflammation of the lung (e.g. Singh et al., 2003, Virchows Arch 442:491-495) suggesting that VAP-1 blockers would reduce lung inflammation and thus be of benefit to the treatment of cystic fibrosis by treating both the pro-fibrotic and pro-inflammatory aspects of the disease.

SSAO (VAP-1) is up regulated in gastric cancer and has been identified in the tumour vasculature of human melanoma, hepatoma and head and neck tumours (Yoong KF, McNab G, Hubscher SG, Adams DH. (1998), J Immunol 160, 3978-88.; Irjala H, Salmi M, Alanen K, Gre'nman R, Jalkanen S (2001), Immunol. 166, 6937-6943; Forster-Horvath C, Dome B, Paku S, et al. (2004), Melanoma Res. 14, 135-40.). One report (Marttila-Ichihara F, Castermans K, Auvinen K, Oude Egbrink MG, Jalkanen S, Griffioen AW, Salmi M. (2010), J Immunol. 184, 3164-3173.) has shown that mice bearing enzymically inactive VAP-1 grow melanomas more slowly, and have reduced tumour blood vessel number and diameter. The reduced growth of these tumours was also reflected in the reduced (by 60-70%) infiltration of myeloid suppressor cells. Encouragingly VAP-1 deficiency had no effect on vessel or lymph formation in normal tissue.

Small molecules of different structural classes have previously been disclosed as SSAO inhibitors, for example in WO 02/38153 (tetrahydroimidazo[4,5-c]pyridine derivatives), in WO 03/006003 (2-indanylhydrazine derivatives), in WO 2005/014530 (allylhydrazine and hydroxylamine (aminooxy) compounds) and in WO 2007/120528 (allylamino compounds). Additional SSAO inhibitors are disclosed in PCT/EP2009/062011 and PCT/EP2009/062018. Additional SSAO inhibitors are disclosed in PCT/GB2012/052265.

Patent application PCT/US2012/066153 (published as WO2013/078254) discloses compounds apparently useful as inhibitors of serine/threonine protein kinases. The compounds are structurally related to the claimed compounds, and have a bicyclic heteroaryl ring system substituted with a phenyl-cyclobutaneamine substituent.

The invention described here relates to a new class of SSAO inhibitors with biological, pharmacological, and pharmacokinetic characteristics that make them suitable for use as prophylactic or therapeutic agents in a wide range of human inflammatory diseases and immune disorders. This therapeutic capacity is designed to block SSAO enzyme action, reducing the levels of pro-inflammatory enzyme products (aldehydes, hydrogen peroxide and ammonia) whilst also decreasing the adhesive capacity of immune cells and correspondingly their activation and final extra-vasation. Diseases where such an activity is expected to be therapeutically beneficial include all diseases where immune cells play a prominent role in the initiation, maintenance or resolution of the pathology, such as multiple sclerosis, arthritis and vasculitis.

### Detailed Description of the Invention

It has surprisingly been found that the compounds of formula (I) below are inhibitors of SSAO. They are therefore useful for the treatment or prevention of diseases in which inhibition of SSAO activity is beneficial, such as inflammation, inflammatory diseases, immune or autoimmune disorders, and inhibition of tumour growth.

According to a first aspect of the invention there is provided a compound of formula (I) or a pharmaceutically acceptable salt, or N-oxide thereof as defined in the attached claims, i.e. 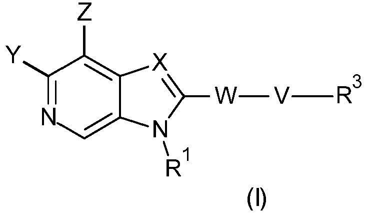 Wherein:
**Y** is selected from hydrogen, hydroxyl, -NH₂, -NH-C₁₋₄-alkyl, -NH-halo-C₁₋4-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-Cₗ₋₄-alkyl, a 3-7 membered cycloalkyl ring, -OR⁵,-NR⁶C(O)OR⁵, -NR⁶C(O)R⁵, -NR⁶C(O)NR^{4A}R^{4B}, -C(O)NR^{4A}R^{4B}, -C(O)R⁵, -C(O)OR⁵, and -NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is -N=;
**W** is a phenyl ring or a 5 or 6-membered heteroaryl ring selected from pyridinyl, pyridazinyl, pyrazinyl, pyrimidinyl, oxazolyl, thiazolyl or imidazolyl, any of which rings being optionally substituted with one or more substituents selected from halogen, cyano, oxo, C₁₋₄-alkyl, halo-C₁₋₄-alkyl, cyano-C₁₋₄-alkyl, -OR⁵, -NR^{7A}R^{7B},-NR⁶C(O)OR⁵, -NR⁶C(O)R⁵, -NR⁶C(O)NR^{7A}R^{7B}, -C(O)NR^{7A}R^{7B}, -C(O)R⁵, -C(O)OR⁵,-SO₂R⁵, -SO₂NR^{7A}R^{7B} and -NR⁶S(O)₂R⁵;
R^{7A} and R^{7B} are independently hydrogen, C₁₋₄-alkylor halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is selected from hydrogen, -C₁₋₄-alkyl, -C₁₋₄-alkyl-C₁₋₄-alkoxy or a 3-7 membered heterocyclic ring or 3-7 membered cycloalkyl ring, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{4B},-C(O)NR^{4A}R^{4B}, -C(O)R⁵, -C(O)OR⁵, -SO₂R⁵, -SO₂NR^{4A}R^{4B} and -NR⁶S(O)₂R⁵;
PROVIDED THAT groups -WVR³ and/or R¹ are not: 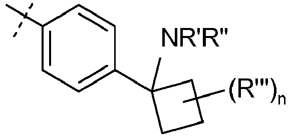 wherein
n is 0, 1, or 2;
R' and R" are independently selected from the group consisting of H, -C₁-C₆alkyl ,-(C=O)-C₁-C₆ alkyl and -(C=O)OC(CH₃)₃; and
R''' is H, OH, or C₁-C₆ alkyl.

Also disclosed herein are compound of formula (I) or a pharmaceutically acceptable salt, or N-oxide thereof as defined as follows: 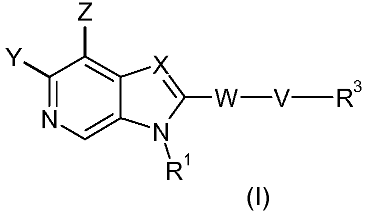 Wherein:
**Y** is selected from hydrogen, hydroxyl, -NH₂, -NH-C₁₋₄-alkyl, -NH-halo-C₁₋₄-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, a 3-7 membered cycloalkyl ring, -OR⁵,-NR^{4A}R^{4B}, -NR⁶C(O)OR⁵, -NR⁶C(O)R⁵, -NR⁶C(O)NR^{4A}R^{4B}, -C(O)NR^{4A}R^{4B}, -C(O)R⁵,-C(O)OR⁵, and -NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is selected from -N= or -C(R²)=;
R² is selected from hydrogen, halogen, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{4B},-C(O)NR^{4A}R^{4B}, -C(O)R⁵, -C(O)OR⁵, -SO₂R⁵, -SO₂NR^{4A}R^{4B} and -NR⁶S(O)₂R⁵;
**W** is a phenyl ring or a 5 or 6-membered heteroaryl ring, either ring being optionally substituted with one or more substituents selected from halogen, cyano, oxo C₁₋₄-alkyl, halo-C₁₋₄-alkyl, cyano-C₁₋₄-alkyl, -OR⁵, -NR^{7A}R^{7B}, -NR⁶C(O)OR⁵,-NR⁶C(O)R⁵, -NR⁶C(O)NR^{7A}R^{7B}, -C(O)NR^{7A}R^{7B}, -C(O)R⁵, -C(O)OR⁵,-SO₂R⁵,-SO₂NR^{7A}R^{7B} and -NR⁶S(O)₂R⁵;
R^{7A} and R^{7B} are independently hydrogen, C₁₋₄-alkylor halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is selected from hydrogen, -C₁₋₄-alkyl, -C₁₋₄-alkyl-C₁₋₄-alkoxy or a 3-7 membered heterocyclic ring or 3-7 membered cycloalkyl ring, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{4B},-C(O)NR^{4A}R^{4B}, -C(O)R⁵, -C(O)OR⁵, -SO₂R⁵, -SO₂NR^{4A}R^{4B} and -NR⁶S(O)₂R⁵;
with the proviso that groups -WVR³ and/or R¹ are not: 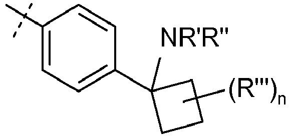 wherein
n is 0, 1, or 2;
R' and R" are independently selected from the group consisting of H, -C₁-C₆alkyl,-(C=O)-C₁-C₆ alkyl and -(C=O)OC(CH₃)₃; and
R''' is H, OH, or C₁-C₆ alkyl.

And according to a second aspect of the invention there is provided a compound of formula (I) or a pharmaceutically acceptable salt, or N-oxide thereof: 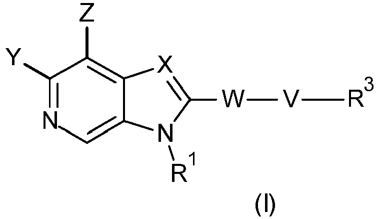 Wherein:
**Y** is selected from hydrogen, hydroxyl, -NH₂, -NH-C₁₋₄-alkyl, -NH-halo-C₁₋₄-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR⁶C(O)OR⁵, -NR⁶C(O)R⁵, - NR⁶C(O)NR^{4A}R^{4B}, -C(O)NR^{4A}R^{4B}, -C(O)R⁵, -C(O)OR⁵, and -NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is -N=;
**W** is a phenyl ring or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR^{7A}R^{7B}, -NR⁶C(O)OR⁵,-NR⁶C(O)R⁵, -NR⁶C(O)NR^{7A}R^{7B}, -C(O)NR^{7A}R^{7B}, -C(O)R⁵, -C(O)OR⁵, -SO₂R⁵,-SO₂NR^{7A}R^{7B} and -NR⁶S(O)₂R⁵;
R^{7A} and R^{7B} are independently hydrogen, C₁₋₄-alkyl or halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is hydrogen, or a 3-7 membered heterocyclic ring, or 3-7 membered cycloalkyl ring selected from cyclopropyl, cyclopentyl or cyclohexyl, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{4B},-C(O)NR^{4A}R^{4B}, -C(O)R⁵, -C(O)OR⁵, -SO₂R⁵, -SO₂NR^{4A}R^{4B} and -NR⁶S(O)₂R⁵.

Also disclosed herein is a compound of formula (I) or a pharmaceutically acceptable salt, or N-oxide thereof 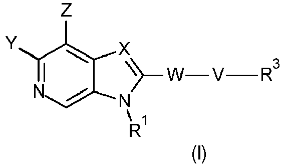 Wherein:
**Y** is selected from hydrogen, hydroxyl, -NH₂, -NH-C₁₋₄-alkyl, -NH-halo-C₁₋₄-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR^{4A}R^{4B}, -NR⁶C(O)OR⁵,-NR⁶C(O)R⁵, -NR⁶C(O)NR^{4A}R^{4B}, -C(O)NR^{4A}R^{4B}, -C(O)R⁵, -C(O)OR⁵, and-NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is selected from -N= or -C(R²)=;
R² is selected from hydrogen, halogen, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{4B},-C(O)NR^{4A}R^{4B}, -C(O)R⁵, -C(O)OR⁵, -SO₂R⁵, -SO₂NR^{4A}R^{4B} and -NR⁶S(O)₂R⁵;
**W** is a phenyl ring or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR^{7A}R^{7B}, -NR⁶C(O)OR⁵,-NR⁶C(O)R⁵, -NR⁶C(O)NR^{7A}R^{7B}, -C(O)NR^{7A}R^{7B}, -C(O)R⁵, -C(O)OR⁵,-SO₂R⁵,-SO₂NR^{7A}R^{7B} and -NR⁶S(O)₂R⁵;
R^{7A} and R^{7B} are independently hydrogen, C₁₋₄-alkyl or halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is hydrogen or a 3-7 membered heterocyclic ring or 3-7 membered cycloalkyl ring selected from cyclopropyl, cyclopentyl or cyclohexyl, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵,-NR⁶C(O)NR^{4A}R^{4B}, -C(O)NR^{4A}R^{4B}, -C(O)R⁵, -C(O)OR⁵, -SO₂R⁵, -SO₂NR^{4A}R^{4B} and-NR⁶S(O)₂R⁵.

In addition to the surprising activity of the compounds of formula (I) at the SSAO receptor, it has been surprisingly found that the claimed compounds have surprisingly low activity at the hERG ion channel. The person skilled in the art, for example a medicinal chemist, understands that low hERG activity is an important property for a pharmaceutical drug compound. Without wishing to be bound by theory, it is believed that the -WVR³ group as defined in claim 1 is especially advantageous in relation to reduced hERG activity.

It is expected that compounds of the invention may be prepared in the form of hydrates, and solvates. Any reference herein, including the claims herein, to "compounds with which the invention is concerned" or "compounds of the invention" or "the present compounds", and the like, includes reference to salts, hydrates, and solvates of such compounds. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Individual compounds of the invention may exist in an amorphous form and /or several polymorphic forms and may be obtained in different crystal habits. Any reference herein, including the claims herein, to "compounds with which the invention is concerned" or "compounds of the invention" or "the present compounds", and the like, includes reference to the compounds irrespective of amorphous or polymorphic form.

Since compounds of the invention have a nitrogen atom in an aromatic ring they may form N-oxides, and the invention includes compounds of the invention in their N-oxide form.

### DEFINITIONS

The following definitions shall apply throughout the specification and the appended claims, unless otherwise stated or indicated.

The term "C₁₋₄-alkyl" denotes a straight or branched alkyl group having from 1 to 4 carbon atoms. For parts of the range C₁₋₄-alkyl all subgroups thereof are contemplated such as C₁₋₃-alkyl, C₁₋₂-alkyl, C₂₋₄-alkyl, C₂₋₃-alkyl and C₃₋₄-alkyl. Examples of said C₁₋₄-alkyl include methyl, ethyl, n-propyl, isopropyl, n-butyl, isobutyl, *sec*-butyl and *tert*-butyl*.*

Unless otherwise specified, the term "C₃₋₇-cycloalkyl" refers to a monocyclic saturated or partially unsaturated hydrocarbon ring system having from 3 to 7 carbon atoms. Examples of said C₃₋₇-cycloalkyl include cyclopropyl, cyclobutyl, cyclopentyl, cyclohexyl, cyclohexenyl, cycloheptyl, and cycloheptenyl. For parts of the range "C₃₋₇-cycloalkyl" all subgroups thereof are contemplated such as C₃₋₇-cycloalkyl, C₃₋₆-cycloalkyl, C₃₋₅-cycloalkyl, C₃₋₄-cycloalkyl, C₄₋₇-cycloalkyl, C₄₋₆-cycloalkyl, C₄₋₅-cycloalkyl, C₅₋₇-cycloalkyl, C₅₋₆-cycloalkyl, and C₆₋₇-cycloalkyl.

The term "C₁₋₄-alkoxy" refers to a straight or branched C₁₋₄-alkyl group which is attached to the remainder of the molecule through an oxygen atom. For parts of the range C₁₋₄-alkoxy, all subgroups thereof are contemplated such as C₁₋₃-alkoxy, C₁₋₂-alkoxy, C₂₋₄-alkoxy, C₂₋₃-alkoxy and C₃₋₄-alkoxy. Examples of said C₁₋₄-alkoxy include methoxy, ethoxy, *n*-propoxy, isopropoxy, n-butoxy, isobutoxy, *sec*-butoxy and *tert-*butoxy.

The term "haloC₁₋₄-alkoxy" refers to a straight or branched C₁₋₄-alkyl group which is attached to the remainder of the molecule through an oxygen atom and has one or more hydrogen atoms thereof replaced with halogen such as fluoro or chloro. For parts of the range C₁₋₄-alkoxy, all subgroups thereof are contemplated. Examples of said C₁₋₄-alkoxy include trifluoromethoxy.

The term "hydroxy-C₁₋₄-alkyl" denotes a straight or branched C₁₋₄-alkyl group that has one or more hydrogen atoms thereof replaced with OH. Examples of said hydroxy-C₁₋₄-alkyl include hydroxymethyl, 2-hydroxyethyl and 2,3-dihydroxypropyl.

The term "halo-C₁₋₄-alkyl" denotes a straight or branched C₁₋₄-alkyl group that has one or more hydrogen atoms thereof replaced with halogen. Examples of said halo-C₁₋₄-alkyl include fluoromethyl, trifluoromethyl, trichloromethyl and 2-fluoroethyl.

The term "cyano-C₁₋₄-alkyl" denotes a straight or branched C₁₋₄-alkyl group that has one or more hydrogen atoms thereof replaced with cyano. Examples of said cyano-C₁₋₄-alkyl include cyanomethyl, 2-cyanoethyl and 3-cyanopropyl.

The term "amino-C₁₋₄-alkyl" denotes a straight or branched C₁₋₄-alkyl group substituted with an amino group. Examples of said amino-C₁₋₄-alkyl group include aminomethyl and 2-aminoethyl.

The term "C₁₋₄-alkylamino-C₁₋₄-alkyl" denotes an amino-C₁₋₄-alkyl group as defined above, wherein the amino group is substituted with a straight or branched C₁₋₄-alkyl group. Examples of said C₁₋₄-alkylamino-C₁₋₄-alkyl include methylaminoethyl and ethylaminopropyl.

The term "di(C₁₋₄-alkyl)amino-C₁₋₄-alkyl" denotes an amino-C₁₋₄-alkyl group as defined above, wherein the amino group is disubstituted with straight or branched C₁₋₄-alkyl groups, which can be the same or different. Examples of said di(C₁₋₄-alkyl)amino-C₁₋₄-alkyl include *N,N*-dimethylaminomethyl, *N*-ethyl-*N-*methylaminoethyl and *N,N*-diethylaminomethyl.

The terms "heteroaryl" and "heteroaromatic ring" denote a monocyclic heteroaromatic ring comprising 5 to 6 ring atoms in which one or more of the ring atoms are other than carbon, such as nitrogen, sulphur or oxygen. Examples of heteroaryl groups include furyl, pyrrolyl, thienyl, oxazolyl, isoxazolyl, imidazolyl, thiazolyl, isothiazolyl, pyridinyl, pyrimidinyl, tetrazolyl, pyrazolyl, pyridazinyl, pyrazinyl and thiadiazolyl.

The terms "heterocyclyl" and "heterocyclic ring" denote a non-aromatic, fully saturated or partially unsaturated, preferably fully saturated, monocyclic ring system having from 3 to 7 ring atoms, especially 5 or 6 ring atoms, in which one or more of the ring atoms are other than carbon, such as nitrogen, sulphur or oxygen. Examples of heterocyclic groups include piperidinyl, morpholinyl, homomorpholinyl, azepanyl, piperazinyl, oxo-piperazinyl, diazepinyl, tertahydropyridinyl, tetrahydropyranyl, pyrrolidinyl, tertrahydrofuranyl, and dihydropyrrolyl, groups.

The term "heterocyclic-C₁₋₄-alkyl" refers to a heterocyclic ring that is directly linked to a straight or branched C₁₋₄-alkyl group via a carbon or nitrogen atom of said ring. Examples of said heterocyclic-C₁₋₄-alkyl include piperidin-4-ylmethyl, piperidin-1-ylmethyl, morpholin-4-yl-methyl and piperazin-4-ylmethyl. The C₁₋₄-alkylpart, which includes methylene, ethylene, propylene or butylene, is optionally substituted by one or more substituents selected from halogen, amino, methoxy, or hydroxyl.

The term "C₁₋₄-alkylene" denotes a straight or branched divalent saturated hydrocarbon chain having from 1 to 4 carbon atoms. The C₁₋₄-alkylene chain may be attached to the rest of the molecule and to the radical group through one carbon within the chain or through any two carbons within the chain. Examples of C₁₋₄-alkylene radicals include methylene [-CH₂-], 1,2-ethylene [-CH₂-CH₂-], 1,1-ethylene [-CH(CH₃)-], 1,2-propylene [-CH₂-CH(CH₃)-] and 1,3-propylene [-CH₂-CH₂-CH₂-]. When referring to a "C₁₋₄-alkylene" radical, all subgroups thereof are contemplated, such as C₁₋₂-alkylene, C₂₋₃-alkylene, or C₃₋₄-alkylene.

"Halogen" refers to fluorine, chlorine, bromine or iodine, preferably fluorine and chlorine, most preferably fluorine.

"Hydroxy" refers to the -OH radical.

"Cyano" refers to the -CN radical.

"Oxo" refers to the carbonyl group =O.

"Optional" or "optionally" means that the subsequently described event or circumstance may but need not occur, and that the description includes instances where the event or circumstance occurs and instances in which it does not.

"Pharmaceutically acceptable" means being useful in preparing a pharmaceutical composition that is generally safe, non-toxic and neither biologically nor otherwise undesirable and includes being useful for veterinary use as well as human pharmaceutical use.

"Treatment" as used herein includes prophylaxis of the named disorder or condition, or amelioration or elimination of the disorder once it has been established.

"An effective amount" refers to an amount of a compound that confers a therapeutic effect on the treated subject. The therapeutic effect may be objective (i.e., measurable by some test or marker) or subjective (i.e., subject gives an indication of or feels an effect).

"Prodrugs" refers to compounds that may be converted under physiological conditions or by solvolysis to a biologically active compound of the invention. A prodrug may be inactive when administered to a subject in need thereof, but is converted *in vivo* to an active compound of the invention. Prodrugs are typically rapidly transformed *in vivo* to yield the parent compound of the invention, *e.g.* by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), pp. 498-549). Prodrugs of a compound of the invention may be prepared by modifying functional groups, such as a hydroxy, amino or mercapto groups, present in a compound of the invention in such a way that the modifications are cleaved, either in routine manipulation or *in vivo,* to the parent compound of the invention. Examples of prodrugs include, but are not limited to, acetate, formate and succinate derivatives of hydroxy functional groups or phenyl carbamate derivatives of amino functional groups.

Throughout the specification and the appended claims, a given chemical formula or name shall also encompass all salts, hydrates, solvates, N-oxides and prodrug forms thereof. Further, a given chemical formula or name shall encompass all tautomeric and stereoisomeric forms thereof. Tautomers include enol and keto forms. Stereoisomers include enantiomers and diastereomers. Enantiomers can be present in their pure forms, or as racemic (equal) or unequal mixtures of two enantiomers. Diastereomers can be present in their pure forms, or as mixtures of diastereomers. Diastereomers also include geometrical isomers, which can be present in their pure *cis* or *trans* forms or as mixtures of those.

The compounds of formula (I) may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, *p*-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

### The Group Y

In an embodiment Y is from hydrogen, hydroxyl, -NH₂, -NH-C₁₋₄-alkyl such as -NH-Methyl, -NH-ethyl, or -NH-isopropyl, -NH-halo-C₁₋₄-alkyl such as-NHtrifluoromethyl, or -C₁₋₄-alkoxy such as methoxy. In an embodiment Y is hydrogen.

### The Group Z

In an embodiment Z is hydrogen, halogen such as fluoro or chloro, hydroxyl, cyano, C₁₋₄-alkyl such as methyl or isopropyl, halo-C₁₋₄-alkyl such as triflouromethyl, C₁₋₄-alkoxy such as methoxy, halo-C₁₋₄-alkoxy such as trifluoromethoxy, -CONH₂,-SO₂NH₂, -NH₂, -NHC₁₋₄-alkyl such as -NH-Methyl, -NH-ethyl, or -NH-isopropyl, or-NHhalo-C₁₋₄-alkyl. In an embodiment Z is hydrogen.

### The Group R¹

In an R¹ embodiment is a phenyl ring, or a 5 or 6-membered heteroaryl ring either ring being optionally substituted with one or more substituents selected from halogen such as fluoro or chloro, cyano, C₁₋₄-alkylsuch as methyl or isopropyl, halo-C₁₋₄-alkyl such as trifluoromethyl, cyano-C₁₋₄-alkyl such as methylcyano, -OR⁵ such as methoxy or trifluoromethoxy, -NHMethyl, -NHisopropyl, -NR⁶C(O)OR⁵,-NR⁶C(O)R⁵, -NR⁶C(O)NR^{4A}R^{4B}, -C(O)NR^{4A}R^{4B}, -C(O)R⁵ such as -COCH₃,-C(O)OR⁵, and -NR⁶S(O)₂R⁵. In an embodiment R¹ is optionally substituted phenyl, pyridyl, pyrrole, furan, imidazole, or thiophene.

In an embodiment R¹ is a phenyl ring, or a 5 or 6-membered heteroaryl ring substituted with a 3-7 membered cycloalkyl group such as cyclopropyl, cyclobutyl, cyclopentyl, cyclohexyl; preferably cyclopropyl.

R^{4A}, R^{4B} R⁵ and R⁶ are each independently selected from hydrogen, C₁₋₄-alkyl such as methyl, ethyl or isopropyl, or halo-C₁₋₄-alkyl such as trifluoromethyl, or

R^{4A} and R^{4B} together with the nitrogen to which they are attached form a 3-7 membered cyclic amino group such as aziridine, azetidine, oxetane, pyrrolidine, piperidine, piperazine, homopiperidine, homopiperazine, morpholine, or tetrahydrofuran, optionally substituted by one or more substituents selected from: halogen such as fluoro or chloro, hydroxyl, cyano, C₁₋₄-alkyl such as methyl or isopropyl, halo-C₁₋₄-alkyl such as triflouromethyl, C₁₋₄-alkoxy such as methoxy, halo-C₁₋₄-alkoxy such as trifluoromethoxy, -CONH₂, -SO₂NH₂, -NH₂, -NHC₁₋₄-alkyl,-NHhalo-C₁₋₄-alkyl;

### The Group X

In an embodiment X is-N=;

### The Group R²

As used herein, R² is hydrogen, halogen such as fluoro or chloro, cyano, C₁₋₄-alkyl such as methyl or ethyl or isopropyl, halo-C₁₋₄-alkyl such as trifluoromethyl. In an embodiment R² is hydrogen.

### The Group W

In an embodiment W is a phenyl ring. In an alternative embodiment W a 6-membered heterocyclic ring selected from pyridine, pyridazine, pyrazine, or pyrimidine. In an alternative embodiment W is a 5-membered ring selected from oxazole, thiazole or imidazole. In an embodiment W is imidazolyl and the imidazolyl ring is connected to the pyrrolopyridine core (i.e. the rest of the molecule) via an imidazolyl ring carbon atom. In an embodiment W is a pyrazole ring.

Any of the aforementioned rings are optionally substituted with one or more substituents as defined in claim 1. In an embodiment W is substituted with one or more groups selected from fluoro, chloro, cyano, methyl or trifluoromethyl.

In an embodiment W is a divalent group selected from any one of the following rings, any of which rings is optionally substituted with one or more substituents as defined in relation to formula (I). 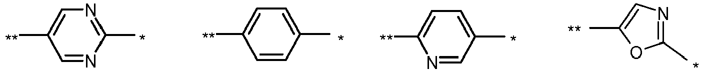 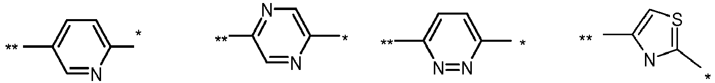 wherein the bond marked ** is directly connected to the rest of the molecule and the atom marked * is directly connected to V.

### The Group V

In an embodiment V is selected from a bond, -O-, -N(R⁶)- such as -NH- or-N(CH₃)-, -(C=O)-, -CONR⁶- such as -CONH- or -CON(CH₃)-, -NR⁶C(O)- such as-NHC(O)- or -N(CH₃)C(O)-, or -C₁₋₄-alkylene-, wherein the C₁₋₄-alkylene group is optionally substituted by halogen such as fluoro or chloro, and wherein any one of the carbon atoms of the C₁₋₄-alkylene group may be replaced by -O- or -N(R⁶)-such as -CH₂O- in either direction or -CH₂-NH-; -CH₂-N(CH₃)- in either direction.

### The Group R³

In an embodiment R³ is hydrogen. In an alternative embodiment R³ an optionally substituted 3-7 membered heterocyclic ring such as aziridine, azetidine, oxetane, pyrrolidine, piperidine, piperazine, homopiperidine, homopiperazine, morpholine, or tetrahydrofuran. In an embodiment R³ is an optionally substituted 3-7 membered cycloalkyl ring such as cyclopropyl, cyclobutyl, cyclopentyl or cyclohexyl. In an alternative embodiment R³ is an optionally substituted 5 or 6-membered heteroaryl ring such as imidazole, phenyl, pyridine, thophene. The optional substituents are defined in formula (I). In an embodiment any one of the rings is optionally substituted with one or more substituents selected from halogen such as fluoro or chloro, oxo, hydroxyl, cyano, C₁₋₄-alkyl such as methyl, ethyl, propyl, t-butyl, or isopropyl, halo-C₁₋₄-alkyl such as trifluoromethyl, cyano-C₁₋₄-alkyl, -OR⁵ such as methocy or trifluoromethoxy, -NR^{4A}R^{4B} such as -NH₂, NHmethyl, or morpholine or piperidine, -NR⁶C(O)OR⁵, -NR⁶C(O)R⁵, -NR⁶C(O)NR^{4A}R^{4B}, -C(O)NR^{4A}R^{4B}, -C(O)R⁵,-C(O)OR⁵, -SO₂R⁵, -SO₂NR^{4A}R^{4B} and -NR⁶S(O)₂R⁵.

In an embodiment R³ is selected from the following ring systems:

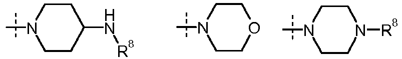

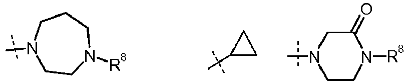

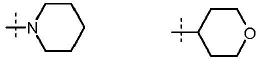

Wherein R⁸ is selected from hydrogen, CH₃, -CONH₂, -NHCONH₂, -S(O)₂CH₃, -COCH₃.

In an embodiment R³ is selected from the following ring systems:

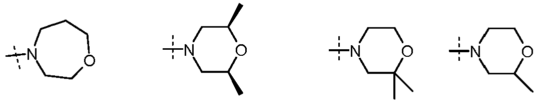

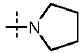

In an embodiment R³ is selected from hydrogen, -C₁₋₄-alkyl such as methyl, ethyl, propyl and isopropyl, and -C₁₋₄-alkyl-C₁₋₄-alkoxy such as -(CH₂)₂OCH₃.

In an embodiment the group -VR³ is selected from: 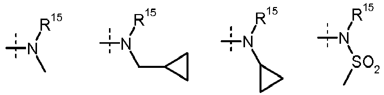 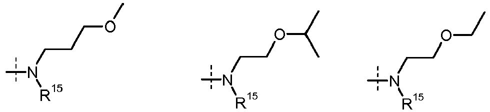 wherein R¹⁵ is hydrogen or methyl.

In an embodiment, the invention includes a compound of formula (Xa) 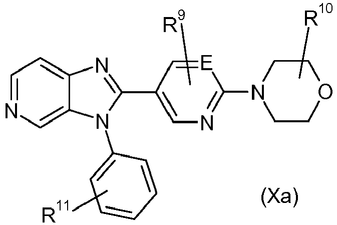
wherein E is -C= or -N=,
R⁹ and R¹⁰ are each independently one or more substituents selected from hydrogen, halogen, cyano, oxo, C₁₋₄-alkyl such as methyl, -OC₁₋₄-alkyl such as OCH₃, and halo-C₁₋₄-alkyl; and
R¹¹ is one or more substituents selected from hydrogen, halogen such as fluoro and/or chloro, cyano, cyclopropyl, C₁₋₄-alkyl such as methyl, and halo-C₁₋₄-alkyl.

In one aspect, the invention relates to a compound of formula (I) for use in therapy. The compounds as defined above are useful as inhibitors of SSAO activity. As such, they are useful in the treatment or prevention of conditions and diseases in which inhibition of SSAO activity is beneficial. More specifically, they are useful for the treatment or prevention of inflammation, inflammatory diseases, immune or autoimmune disorders, cystic fibrosis, or inhibition of tumour growth; and they are useful in the manufacture of a medicament for treatment or prevention of inflammation, inflammatory diseases, immune or autoimmune disorders, cystic fibrosis, or inhibition of tumour growth

In particular, it is believed that compounds of formula (I) are useful for the treatment or prevention of arthritis (such as rheumatoid arthritis, juvenile rheumatoid arthritis, osteoarthritis and psoriatic arthritis), synovitis, vasculitis, conditions associated with inflammation of the bowel (such as Crohn's disease, ulcerative colitis, inflammatory bowel disease and irritable bowel syndrome), atherosclerosis, multiple sclerosis, Alzheimer's disease, vascular dementia, pulmonary inflammatory diseases (such as asthma, chronic obstructive pulmonary disease and acute respiratory distress syndrome), fibrotic diseases (including idiopathic pulmonary fibrosis, cardiac fibrosis and systemic sclerosis (scleroderma)), inflammatory diseases of the skin (such as contact dermatitis, atopic dermatitis and psoriasis), systemic inflammatory response syndrome, sepsis, inflammatory and/or autoimmune conditions of the liver (such as autoimmune hepatitis, primary biliary cirrhosis, alcoholic liver disease, sclerosing cholangitis, and autoimmune cholangitis), diabetes (type I or II) and/or the complications thereof, chronic heart failure, congestive heart failure, ischemic diseases (such as stroke and ischemia-reperfusion injury), and myocardial infarction and/or the complications thereof, or epilepsy.

It is believed that the compounds of the invention are especially useful for the treatment or prevention of vasculitis, including, but not limited to, giant cell arteritis, Takayasu's arteritis, Polyarteritis nodosa, Kawasaki disease, Wegener's granulomatosis, Churg-Strauss syndrome, microscopic polyangiitis, Henoch-Schönlein purpura, cryoglobulinemia, cutaneous leukocytoclastic angiitis and primary angiitis of the central nervous system.

It is also believed that the compounds of the invention are especially useful for the treatment of rheumatoid arthritis, chronic obstructive pulmonary disease or atopic dermatitis.

In view of the evidence cited in the above introduction that VAP-1 is up regulated in several cancers, including gastric cancer, melanoma, hepatoma and head and neck tumours and that mice bearing enzymatically inactive VAP-1 grow melanomas more slowly, and in view of the link between VAP-1 and angiogenesis, it is also expected that the compounds of the invention are anti-angiogenic and therefore have utility in the treatment of cancers by inhibition of tumour growth.

The invention thus includes the compounds of formula (I) above for use in the treatment or prevention of the above-mentioned conditions and diseases. The invention also includes the use of said compounds in the manufacture of a medicament for the treatment or prevention of the above-mentioned conditions and diseases. Also disclosed herein are methods for treatment or prevention of such conditions and diseases, comprising administering to a mammal, including man, in need of such treatment an effective amount of a compound as defined above.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic sampling of subject tissue, fluids, specimens, cells, proteins, chemical markers, genetic materials, etc. as markers or indicators of the treatment regimen. In other methods, the subject is prescreened or identified as in need of such treatment by assessment for a relevant marker or indicator of suitability for such treatment.

Also disclosed herein is a method of monitoring treatment progress. The method includes the step of determining a level of diagnostic marker (Marker) (e.g., any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. A second level of Marker in the subject may be determined at a time point later than the determination of the first level, and the two levels are compared to monitor the course of disease or the efficacy of the therapy. A pre-treatment level of Marker in the subject may be determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabeling/assay techniques, blotting/chemiluminescence methods, real-time PCR, and the like.

### COMPOSITIONS

A currently preferred embodiment of the invention is a pharmaceutical composition comprising a compound of formula (I), together with one or more pharmaceutically acceptable carriers and/or excipients.

For clinical use, the compounds of the invention are formulated into pharmaceutical formulations for various modes of administration. It will be appreciated that compounds of the invention may be administered together with a physiologically acceptable carrier, excipient, or diluent. The pharmaceutical compositions of the invention may be administered by any suitable route, preferably by oral, rectal, nasal, topical (including buccal and sublingual), sublingual, transdermal, intrathecal, transmucosal or parenteral (including subcutaneous, intramuscular, intravenous and intradermal) administration.

Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration.

The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds of the invention may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

### PREPARATION OF COMPOUNDS OF THE INVENTION

The compounds of formula (I) above may be prepared by, or in analogy with, conventional methods. The preparation of intermediates and compounds according to the examples of the present invention may in particular be illuminated by the following Schemes. Definitions of variables in the structures in schemes herein are commensurate with those of corresponding positions in the formulas delineated herein. 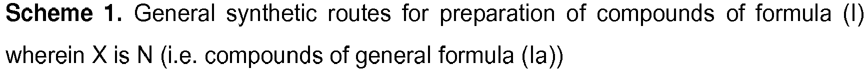 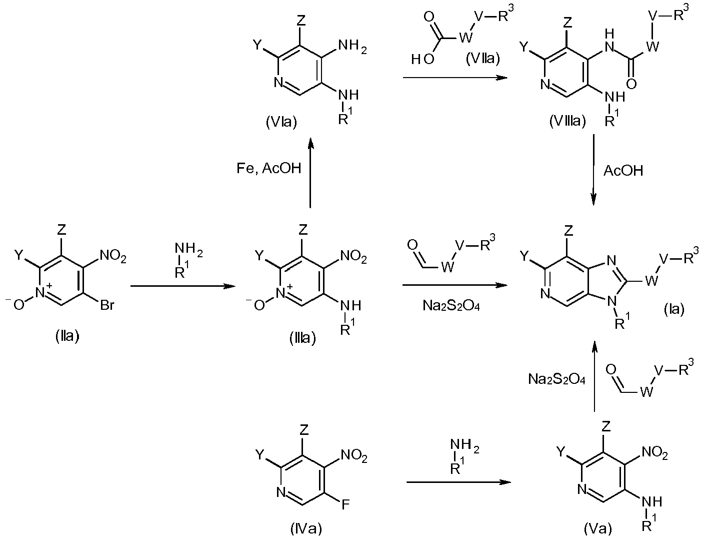 wherein V, W, X, Y, Z, R¹, R² and R³ are as defined in formula (I);

Compounds of general formula (I) where X is N (designated compounds of general formula (Ia)), can easily be prepared by a number of alternative routes. For example, 3-bromo-4-nitropyridine N-oxides of general formula (IIa) can undergo SnAr displacement with R¹NH₂ amines to give compounds of general formula (IIIa), which can in turn be reductively cyclised to give compounds of general formula (Ia). Alternatively, 3-fluoro-4-nitropyridines of general formula (IVa) can undergo SnAr displacement with R¹NH₂ amines to give compounds of general formula (Va), which can in turn be reductively cyclised to give compounds of general formula (Ia). Alternatively, compounds of general formula (IIIa) can be reduced to pyridine-3,4-diamines of general formula (Via). Compounds of general formula (VIa) can then undergo amide formation with carboxylic acids of general formula (Vila) to give amides of general formula (Villa) which can be cyclised to give compounds of general formula (Ia).

Optionally, the group W-V-R³ can be built up sequentially using standard chemistry methodologies including amide, urea and sulphonamide formation. If required, standard protecting group strategies can be employed to facilitate the synthesis.

Optionally, a compound of formula (Ia) can also be transformed into another compound of formula (Ia) in one or more synthetic steps. 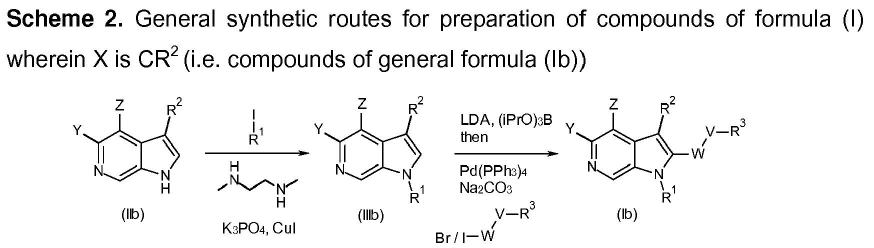 wherein V, W, X, Y, Z, R¹, R² and R³ are as defined in formula (I);
Compounds of general formula (I) where X is CR² (designated compounds of general formula (Ib)), can easily be prepared standard means. For example, 6-azaindoles of general formula (IIb) can be N-arylated with R¹-I iodides to give compounds of general formula (IIIb) which can in turn be converted to compounds of general formula (Ib) by boronate formation and subsequent Suzuki coupling.

Optionally, the group W-V-R³ can be built up sequentially using standard chemistry methodologies including amide, urea and sulphonamide formation. If required, standard protecting group strategies can be employed to facilitate the synthesis.

Optionally, a compound of formula (Ib) can also be transformed into another compound of formula (Ib) in one or more synthetic steps.

The following abbreviations have been used:
- Ac: acetyl
- aq: aqueous
- Boc: *tertiary*-butyloxycarbonyl
- calcd: calculated
- d: day(s)
- DCM: dichloromethane
- DIPEA: diisopropylethylamine
- DMA: dimethylacetamide
- DMF: dimethylformamide
- DMSO: Dimethyl sulfoxide
- EDC: *N*-(3-dimethylaminopropyl)-*N'*-ethylcarbodiimide
- ES⁺: electrospray ionization
- Et₃N: triethylamine
- EtOAc: ethyl acetate
- EtOH: ethanol
- Ex: Example
- h: hour(s)
- HATU: O-(7-azabenzotriazol-1-yl)-*N*,*N,N·,N*·-tetramethyluronium hexafluorophosphate
- HBTU: O-benzotriazole-*N*,*N*,*N'*,*N'*-tetramethyl-uronium-hexafluoro phosphate
- HPLC: High Performance Liquid Chromatography
- Int: Intermediate
- LCMS: Liquid Chromatography Mass Spectrometry
- LDA: Lithium diisopropylamide
- M: molar
- MeCN: acetonitrile
- MeOH: methanol
- [MH]⁺: protonated molecular ion
- Min: minute(s)
- NMP: 1-methyl-2-pyrrolidinone
- QTOF: Quadrupole time-of-flight mass spectrometer
- RP: reverse phase
- RT: room temperature
- Rt: retention time
- sat: saturated
- TFA: trifluoroacetic acid
- THF: Tetrahydrofuran
- UV: Ultra violet
- XPhos: 2-dicyclohexylphosphino-2',4',6'-triisopropylbiphenyl

### EXAMPLES, REFERENCE EXAMPLES, AND INTERMEDIATE COMPOUNDS

### Experimental Methods

Reactions were conducted at room temperature unless otherwise specified. Microwave reactions were performed with a Biotage microwave reactor using process vials fitted with aluminium caps and septa. Preparative chromatography was performed using a Flash Master Personal system equipped with Isolute Flash II silica columns or using a CombiFlash Companion system equipped with GraceResolv silica column. Reverse Phase HPLC was performed on a Gilson system with a UV detector equipped with Phenomenex Synergi Hydro RP 150x10mm, or YMC ODS-A 100/150x20mm columns. The purest fractions were collected, concentrated and dried under vacuum. Compounds were typically dried in a vacuum oven at 40°C prior to purity analysis. Compound analysis was performed by HPLC/LCMS using an Agilent 1100 HPLC system / Waters ZQ mass spectrometer connected to an Agilent 1100 HPLC system with a Phenomenex Synergi, RP-Hydro column (150x4.6mm, 4um, 1.5mL per min, 30°C, gradient 5-100% MeCN (⁺0.085% TFA) in water (⁺0.1% TFA) over 7min, 200-300nm). Accurate masses were measured using a Waters QTOF electrospray ion source and corrected using Leucine Enkephalin lockmass. Spectra were acquired in positive and negative electrospray mode. The acquired mass range was m/z 100-1000. Test compounds were dissolved in DMSO to give a 10mM stock solution. Typically 5mL of the DMSO stock were diluted with 495mL of acetonitrile and then further diluted with acetonitrile and water (1:1) to give a final concentration of 0.2mM. The mass values reported correspond either to the parent molecule with a hydrogen added [MH] or with a hydrogen subtracted [M-H]. The compounds prepared were named using IUPAC nomenclature.

### INTERMEDIATE 1

### 3-[(4-Chlorophenyl)amino]-4-nitropyridin-1-ium-1-olate

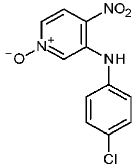

3-Bromo-4-nitropyridine *N*-oxide (1.00g, 4.57mmol) and 4-chloroaniline (1.75g, 13.7mmol) were dissolved in EtOH and heated at 60°C for 18h. The reaction mixture was cooled to 0°C and the precipitate was collected by filtration and washed with cold EtOH to give the title compound as an orange solid (317mg, 26.1%). LCMS (ES⁺): 266.1 [MH]⁺. HPLC: Rt 5.44min, 99.5% purity.

### INTERMEDIATES 2-3

Intermediates 2-3 were prepared similarly to Intermediate 1, by coupling of 3-bromo-4-nitropyridine *N*-oxide with the appropriate aniline; see Table 1 below.

**Table 1: SnAr formation of anilines**

| | | | |
|---|---|---|---|
| 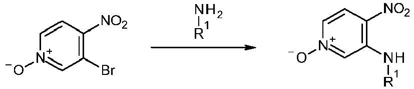 | | | |

| **Int** | **Structure** | **Name** | **Form, Yield, LCMS, HPLC** |
|---|---|---|---|
| 2 | 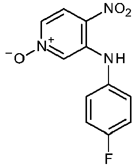 | 3-[(4-Fluorophenyl)amino]-4-nitropyridin-1-ium-1-olate | Orange solid |
| | | | Yield 2.66g, 46.7% |
| | | | LCMS (ES⁺): 250.1 [MH]⁺ HPLC: Rt 5.00min, 97.3% purity |
| 3 | 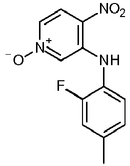 | 3-[(2-Fluoro-4-methylphenyl)amino]-4-nitropyridin-1-ium-1-olate | Orange solid |
| | | | Yield 200mg, 5.55% |
| | | | LCMS (ES⁺): 264.0 [MH]⁺ HPLC: Rt 5.52min, 93.2% purity |

### INTERMEDIATE 4

### 3-[(4-Fluoro-2-methylphenyl)amino]-4-nitropyridin-1-ium-1-olate

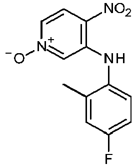

3-Fluoro-4-nitropyridine *N*-oxide (1.00g, 6.33mmol) and 4-fluoro-2-methylaniline (2.42mL, 25.3mmol) were dissolved in EtOH (12mL) and heated at 90°C for 16h. The reaction mixture was cooled to RT, the precipitate was collected by filtration and washed with cold EtOH (10mL) to give the title compound (1.60g, 96.2%) as a yellow solid. LCMS (ES⁺): 264.1 [MH]⁺. HPLC: Rt 5.56min, 95.9% purity.

### INTERMEDIATES 5-12

Intermediates 5-12 were prepared similarly to Intermediate 4, by coupling of 3-fluoro-4-nitropyridine *N*-oxide with the appropriate aniline; see Table 2 below.

**Table 2: SnAr formation of anilines**

| | | | |
|---|---|---|---|
| 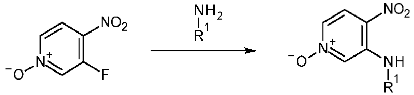 | | | |

| **Int** | **Structure** | **Name** | **Form, Yield, LCMS, HPLC** |
|---|---|---|---|
| 5 | 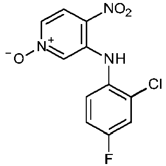 | 3-[(2-Chloro-4-fluorophenyl)amino]-4-nitropyridin-1-ium-1-olate | Yellow solid |
| | | | Yield 1.93g, 53.9% |
| | | | LCMS (ES⁺): 284.2 [MH]⁺ HPLC: Rt 5.67min, 96.0% purity |
| 6 | 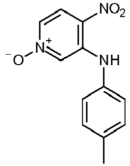 | 3-[(4-Methylphenyl)amino]-4-nitropyridin-1-ium-1-olate | Orange solid |
| | | | Yield 1.12g, 96.2% |
| | | | LCMS (ES⁺): 246.1 [MH]⁺ HPLC: Rt 5.65min, 99.3% purity |
| 7 | 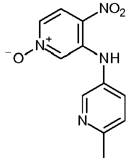 | 3-[(6-Methylpyridin-3-yl)amino]-4-nitropyridin-1-ium-1-olate | Orange solid |
| | | | Yield 1.04g, 89.0% |
| | | | LCMS (ES⁺): 247.0 [MH]⁺ |
| | | | HPLC: Rt 2.86min, 64.5% purity |
| 8 | 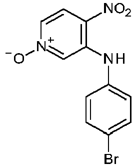 | 3-[(4-Bromophenyl)amino]-4-nitropyridin-1-ium-1-olate | Orange solid |
| | | | Yield 1.55g, crude |
| | | | LCMS (ES⁺): 309.9 [MH]⁺ |
| | | | HPLC: Rt 5.66min, 95.8% purity |
| 9 | 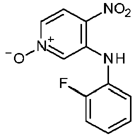 | 3-[(2-Fluorophenyl)amino]-4-nitropyridin-1-ium-1-olate | Orange solid |
| | | | Yield 1.08g, 91.3% |
| | | | LCMS (ES⁺): 250.0 [MH]⁺ |
| | | | HPLC: Rt 5.14min, 100% purity |
| 10 | 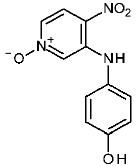 | 3-[(4-Hydroxyphenyl)amino]-4-nitropyridin-1-ium-1-olate | Dark orange solid |
| | | | Yield 1.40g, crude |
| | | | LCMS (ES⁺): 248.0 [MH]⁺ |
| | | | HPLC: Rt 4.25min, 96.8% purity |
| 11 | 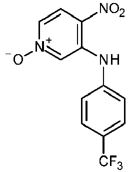 | 4-Nitro-3⁻{[4-(trifluoromethyl)phenyl]amino}pyridin-1-ium-1-olate | Orange solid |
| | | | Yield 1.38g, 97.3% |
| | | | LCMS (ES⁺): 300.0 [MH]⁺ |
| | | | HPLC: Rt 5.84min, 93.8% purity |
| 12 | 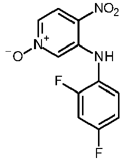 | 3-[(2,4-Difluorophenyl)amino]-4-nitropyridin-1-ium-1-olate | Yellow solid |
| | | | Yield 2.16g, 63.9% |
| | | | LCMS (ES⁺): 268.0 [MH]⁺ |
| | | | HPLC: Rt 5.14min, 99.3% purity |

### INTERMEDIATE 13

### N-(4-Chloro-3-fluorophenyl)-4-nitropyridin-3-amine

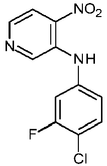

NaH (422mg, 60% dispersion in mineral oil, 10.6mmol) was suspended in THF (20mL) and 4-chloro-3-fluoroaniline (1.54g, 10.6mmol) and 3-fluoro-4-nitropyridine (500mg, 3.52mmol) were added. The reaction mixture was stirred for 18h, quenched with sat. aq. NH₄Cl (2mL), and concentrated *in vacuo.* The residue was partitioned between water (50mL) and DCM (50mL) and the organic fraction was dried (MgSO₄) and concentrated *in vacuo.* The residue was purified by column chromatography to give the title compound (207mg, 22.0%) as an orange solid. LCMS (ES⁺): 268.0 [MH]⁺.

### INTERMEDIATES 14-18

Intermediates 14-18 were prepared similarly to Intermediate 13, by coupling of 3-fluoro-4-nitropyridine with the appropriate aniline; see Table 3 below.

**Table 3: SnAr formation of anilines**

| | | | |
|---|---|---|---|
| 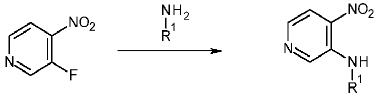 | | | |

| **Int** | **Structure** | **Name** | **Form, Yield, LCMS, HPLC** |
|---|---|---|---|
| 14 | 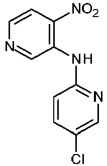 | *N*-(5-Chloropyridin-2-yl)-4-nitropyridin-3-amine | Orange gum |
| | | | Yield 221mg, 25.1% |
| | | | LCMS (ES⁺): 251.1 [MH]⁺ HPLC: Rt 5.99min, 93.6% purity |
| 15 | 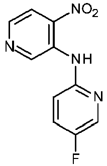 | 5-Fluoro-*N*-(4-nitropyridin-3-yl)pyridin-2-amine | Orange solid |
| | | | Yield 441mg, 53.5% |
| | | | LCMS (ES⁺): 235.0 [MH]⁺ HPLC: Rt 5.40min, 95.2% purity |
| 16 | 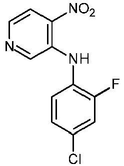 | *N*-(4-Chloro-2-fluorophenyl)-4-nitropyridin-3-amine | Orange solid |
| | | | Yield 1.26g, 66.9% |
| | | | LCMS (ES⁺): 268.1 [MH]⁺ |
| 17 | 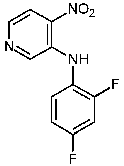 | *N*-(2,4-Difluorophenyl)-4-nitropyridin-3-amine | Orange solid |
| | | | Yield 752mg, 42.5% |
| | | | LCMS (ES⁺): 252.0 [MH]⁺ |
| | | | HPLC: Rt 5.91min, 77.8% purity |
| 18 | 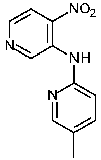 | 5-Methyl-*N*-(4-nitropyridin-3-yl)pyridin-2-amine | Dark red solid |
| | | | Yield 611mg, 37.7% |
| | | | LCMS (ES⁺): 231.1 [MH]⁺ |
| | | | HPLC: Rt 4.61min, 97.5% purity |

### INTERMEDIATE 19

### 3-N-(4-Chlorophenyl)pyridine-3,4-diamine

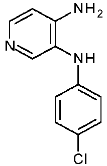

Intermediate 1 (317mg, 1.19mmol) was dissolved in AcOH (10mL) and iron powder (333mg, 5.97mmol) was added. The reaction mixture was heated at reflux for 1h, diluted with water (50mL), basified with Na₂CO₃ and extracted into DCM (3x50mL). The combined organic fractions were dried (MgSO₄) and concentrated *in vacuo* to give the title compound as a red gum (254mg, 96.9%). LCMS (ES⁺): 220.2 [MH]⁺. HPLC: Rt 4.31min, 99.5% purity.

### INTERMEDIATE 20

### 3-N-(4-Fluorophenyl)pyridine-3,4-diamine

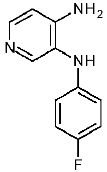

Intermediate 20 was prepared similarly to Intermediate 19, using Intermediate 2 instead of Intermediate 1, to give the title compound (6.33g, 91.3%) as a brown solid. LCMS (ES⁺): 204.1 [MH]⁺.

### INTERMEDIATE 21

### 3-N-(4-Methylphenyl)pyridine-3,4-diamine

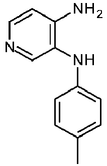

Intermediate 6 (6.00g, 24.5mmol) and ammonium formate (12.3g, 196mmol) were suspended in EtOH (200mL), Raney Nickel (50% slurry in water; 11.0mL) was added and the reaction mixture was heated at 85°C for 2h, filtered through Celite and concentrated *in vacuo.* The residue was partitioned between water (150mL) and DCM/MeOH (150mL/20mL) and the aqueous fraction was extracted with DCM (100mL). The combined organic fractions were dried (MgSO₄) and concentrated *in vacuo* to give the title compound (3.20g, 65.6%) as a blue solid. LCMS (ES⁺): 200.1 [MH]⁺.

### INTERMEDIATE 22

### 3-N-(4-Chloro-2-fluorophenyl)pyridine-3,4-diamine

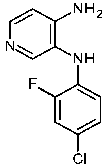

Intermediate 22 was prepared similarly to Intermediate 21, using Intermediate 16 instead of Intermediate 6, to give the title compound (509mg, 31.7%) as an orange solid. LCMS (ES⁺): 238.1 [MH]⁺.

### INTERMEDIATE 23

### 3-N-(6-Methylpyridin-3-yl)pyridine-3,4-diamine

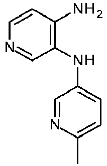

Intermediate 7 (1.40g, 5.69mmol) and hydrazine monohydrate (1.11mL, 22.8mmol) were suspended in THF (20mL) and EtOH (20mL), Raney nickel (∼50% slurry in water; 2mL) was added slowly at 0°C and the reaction mixture was warmed to RT and stirred for 2h. The mixture was filtered through Celite washing with MeOH (80mL) and the combined filtrate was concentrated *in vacuo.* The residue was purified by column chromatography to give the title compound (836mg, 73.4%) as an off white solid. LCMS (ES⁺): 201.1 [MH]⁺.

### INTERMEDIATE 24

### 3-N-(5-Methylpyridin-2-yl)pyridine-3,4-diamine

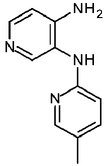

Intermediate 24 was prepared similarly to Intermediate 23, using Intermediate 18 instead of Intermediate 7, to give the title compound (741mg, 70.1%) as a pale purple solid. LCMS (ES⁺): 201.1 [MH]⁺. HPLC: Rt 2.39min, 98.5% purity.

### INTERMEDIATE 25

### 3-N-(2,4-Difluorophenyl)pyridine-3,4-diamine

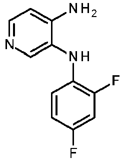

Intermediate 25 was prepared similarly to Intermediate 23, using Intermediate 12 instead of Intermediate 7, to give the title compound (1.32g, 73.6%) as a pink solid. LCMS (ES⁺): 222.0 [MH]⁺. HPLC: Rt 4.08min, 99.2% purity.

### INTERMEDIATE 26

### Methyl 6-[(morpholin-4-yl)carbonyl]pyridine-3-carboxylate

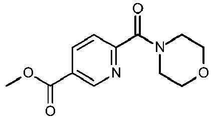

5-(Methoxycarbonyl)pyridine-2-carboxylic acid (758mg, 4.18mmol) was dissolved in DMF (25mL) and morpholine (603uL, 5.23mmol), Et₃N (2.45mL, 16.7mmol) and HBTU (1.67g, 4.39mmol) were added. The reaction mixture was stirred for 16h and concentrated *in vacuo.* The residue was dissolved in EtOAc (100mL), washed with sat. aq. Na₂CO₃ (100mL), dried (MgSO₄) and concentrated *in vacuo.* The residue was purified by column chromatography to give the title compound (914mg, 87.3%) as a yellow oil. LCMS (ES⁺): 251.2 [MH]⁺.

### INTERMEDIATE 27

### Methyl 6-(cyclopropylcarbamoyl)pyridine-3-carboxylate

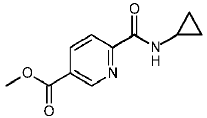

Intermediate 27 was prepared similarly to Intermediate 26, using cyclopropylamine instead of morpholine, to give the title compound (774mg, 42.4%) as a white solid. LCMS (ES⁺): 221.2 [MH]⁺.

### INTERMEDIATE 28

### Methyl 5-[(oxan-4-yl)amino]pyrazine-2-carboxylate

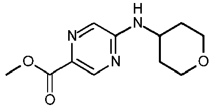

Methyl 5-chloro-2-pyrazinecarboxylate (507mg, 2.94mmol), Et₃N (1.08mL, 7.64mmol) and 4-aminotetrahydropyran (395uL, 3.82mmol) were dissolved in dioxane (5mL) and heated in a microwave reactor at 100°C for 20min. Water (50mL) and brine (25mL) were added and the reaction mixture was extracted into EtOAc (2x100mL), dried (MgSO₄) and concentrated *in vacuo* to give the title compound (236mg, 33.9%) as a yellow oil. LCMS (ES⁺): 238.2 [MH]⁺.

### INTERMEDIATES 29-35

Intermediates 29-35 were prepared similarly to Intermediate 28, by coupling of with the appropriate aromatic ester with the appropriate amine; see Table 4 below.

**Table 4: Coupling of with the appropriate aromatic ester with the appropriate amine**

| | | | |
|---|---|---|---|
| 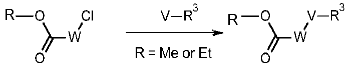 | | | |

| **Int** | **Structure** | **Name** | **Form, Yield, LCMS, HPLC** |
|---|---|---|---|
| 29 | 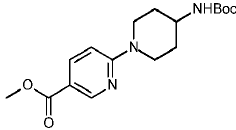 | Methyl 6-(4-{[(*tert-*butoxy)carbonyl]amino}piperidin-1-yl)pyridine-3-carboxylate | Off white solid |
| | | | Yield 1.66g, 84.9% |
| | | | LCMS (ES⁺): 336.1 [MH]⁺ HPLC: Rt 4.73min, 98.2% purity. |
| 30 | 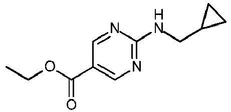 | Ethyl 2-[(cyclopropylmethyl)amino]pyrimidine-5-carboxylate | Yellow solid |
| | | | Yield 566mg, 95.5% |
| | | | LCMS (ES⁺): 222.1 [MH]⁺ HPLC: Rt 5.79min, 92.9% purity. |
| 31 | 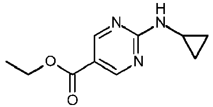 | Ethyl 2-(cyclopropylamino)pyrimidine-5-carboxylate | White solid |
| | | | Yield 526mg, 94.7% |
| | | | LCMS (ES⁺): 208.1 [MH]⁺ HPLC: Rt 4.86min, 93.7% purity. |
| 32 | 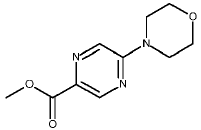 | Methyl 5-(morpholin-4-yl)pyrazine-2-carboxylate | Pale yellow solid |
| | | | Yield 628mg, 95.8% |
| | | | LCMS (ES⁺): 224.2 [MH]⁺ |
| 33 | 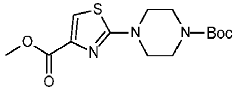 | *tert*-Butyl 4-[4-(methoxycarbonyl)-1,3-thiazol-2-yl]piperazine-1-carboxylate | White solid |
| | | | Yield 324mg, 35.1% |
| | | | LCMS (ES⁺): 350.1 [MNa]⁺. HPLC: Rt 6.04min, 100% purity. |
| 34 | 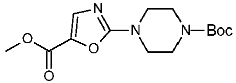 | *tert*-Butyl 4-[5-(methoxycarbonyl)-1,3-oxazol-2-yl]piperazine-1-carboxylate | Pale yellow solid |
| | | | Yield 406mg, 42.1% |
| | | | LCMS (ES⁺): 334.2 [MNa]⁺. HPLC: Rt 5.81min, 97.1% purity. |
| 35 | 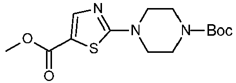 | *tert*-Butyl 4-[5-(methoxycarbonyl)-1,3-thiazol-2-yl]piperazine-1-carboxylate | White solid |
| | | | Yield 712mg, 64.4% |
| | | | LCMS (ES⁺): 350.2 [MNa]⁺. HPLC: Rt 6.34min, 99.0% purity. |

### INTERMEDIATE 36

### Methyl 6-(morpholin-4-ylmethyl)pyridine-3-carboxylate

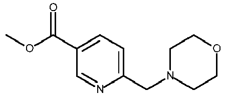

Methyl 6-formylnicotinate (507mg, 3.07mmol) and morpholine (267uL, 3.07mmol) were dissolved in DCM (20mL) and NaBH(OAc)₃ (976mg, 4.60mmol) was added.

The reaction mixture was stirred for 2h. The reaction mixture was diluted with DCM (40mL) then washed with sat. aq. Na₂CO₃ (40mL), dried (MgSO₄) and the solvents removed *in vacuo* to yield the title compound (660mg, 91.0%) as a yellow oil. LCMS (ES⁺): 237.2 [MH]⁺.

### INTERMEDIATE 37

### Methyl 6-(cyclopropylcarbamoyl)pyridine-3-carboxylate

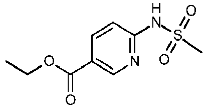

Ethyl 6-aminopyridine-3-carboxylate (738mg, 4.44mmol) was dissolved in pyridine (20mL), cooled to 0°C and methanesulfonyl chloride (1.72mL, 22.2mmol) was added. The reaction mixture was stirred at RT for 16h, concentrated *in vacuo* and partitioned between DCM (50mL) and 1M aq. citric acid (50mL). The organic fraction was dried (MgSO₄) and concentrated *in vacuo* to give the title compound (1.34g, crude) as a brown solid. LCMS (ES⁺): 245.1 [MH]⁺.

### INTERMEDIATE 38

### 2-[(Oxan-4-yl)amino]pyrimidine-5-carboxylic acid

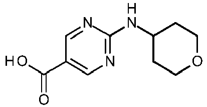

2-Chloropyrimidine-5-carboxylic acid (500mg, 3.15mmol), Et₃N (1.15mL, 8.20mmol) and 4-aminotetrahydropyran (335mg, 3.31 mmol) were dissolved in dioxane (10mL) and heated in a microwave reactor at 150°C for 30min. The reaction mixture was concentrated *in vacuo* to give the title compound (701mg, crude) as a beige solid. LCMS (ES⁺): 224.1 [MH]⁺.

### INTERMEDIATES 39-50

Intermediates 39-50 were prepared similarly to Intermediate 38, by coupling of with the appropriate carboxylic acid with the appropriate amine; see Table 5 below.

**Table 5: Coupling of with the appropriate carboxylic acid with the appropriate amine.**

| | | | |
|---|---|---|---|
| 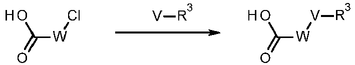 | | | |

| **Int** | **Structure** | **Name** | **Form, Yield, LCMS, HPLC** |
|---|---|---|---|
| 39 | 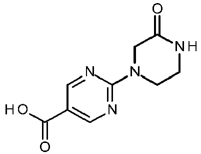 | 2-(3-Oxopiperazin-1-yl)pyrimidine-5-carboxylic acid | Beige solid |
| | | | Yield 701mg, crude |
| | | | LCMS (ES⁺): 223.0 [MH]⁺ |
| 40 | 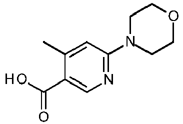 | 4-Methyl-6-(morpholin-4-yl)pyridine-3-carboxylic acid | Off white solid |
| | | | Yield 401mg, 31.0% |
| | | | LCMS (ES⁺): 223.1 [MH]⁺ |
| | | | HPLC: Rt 3.12min, 80.5% purity |
| 41 | 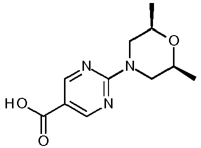 | 2-[(2R,6S)-2,6-Dimethylmorpholin-4-yl]pyrimidine-5-carboxylic acid | Yellow gum |
| | | | Yield 750mg, 100% |
| | | | LCMS (ES⁺): 238.1 [MH]⁺ |
| | | | HPLC: Rt 4.75min, 96.5% purity |
| 42 | 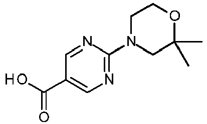 | 2-(2,2-Dimethylmorpholin-4-yl)pyrimidine-5-carboxylic acid | Yellow gum |
| | | | Yield 224mg, 99.8% |
| | | | LCMS (ES⁺): 238.1 [MH]⁺ |
| | | | HPLC: Rt 4.57min, 96.2% purity |
| 43 | 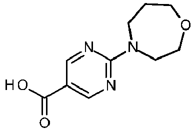 | 2-(1,4-Oxazepan-4-yl)pyrimidine-5-carboxylic acid | Beige solid |
| | | | Yield 1.50mg, 100% |
| | | | LCMS (ES⁺): 224.1 [MH]⁺ |
| 44 | 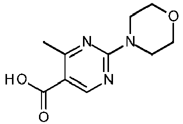 | 4-Methyl-2-(morpholin-4-yl)pyrimidine-5-carboxylic acid | Yellow solid |
| | | | Yield 540mg, 83.5% |
| | | | LCMS (ES⁺): 224.1 [MH]⁺ |
| | | | HPLC: Rt 4.28min, 98.2% purity |
| 45 | 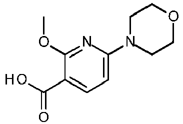 | 2-Methoxy-6-(morpholin-4-yl)pyridine-3-carboxylic acid | White solid |
| | | | Yield 151mg, 23.8% |
| | | | LCMS (ES⁺): 239.1 [MH]⁺ |
| | | | HPLC: Rt 4.55min, 84.0% purity |
| 46 | 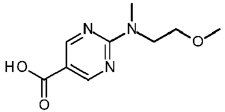 | 2-[(2-methoxyethyl)(methyl)amino]pyrimidine-5-carboxylic acid | Pale orange solid |
| | | | Yield 752mg, crude |
| | | | LCMS (ES⁺): 212.0 [MH]⁺ |
| | | | HPLC: Rt 4.10min, 94.9% purity |
| 47 | 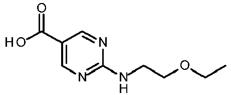 | 2-[(2-Ethoxyethyl)amino]pyrimidine-5-carboxylic acid | Orange solid |
| | | | Yield 1.34g, crude |
| | | | LCMS (ES⁺): 212.1 [MH]⁺ |
| | | | HPLC: Rt 3.84min, 87.0% purity |
| 48 | 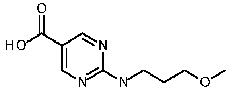 | 2-[(3-Methoxypropyl)amino]pyrimidine-5-carboxylic acid | Pale yellow solid |
| | | | Yield 758mg, crude |
| | | | LCMS (ES⁺): 212.1 [MH]⁺ |
| | | | HPLC: Rt 3.67min, 97.7% purity |
| 49 | 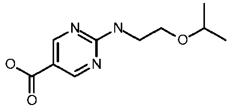 | 2-{[2-(Propan-2-yloxy)ethyl]amino}pyrimidine-5-carboxylic acid | Orange solid |
| | | | Yield 1.79g, crude |
| | | | LCMS (ES⁺): 226.1 [MH]⁺ |
| | | | HPLC: Rt 4.25min, 98.4% purity |
| 50 | 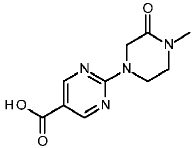 | 2-(4-Methyl-3-oxopiperazin-1-yl)pyrimidine-5-carboxylic acid | White solid |
| | | | Yield 1.95g, 87.0% |
| | | | LCMS (ES⁺): 237.1 [MH]⁺ |
| | | | HPLC: Rt 3.63min, 99.8% purity |

### INTERMEDIATE 51

### 6-[(Morpholin-4-yl)carbonyl]pyridine-3-carboxylic acid

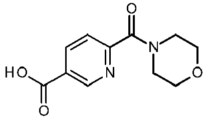

Intermediate 26 (914mg, 3.65mmol) was dissolved in THF/water (24mL, 1:1), lithium hydroxide monohydrate (184mg, 4.38mmol) was added and the reaction mixture was stirred for 20min. 1M aq. HCI (5mL) was added and the reaction mixture was extracted with EtOAc (2x100mL), dried (MgSO₄) and concentrated *in vacuo* to give the title compound (633mg, 73.4%) as a white solid. LCMS (ES⁺): 237.1 [MH]⁺.

### INTERMEDIATES 52-62

Intermediates 52-62 were prepared similarly to Intermediate 51, by LiOH mediated ester hydrolysis; see Table 6 below.

**Table 6: Ester hydrolyses**

| | | | |
|---|---|---|---|
| 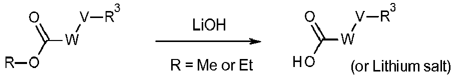 | | | |

| **Int** | **Structure** | **Name** | **Intermediate(s), Form, Yield, LCMS, HPLC** |
|---|---|---|---|
| 52 | 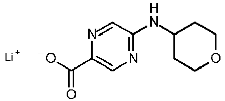 | Lithium 5-[(oxan-4-yl)amino]pyrazine-2-carboxylate | From Intermediate 28 |
| | | | Yellow solid |
| | | | Yield 222mg, 100% |
| | | | LCMS (ES⁺): 224.1 [MH]⁺ |
| 53 | 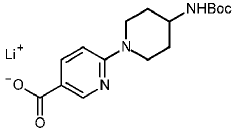 | Lithium 6-(4-{[(*tert*-butoxy) carbonyl]amino}piperidin-1-yl)pyridine-3-carboxylate | From Intermediate 29 |
| | | | White solid |
| | | | Used crude |
| | | | LCMS (ES⁺): 322.1 [MH]⁺ HPLC: Rt 4.20min, 96.8% purity |
| 54 | 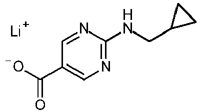 | Lithium 2-[(cyclopropylmethyl)amino]pyrimidine-5-carboxylate | From Intermediate 30 |
| | | | Off white solid |
| | | | Used crude |
| | | | LCMS (ES⁺): 194.1 [MH]⁺ HPLC: Rt 4.09min, 97.4% purity |
| 55 | 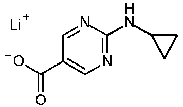 | Lithium 2-(cyclopropylamino)pyrimidine-5-carboxylate | From Intermediate 31 |
| | | | Off white solid |
| | | | Used crude |
| | | | LCMS (ES⁺): 180.1 [MH]⁺ HPLC: Rt 3.23min, 100% purity |
| 56 | 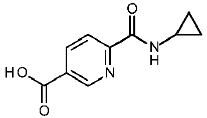 | 6-(Cyclopropylcarbamoyl)pyridine-3-carboxylic acid | From Intermediate 27 |
| | | | Pink solid |
| | | | Yield 559mg, 77.1% |
| | | | LCMS (ES⁺): 207.1 [MH]⁺ |
| 57 | 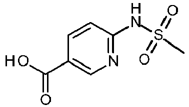 | 6-Methanesulfonamidopyridine-3-carboxylic acid | From Intermediate 37 |
| | | | Beige solid |
| | | | Yield 737mg, 76.4% |
| | | | LCMS (ES⁺): 217.0 [MH]⁺ |
| 58 | 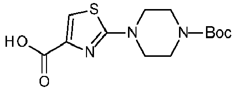 | 2-{4-[(*tert*-Butoxy)carbonyl]piperazin-1-yl}-1,3-thiazole-4-carboxylic acid | From Intermediate 33 |
| | | | White solid |
| | | | Yield 275mg, 88.7% |
| | | | LCMS (ES⁺): 336.1 [MNa]⁺ HPLC: Rt 5.12min, 100% purity |
| 59 | 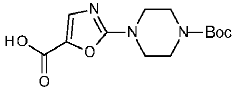 | 2-{4-[(*tert*-Butoxy)carbonyl]piperazin-1-yl}-1,3-oxazole-5-carboxylic acid | From Intermediate 34 |
| | | | White solid |
| | | | Yield 324mg, 84.4% |
| | | | LCMS (ES⁺): 320.1 [MNa]⁺ HPLC: Rt 4.77min, 100% purity |
| 60 | 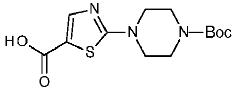 | 2-{4-[(*tert*-Butoxy)carbonyl]piperazin-1-yl}-1,3-thiazole-5-carboxylic acid | From Intermediate 35 |
| | | | White solid |
| | | | Yield 656mg, 97.9% |
| | | | LCMS (ES⁺): 336.1 [MNa]⁺ HPLC: Rt 5.19min, 99.3% purity |
| 61 | 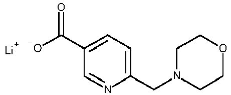 | Lithium 6-(morpholin-4-yl methyl)pyridine-3-carboxylate | From Intermediate 36 |
| | | | Yellow solid |
| | | | Used crude |
| | | | LCMS (ES⁺):223.1 [MH]⁺ |
| 62 | 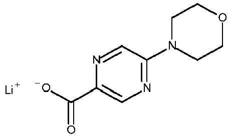 | Lithium 5-(morpholin-4-yl)pyrazine-2-carboxylate | From Intermediate 32 |
| | | | Yellow solid |
| | | | Used crude |
| | | | LCMS (ES⁺): 210.1 [MH]⁺ |

### INTERMEDIATE 63

### 6-(3,6-Dihydro-2H-pyran-4-yl)pyridazine-3-carboxylic acid

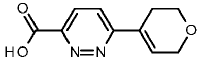

Methyl 6-chloropyridazine-3-carboxylate (1.00g, 5.79mmol), 4-(4,4,5,5-tetramethyl-1,3,2-dioxaborolan-2-yl)-3,6-dihydro-2H-pyran (1.22g, 5.79mmol), Pd(PPh₃)₄ (536mg, 0.464mmol) and Cs₂CO₃ (3.40g, 10.4mmol) were suspended in dioxane (8mL) and water (8mL) and heated in a microwave reactor at 125°C for 30min. 1M aq. HCI (10mL) was added, the precipitate was removed by filtration and the filtrate was concentrated *in vacuo.* The residue was passed through a silica pad eluting with 30% MeOH in DCM and concentrated *in vacuo* to give the title compound as a white solid (946mg, 79.2%). LCMS (ES⁺): 207.1 [MH]⁺. HPLC: Rt 3.30min, 49.9% purity.

### INTERMEDIATE 64

### 2-(Dimethylamino)pyrimidine-5-carbaldehyde

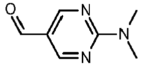

2-Chloropyrimidine-5-carbaldehyde (412mg, 2.89mmol) and Et₃N (482uL, 3.47mmol) were dissolved in dioxane (20mL) and a solution of Me₂NH in THF (1.59mL, 2.0M, 3.18mmol) was added. The reaction mixture was stirred for 1h, filtered, washed with dioxane (5mL), and concentrated *in vacuo* to give the title compound (427mg, 97.7%) as a yellow solid. LCMS (ES⁺): 152.2 [MH]⁺. HPLC: Rt 4.14min, 97.9% purity.

### INTERMEDIATE 65

### 6-(2-Methylmorpholin-4-yl)pyridine-3-carbaldehyde

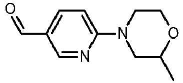

2-Chloro-5-pyridinecarboxaldehyde (500mg, 3.53mmol) and 2-methylmorpholine (750mg, 7.42mmol) were dissolved in DMF (2mL) and the reaction mixture was heated at 100°C in a microwave reactor for 20min and concentrated *in vacuo.* The residue was suspended in dioxane (5mL), filtered and concentrated *in vacuo* to give the title compound (730mg, 100%) as an orange gum. LCMS (ES⁺): 207.1 [MH]⁺.

### INTERMEDIATE 66

### N-{3-[(4-Chlorophenyl)amino]pyridin-4-yl}pyridine-3-carboxamide

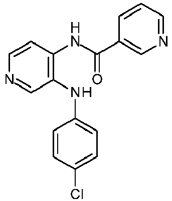

Intermediate 19 (234mg, 1.07mmol), pyridine-4-carboxylic acid (393mg, 3.20mmol) and DIPEA (741uL, 4.26mmol) were dissolved in DMF (10mL) and EDC (613mg, 3.20mmol) was added. The reaction mixture was stirred for 18h and further pyridine-3-carboxylic acid (393mg, 3.20mmol) and EDC (613mg, 3.20mmol) were added. The reaction mixture was stirred for 5h, diluted with 1M aq. Na₂CO₃ (50mL) and extracted into DCM (3x50mL). The combined organic fractions were dried (MgSO₄) and concentrated *in vacuo.* The residue was purified by column chromatography to give the title compound as a red gum (297mg, 85.8%). LCMS (ES⁺): 325.1 [MH]⁺. HPLC: Rt 4.08min, 99.0% purity.

### INTERMEDIATES 67-123

Intermediates 67-123 were prepared similarly to Intermediate 66, by coupling of Intermediates 19-25 with the appropriate carboxylic acid; see Table 7 below.

**Table 7: Amide couplings**

| | | | |
|---|---|---|---|
| 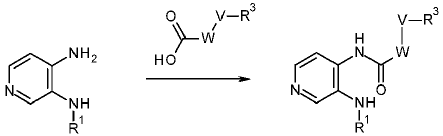 | | | |

| **Int** | **Structure** | **Name** | **Intermediate(s), Form, Yield, LCMS, HPLC** |
|---|---|---|---|
| 67 | 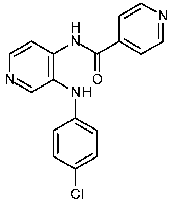 | *N*-{3-[(4-Chlorophenyl)amino]pyridin-4-yl}pyridine-4-carboxamide | From Intermediate 19 |
| | | | Yellow solid |
| | | | Yield 219mg, 58.3% |
| | | | LCMS (ES⁺): 325.2 [MH]⁺ |
| | | | HPLC: Rt 4.18min, 95.8% purity |
| 68 | 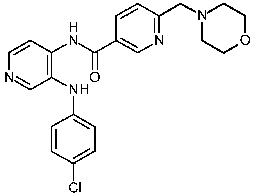 | *N*-{3-[(4-Chlorophenyl)amino]pyridin-4-yl}-6-(morpholin-4-ylmethyl)pyridine-3-carboxamide | From Intermediates 19 and 61 |
| | | | Yellow solid |
| | | | Yield 282mg, 21.1% |
| | | | LCMS (ES⁺): 424.1 [MH]⁺ |
| 69 | 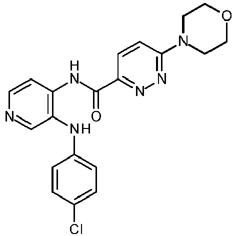 | *N*-{3-[(4-Chlorophenyl)amino]pyridin-4-yl}-6-(morpholin-4-yl)pyridazine-3-carboxamide | From Intermediate 19 |
| | | | Yellow solid |
| | | | Yield 500mg, 58.2% |
| | | | LCMS (ES⁺): 411.0 [MH]⁺ |
| 70 | 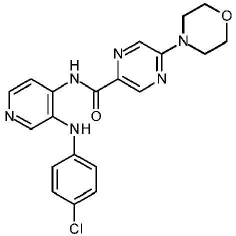 | *N*-{3-[(4-Chlorophenyl)amino]pyridin-4-yl}-5-(morpholin-4-yl)pyrazine-2-carboxamide | From Intermediates 19 and 62 |
| | | | Yellow oil |
| | | | Yield 633mg, 73.5% |
| | | | LCMS (ES⁺): 411.0 [MH]⁺ |
| 71 | 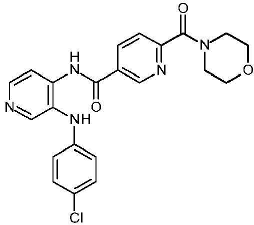 | *N*-{3-[(4-Chlorophenyl)amino]pyridin-4-yl}-6-[(morpholin-4-yl)carbonyl]pyridine-3-carboxamide | From Intermediates 19 and 51 |
| | | | Yellow oil |
| | | | Yield 437mg, 54.9% |
| | | | LCMS (ES⁺): 438.0 [MH]⁺ |
| 72 | 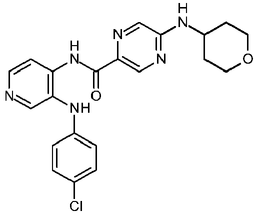 | *N*-{3-[(4-Chlorophenyl)amino]pyridin-4-yl}-5-[(oxan-4-yl)amino]pyrazine-2-carboxamide | From Intermediates 19 and 52 |
| | | | Yellow oil |
| | | | Yield 174mg, 45.6% |
| | | | LCMS (ES⁺): 425.1 [MH]⁺ |
| 73 | 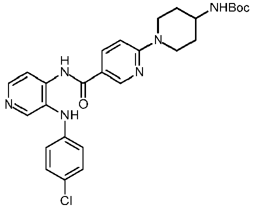 | *tert*-Butyl *N*-{1-[5-({3-[(4-chlorophenyl)amino]pyridin-4-yl}carbamoyl)pyridin-2-yl]piperidin-4-yl}carbamate | From Intermediates 19 and 53 |
| | | | Off white solid |
| | | | Yield 954mg, 76.4% |
| | | | LCMS (ES⁺): 523.1 [MH]⁺ |
| | | | HPLC: Rt 5.16min, 97.8% purity |
| 74 | 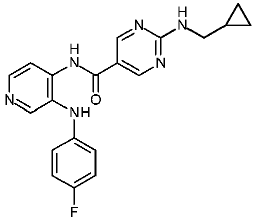 | 2-[(Cyclopropylmethyl)amino]-*N*-{3-[(4-fluorophenyl)amino]pyridin-4-yl}pyrimidine-5-carboxamide | From Intermediates 20 and 54 |
| | | | Yellow solid |
| | | | Yield 312mg, 32.3% |
| | | | LCMS (ES⁺): 379.2 [MH]⁺ |
| | | | HPLC: Rt 4.91min, 96.3% purity |
| 75 | 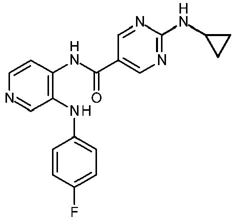 | 2-(Cyclopropylamino)-*N*-{3-[(4-fluorophenyl)amino]pyridin-4-yl}pyrimidine-5-carboxamide | From Intermediates 20 and 55 |
| | | | Yellow solid |
| | | | Yield 659mg, 71.2% |
| | | | LCMS (ES⁺): 365.1 [MH]⁺ |
| | | | HPLC: Rt 4.41min, 68.3% purity |
| 76 | 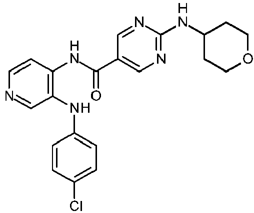 | *N*-{3-[(4-Chlorophenyl)amino]pyridin-4-yl}-2-[(oxan-4-yl)amino]pyrimidine-5-carboxamide | From Intermediates 19 and 38 |
| | | | Yellow oil |
| | | | Yield 917mg, 86.3% |
| | | | LCMS (ES⁺): 425.1 [MH]⁺ |
| 77 | 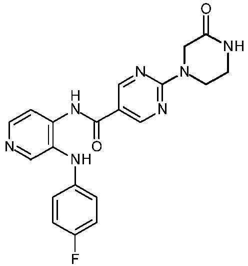 | *N*-{3-[(4-Fluorophenyl)amino]pyridin-4-yl}-2-(3-oxopiperazin-1-yl)pyrimidine-5-carboxamide | From Intermediates 20 and 39 |
| | | | Yellow gum |
| | | | Used crude (1.88g) |
| | | | LCMS (ES⁺): 408.1 [MH]⁺ |
| 78 | 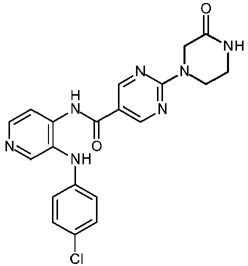 | *N*-{3-[(4-Chlorophenyl)amino]pyridin-4-yl}-2-(3-oxopiperazin-1-yl)pyrimidine-5-carboxamide | From Intermediates 19 and 39 |
| | | | Yellow solid |
| | | | Yield 712mg, 58.8% |
| | | | LCMS (ES⁺): 424.1 [MH]⁺ |
| 79 | 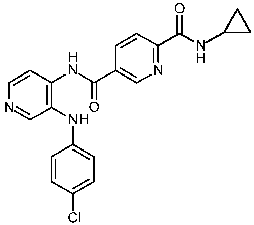 | 5-*N*-{3-[(4-Chlorophenyl)amino]pyridin-4-yl}-2-*N*-cyclopropylpyridine-2,5-dicarboxamide | From Intermediates 19 and 56 |
| | | | Yellow solid |
| | | | Yield 790mg, 84.9% |
| | | | LCMS (ES⁺): 408.1 [MH]⁺ |
| 80 | 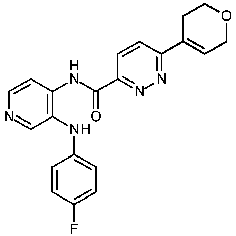 | 6-(3,6-Dihydro-2H-pyran-4-yl)-*N*-{3-[(4-fluorophenyl)amino]pyridin-4-yl}pyridazine-3-carboxamide | From Intermediates 20 and 63 |
| | | | Orange semi-solid |
| | | | Yield 467mg, 54.7% |
| | | | LCMS (ES⁺): 392.2 [MH]⁺ |
| | | | HPLC: Rt 4.87min, 50.9% purity |
| 81 | 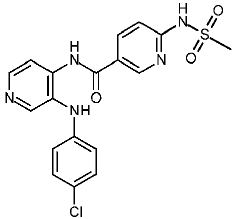 | *N*-{3-[(4-Chlorophenyl)amino]pyridin-4-yl}-6-methanesulfonamidopyridine-3-carboxamide | From Intermediates 19 and 57 |
| | | | Beige solid |
| | | | Yield 348mg, 40.6% |
| | | | LCMS (ES⁺): 418.0 [MH]⁺ |
| 82 | 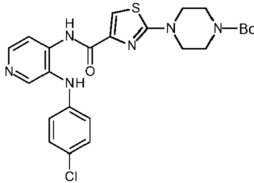 | *tert*-Butyl 4-[4-({3-[(4-chlorophenyl)amino]pyridin-4-yl}carbamoyl)-1,3-thiazol-2-yl]piperazine-1-carboxylate | From Intermediates 19 and 58 |
| | | | White solid |
| | | | Yield 302mg, 66.8% |
| | | | LCMS (ES⁺): 515.0 [MH]⁺ |
| | | | HPLC: Rt 6.37min, 94.2% purity |
| 83 | 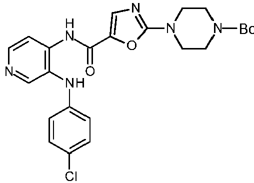 | *tert*-Butyl 4-[5-({3-[(4-chlorophenyl)amino]pyridin-4-yl}carbamoyl)-1,3-oxazol-2-yl]piperazine-1-carboxylate | From Intermediates 19 and 59 |
| | | | Orange solid |
| | | | Used crude (602mg) |
| | | | LCMS (ES⁺): 499.0 [MH]⁺ |
| | | | HPLC: Rt 5.76min, 76.4% purity |
| 84 | 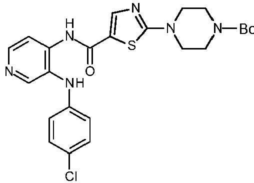 | *tert*-Butyl 4-[5-({3-[(4-chlorophenyl)amino]pyridin-4-yl}carbamoyl)-1,3-thiazol-2-yl]piperazine-1-carboxylate | From Intermediates 19 and 60 |
| | | | Yellow solid |
| | | | Yield 417mg, 39.2% |
| | | | LCMS (ES⁺): 515.1 [MH]⁺ |
| | | | HPLC: Rt 5.95min, 39.1% purity |
| 85 | 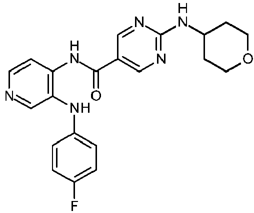 | *N*-{3-[(4-fluorophenyl)amino]pyridin-4-yl}-2-[(oxan-4-yl)amino]pyrimidine-5-carboxamide | From Intermediates 20 and 38 |
| | | | Beige solid |
| | | | Yield 593mg, 59.0% |
| | | | LCMS (ES⁺): 409.1 [MH]⁺ |
| 86 | 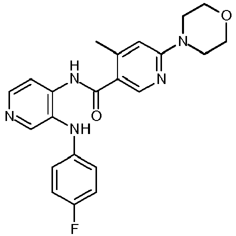 | *N*-{3-[(4-Fluorophenyl)amino]pyridin-4-yl}-4-methyl-6-(morpholin-4-yl)pyridine-3-carboxamide | From Intermediates 20 and 40 |
| | | | Light brown oil |
| | | | Yield 645mg, crude |
| | | | LCMS (ES⁺): 408.2 [MH]⁺ |
| 87 | 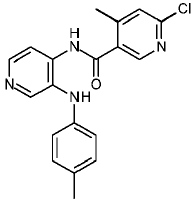 | 6-Chloro-4-methyl-*N*-{3-[(4-methylphenyl)amino]pyridin-4-yl}pyridine-3-carboxamide | From Intermediates 21 Dark oil |
| | | | used crude |
| | | | LCMS (ES⁺): 353.0 [MH]⁺ |
| 88 | 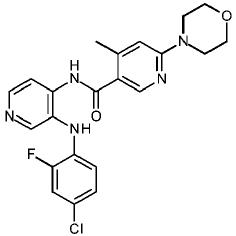 | *N*-{3-[(4-Chloro-2-fluorophenyl)amino]pyridin-4-yl}-4-methyl-6-(morpholin-4-yl)pyridine-3-carboxamide | From Intermediates 22 and 40 |
| | | | Orange oil |
| | | | used crude |
| | | | LCMS (ES⁺): 442.1 [MH⁺] |
| 89 | 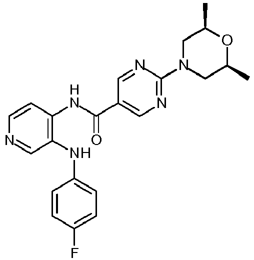 | 2-[(2R,6S)-2,6-Dimethylmorpholin-4-yl]-*N*-{3-[(4-fluorophenyl)amino]pyridin-4-yl}pyrimidine-5-carboxamide | From Intermediates 20 and 41 |
| | | | Yellow gum |
| | | | Yield 422mg, 79.0% |
| | | | LCMS (ES⁺): 423.1 [MH]⁺ |
| | | | HPLC: Rt 5.25min, 67.8% purity |
| 90 | 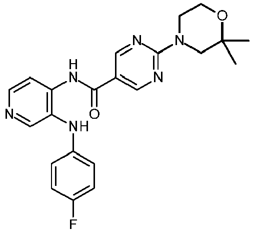 | 2-(2,2-Dimethylmorpholin-4-yl)-*N*-{3-[(4-fluorophenyl)amino]pyridin-4-yl}pyrimidine-5-carboxamide | From Intermediates 20 and 42 |
| | | | Yellow gum |
| | | | Yield 338mg, 84.7% |
| | | | LCMS (ES⁺): 423.1 [MH]⁺ |
| | | | HPLC: Rt 5.12min, 75.5% purity |
| 91 | 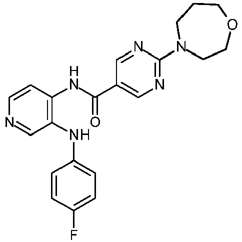 | *N*-{3-[(4-Fluorophenyl)amino]pyridin-4-yl}-2-(1,4-oxazepan-4-yl)pyrimidine-5-carboxamide | From Intermediates 20 and 43 |
| | | | Yellow solid |
| | | | Yield 1.96g, 97.7% |
| | | | LCMS (ES⁺): 409.2 [MH]⁺ |
| 92 | 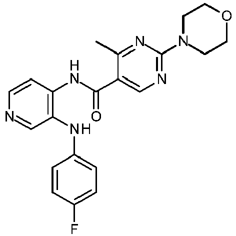 | *N*-{3-[(4-Fluorophenyl)amino]pyridin-4-yl}-4-methyl-2-(morpholin-4-yl)pyrimidine-5-carboxamide | From Intermediates 20 and 44 |
| | | | Orange oil |
| | | | used crude |
| | | | LCMS (ES⁺): 409.2 [MH]⁺ |
| 93 | 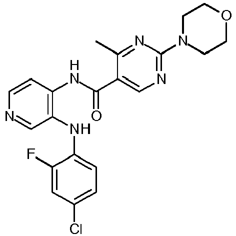 | *N*-{3-[(4-Chloro-2-fluorophenyl)amino]pyridin-4-yl}-4-methyl-2-(morpholin-4-yl)pyrimidine-5-carboxamide | From Intermediates 22 and 44 |
| | | | Orange oil |
| | | | used crude |
| | | | LCMS (ES⁺): 443.1 [MH]⁺ |
| 94 | 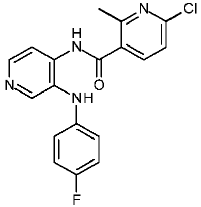 | 6-Chloro-*N*-{3-[(4-fluorophenyl)amino]pyridin-4-yl}-2-methylpyridine-3-carboxamide | From Intermediate 20 |
| | | | Orange oil |
| | | | used crude |
| | | | LCMS (ES⁺): 357.1 [MH]⁺ |
| 95 | 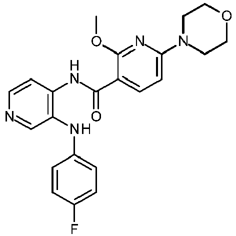 | *N*-{3-[(4-Fluorophenyl)amino]pyridin-4-yl}-2-methoxy-6-(morpholin-4-yl)pyridine-3-carboxamide | From Intermediates 20 and 45 |
| | | | Yellow solid |
| | | | Yield 119mg, 44.3% |
| | | | LCMS (ES⁺): 424.1 [MH]⁺ |
| | | | HPLC: Rt 5.46min, 100% purity |
| 96 | 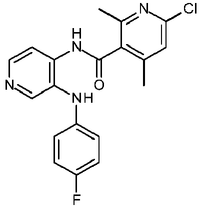 | 6-Chloro-*N*-{3-[(4-fluorophenyl)amino]pyridin-4-yl}-2,4-dimethylpyridine-3-carboxamide | From Intermediate 20 |
| | | | used crude |
| | | | LCMS (ES⁺): 371.0 [MH]⁺ |
| 97 | 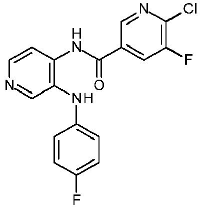 | 6-Chloro-5-fluoro-*N*-{3-[(4-fluorophenyl)amino]pyridin-4-yl}pyridine-3-carboxamide | From Intermediate 20 |
| | | | used crude |
| | | | LCMS (ES⁺): 360.9 [MH]⁺ |
| 98 | 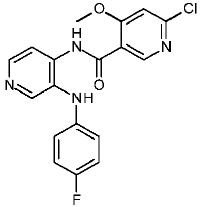 | 6-Chloro-*N*-{3-[(4-fluorophenyl)amino]pyridin-4-yl}-4-methoxypyridine-3-carboxamide | From Intermediate 20 |
| | | | used crude |
| | | | LCMS (ES⁺): 373.0 [MH] |
| 99 | 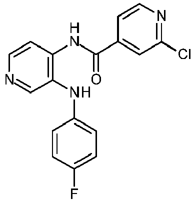 | 2-Chloro-*N*-{3-[(4-fluorophenyl)amino]pyridin-4-yl}pyridine-4-carboxamide | From Intermediate 20 |
| | | | Yellow solid |
| | | | Yield 608mg, 76.6% |
| | | | LCMS (ES⁺): 343.1 [MH]⁺ |
| 100 | 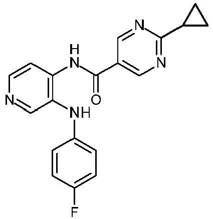 | 2-Cyclopropyl-*N*-{3-[(4-fluorophenyl)amino]pyridin-4-yl}pyrimidine-5-carboxamide | From Intermediate 20 |
| | | | Brown gum |
| | | | Yield 519mg, crude |
| | | | LCMS (ES⁺): 349.8 [MH]⁺ |
| 101 | 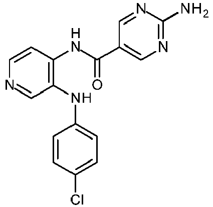 | 2-Amino-*N*-{3-[(4-chlorophenyl)amino]pyridin-4-yl}pyrimidine-5-carboxamide | From Intermediate 19 |
| | | | Orange oil |
| | | | used crude |
| | | | LCMS (ES⁺): 340.7 [MH]⁺ |
| 102 | 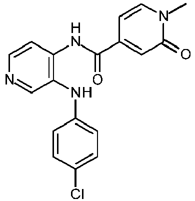 | *N*-{3-[(4-Chlorophenyl)amino]pyridin-4-yl}-1-methyl-2-oxo-1,2-dihydropyridine-4-carboxamide | From Intermediate 19 |
| | | | Orange oil |
| | | | used crude |
| | | | LCMS (ES⁺): 355.1 [MH]⁺ |
| 103 | 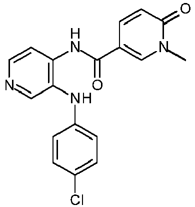 | *N*-{3-[(4-Chlorophenyl)amino]pyridin-4-yl}-1-methyl-6-oxo-1,6-dihydropyridine-3-carboxamide | From Intermediate 19 |
| | | | Orange oil |
| | | | used crude |
| | | | LCMS (ES⁺): 355.1 [MH]⁺ |
| 104 | 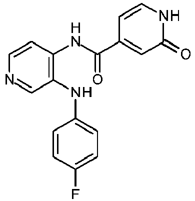 | *N*-{3-[(4-Fluorophenyl)amino]pyridin-4-yl}-2-oxo-1,2-dihydropyridine-4-carboxamide | From Intermediate 20 |
| | | | Orange oil |
| | | | used crude |
| | | | LCMS (ES⁺): 325.1 [MH]⁺ |
| 105 | 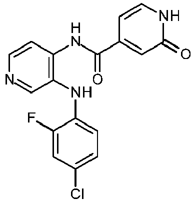 | *N*-{3-[(4-Chloro-2-fluorophenyl)amino]pyridin-4-yl}-2-oxo-1,2-dihydropyridine-4-carboxamide | From Intermediate 22 |
| | | | Orange oil |
| | | | used crude |
| | | | LCMS (ES⁺): 359.1 [MH]⁺ |
| 106 | 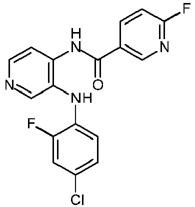 | *N*-{3-[(4-Chloro-2-fluorophenyl)amino]pyridin-4-yl}-6-fluoropyridine-3-carboxamide | From Intermediate 22 |
| | | | Orange oil |
| | | | used crude |
| | | | LCMS (ES⁺): 361.1 [MH]⁺ |
| 107 | 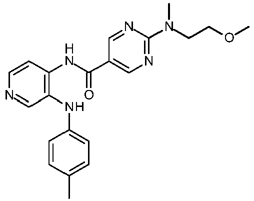 | 2-[(2-Methoxyethyl)(methyl)amino]-*N*-{3-[(4-methylphenyl)amino]pyridin-4-yl}pyrimidine-5-carboxamide | From Intermediates 21 and 46 |
| | | | Dark oil |
| | | | used crude |
| | | | LCMS (ES⁺): 393.0 [MH]⁺ |
| 108 | 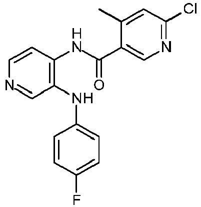 | 6-Chloro-*N*-{3-[(4-fluorophenyl)amino]pyridin-4-yl}-4-methylpyridine-3-carboxamide | From Intermediate 20 |
| | | | used crude |
| | | | LCMS (ES⁺): 357.3 [MH]⁺ |
| 109 | 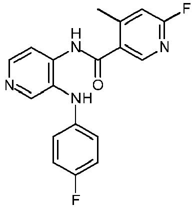 | 6-Fluoro-*N*-{3-[(4-fluorophenyl)amino]pyridin-4-yl}-4-methylpyridine-3-carboxamide | From Intermediate 20 |
| | | | Pale yellow solid |
| | | | Yield 1.11g, 66.0% |
| | | | LCMS (ES⁺): 341.0 [MH]⁺ |
| 110 | 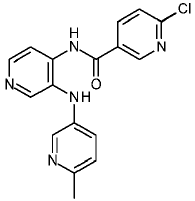 | 6-Chloro-*N*-{3-[(6-methylpyridin-3-yl)amino]pyridin-4-yl}pyridine-3-carboxamide | From Intermediate 23 |
| | | | Dark oil |
| | | | used crude |
| | | | LCMS (ES⁺): 340.0 [MH]⁺ |
| 111 | 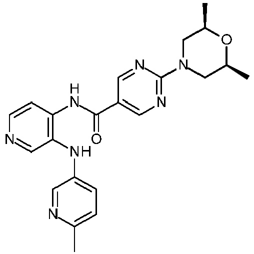 | 2-[(2R,6S)-2,6-Dimethylmorpholin-4-yl]-*N*-{3-[(6-methylpyridin-3-yl)amino]pyridin-4-yl}pyrimidine-5-carboxamide | From Intermediates 23 and 41 |
| | | | Dark orange oil |
| | | | used crude |
| | | | LCMS (ES⁺): 420.1 [MH]⁺ |
| 112 | 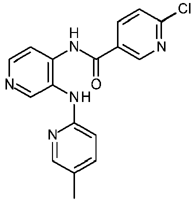 | 6-Chloro-*N*-3-[(5-methylpyridin-2-yl)amino]pyridin-4-yl}pyridine-3-carboxamide | From Intermediate 24 |
| | | | Dark oil |
| | | | used crude |
| | | | LCMS (ES⁺): 340.0 [MH]⁺ |
| 113 | 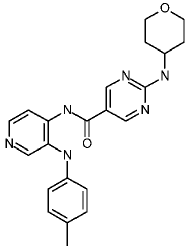 | *N*-{3-[(4-Methylphenyl)amino]pyridin-4-yl}-2-[(oxan-4-yl)amino]pyrimidine-5-carboxamide | From Intermediates 21 and 38 |
| | | | Dark oil |
| | | | used crude |
| | | | LCMS (ES⁺): 405.0 [MH]⁺ |
| 114 | 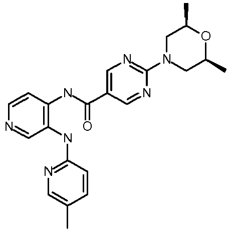 | 2-[(2R,6S)-2,6-Dimethylmorpholin-4-yl]-*N*-{3-[(5-methylpyridin-2-yl)amino]pyridin-4-yl}pyrimidine-5-carboxamide | From Intermediates 24 and 41 |
| | | | Orange oil |
| | | | used crude |
| | | | LCMS (ES⁺): 420.0 [MH]⁺ |
| 115 | 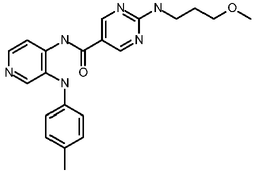 | 2-[(3-Methoxypropyl)amino]-*N-*{3-[(4-methylphenyl)amino]pyridin-4-yl}pyrimidine-5-carboxamide | From Intermediates 21 and 48 |
| | | | Dark oil |
| | | | used crude |
| | | | LCMS (ES⁺): 393.1 [MH]⁺ |
| 116 | 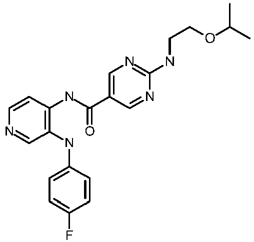 | *N*-{3-[(4-Fluorophenyl)amino]pyridin-4-yl}-2-{[2-(propan-2-yloxy)ethyl]amino}pyrimidine-5-carboxamide | From Intermediates 20 and 49 |
| | | | Dark oil |
| | | | used crude |
| | | | LCMS (ES⁺): 411.0 [MH]⁺ |
| 117 | 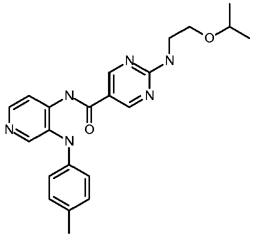 | *N*-{3-[(4-Methylphenyl)amino]pyridin-4-yl}-2-{[2-(propan-2-yloxy)ethyl]amino}pyrimidine-5-carboxamide | From Intermediates 21 and 49 |
| | | | Dark oil |
| | | | used crude |
| | | | LCMS (ES⁺): 407.1 [MH]⁺ |
| 118 | 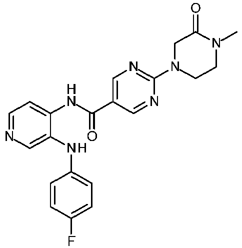 | *N*-{3-[(4-Fluorophenyl)amino]pyridin-4-yl}-2-(4-methyl-3-oxopiperazin-1-yl)pyrimidine-5-carboxamide | From Intermediates 20 and 50 |
| | | | Dark oil |
| | | | used crude |
| | | | LCMS (ES⁺): 422.1 [MH]⁺ |
| 119 | 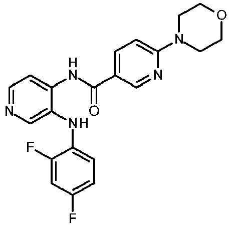 | *N*-{3-[(2,4-Difluorophenyl)amino]pyridin-4-yl}-6-(morpholin-4-yl)pyridine-3-carboxamide | From Intermediate 25 |
| | | | Orange oil |
| | | | used crude |
| | | | LCMS (ES⁺): 412.0 [MH]⁺ |
| 120 | 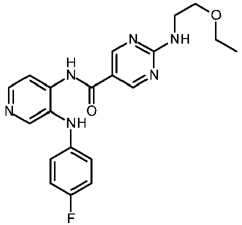 | 2-[(2-Ethoxyethyl)amino]-*N*-{3-[(4-fluorophenyl)amino]pyridin-4-yl}pyrimidine-5-carboxamide | From Intermediate 20 and 47 |
| | | | Orange oil |
| | | | used crude |
| | | | LCMS (ES⁺): 397.1 [MH]⁺ |
| 121 | 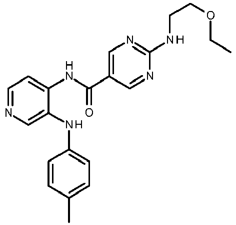 | 2-[(2-Ethoxyethyl)amino]-*N*-{3-[(4-methylphenyl)amino]pyridin-4-yl}pyrimidine-5-carboxamide | From Intermediate 21 and 47 |
| | | | Dark oil |
| | | | used crude |
| | | | LCMS (ES⁺): 393.1 [MH]⁺ |
| 122 | 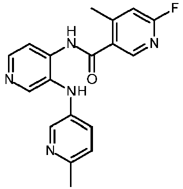 | 6-Fluoro-4-methyl-*N*-{3-[(6-methylpyridin-3-yl)amino]pyridin-4-yl}pyridine-3-carboxamide | From Intermediate 23 |
| | | | Dark oil |
| | | | used crude |
| | | | LCMS (ES⁺): 338.0 [MH]⁺ |
| 123 | 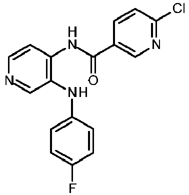 | 6-Chloro-N-{3-[(4-fluorophenyl)amino]pyridin-4-yl}pyridine-3-carboxamide | From Intermediate 20 |
| | | | Yellow solid |
| | | | Yield 1.06g, 63.0% |
| | | | LCMS (ES⁺): 342.9 [MH]⁺ |
| | | | HPLC: Rt 4.86min, 91.2% purity |

### INTERMEDIATE 124

### N-[(4-Fluorophenyl)amino]pyridin-4-yl}-2,4-dimethyl-6-(morpholin-4-yl)pyridine-3-carboxamide

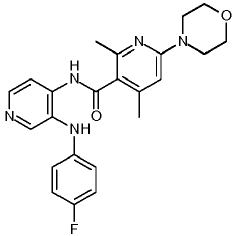

Intermediate 96 (crude) was dissolved in NMP (2mL) and morpholine (1.70mL, 19.7mmol) and Et₃N (708uL, 5.08mmol) were added. The reaction mixture was heated at 190°C in a microwave reactor for 30min and partitioned between EtOAc (50mL) and water (50mL). The organic fraction was washed with water (50mL), brine (50mL), dried (MgSO₄), concentrated *in vacuo* and purified by column chromatography to give the title compound (783mg, 47.1%) as a yellow solid; LCMS (ES⁺): 422.0 [MH]⁺, HPLC: Rt: 3.99min, 83.2% purity.

### INTERMEDIATE 125

### N-13-[(4-Fluorophenyl)amino]pyridin-4-yl}-6-(oxan-4-yl)pyridazine-3-carboxamide

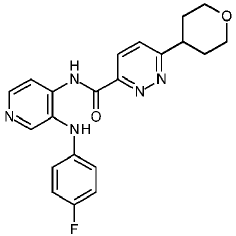

Intermediate 80 (220mg, 0.562mmol) was dissolved in MeOH (10mL), Pd/C (cat) was added and the reaction mixture was stirred under hydrogen for 2h. The reaction mixture was filtered through Celite and concentrated *in vacuo* to give the crude title compound which was used without purification. LCMS (ES⁺): 394.2 [MH] .

### INTERMEDIATE 126

### 1-{5-[3-(4-Fluorophenyl)-3H-imidazo[4,5-c]pyridin-2-yl]pyridin-2-yl}piperazine trihydrochloride

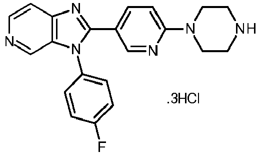

Intermediate 126 was prepared similarly to Example 50, using Intermediate 2 instead of Intermediate 1, to give the title compound (684mg, 100%) as a white solid. LCMS (ES⁺): 375.1 [MH]⁺.

### INTERMEDIATE 127

### 2-Chloro-5-[3-(4-fluorophenyl)-3H-imidazo[4,5-c]pyridin-2-yl]pyridine

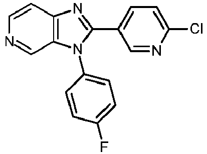

Intermediate 2 (1.00g, 4.01 mmol) and 2-chloro-5-pyridinecarboxaldehyde (682mg, 4.82mmol) were dissolved in EtOH (8mL) and Na₂S₂O₄ (2.79g, 16.1mmol) was added. The reaction mixture was heated in a microwave reactor at 160°C for 1h, diluted with water (25mL) and NaHCO₃ (25mL) and extracted into DCM (3x50mL). The combined organic fractions were dried (MgSO₄) and concentrated *in vacuo.* The residue was purified by column chromatography to give the title compound (375mg, 28.8%) as a yellow oil. LCMS (ES⁺): 325.1 [MH]⁺.

### INTERMEDIATE 128

### 2-Chloro-5-[3-(4-chlorophenyl)-3H-imidazo[4,5-c]pyridin-2-yl]pyridine

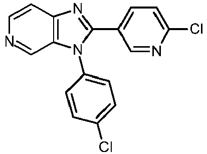

Intermediate 128 was prepared similarly to Intermediate 127, using Intermediate 1 instead of Intermediate 2, to give the title compound (81.0mg, 8.41%) as a yellow solid. LCMS (ES⁺): 341.1 [MH]⁺. HPLC: Rt: 5.10min, 97.0% purity.

### INTERMEDIATE 129

### 1-{5-[3-(4-Fluorophenyl)-3H-imidazo[4,5-c]pyridin-2-yl]pyridin-2-yl}-1,4-diazepane

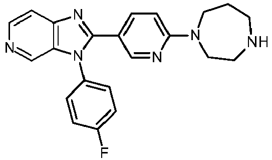

Intermediate 127 (375mg, 1.15mmol) and homopiperazine (578mg, 5.77mmol) were dissolved in DMA (6mL) and the reaction mixture was heated in a microwave reactor at 180°C for 30min and concentrated *in vacuo.* The residue was partitioned between DCM (50mL) and sat. aq. Na₂CO₃ (50mL) and the organic fraction dried (MgSO₄) and concentrated *in vacuo* to give the title compound (410mg, 91.5%) as a red oil. LCMS (ES⁺): 389.2 [MH]⁺.

### INTERMEDIATES 130-133

Intermediates 130-133 were prepared similarly to Example 1, by cyclisation of Intermediates 94, 99, 104 and 108; see Table 8 below.

**Table 8: Cyclisation of Intermediates 94, 99, 104 and 108**

| | | | |
|---|---|---|---|
| 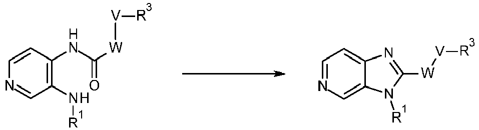 | | | |

| **Ex** | **Structure** | **Name** | **Intermediate(s), Form, Yield, LCMS, HPLC** |
|---|---|---|---|
| 130 | 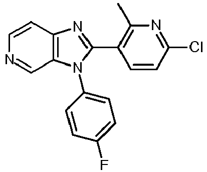 | 6-Chloro-3-[3-(4-fluorophenyl)-3H-imidazo[4,5-c]pyridin-2-yl]-2-methylpyridine | From Intermediate 94 |
| | | | Pale brown gum |
| | | | Yield 123mg, 29.5% |
| | | | LCMS (ES⁺): 339.1 [MH]⁺ |
| | | | HPLC: Rt 4.64min, 74.0% purity |
| 131 | 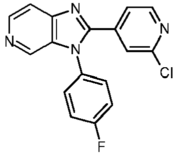 | 2-Chloro-4-[3-(4-fluorophenyl)-3H-imidazo[4,5-c]pyridin-2-yl]pyridine | From Intermediate 99 |
| | | | Yellow solid |
| | | | Yield 397mg, 68.9% |
| | | | LCMS (ES⁺): 325.1 [MH]⁺ |
| | | | HPLC: Rt 4.48min, 80.2% purity |
| 132 | 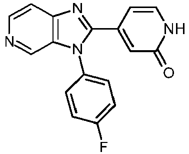 | 4-[3-(4-Fluorophenyl)-3H-imidazo[4,5-c]pyridin-2-yl]-1,2-dihydropyridin-2-one | From Intermediate 104 |
| | | | Pale pink solid |
| | | | Yield 172mg, 22.7% |
| | | | LCMS (ES⁺): 307.2 [MH]⁺ |
| | | | HPLC: Rt 3.42min, 99.7% purity |
| 133 | 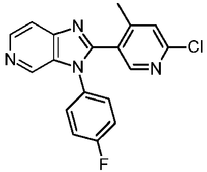 | 2-Chloro-5-[3-(4-fluorophenyl)-3H-imidazo[4,5-c]pyridin-2-yl]-4-methylpyridine | From Intermediate 108 |
| | | | Yellow gum |
| | | | Yield 218mg, 63.1% |
| | | | LCMS (ES⁺): 338.7 [MH]⁺ |

### INTERMEDIATE 134

### 1-(4-Chlorophenyl)-1H-pyrrolo[2,3-c]pyridine

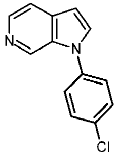

6-Azaindole (5.00g, 42.3mmol) was dissolved in DMF (150mL) under nitrogen and 1-chloro-4-iodo-benzene (12.1g, 50.8mmol), *N,N*-dimethylethylenediamine (911uL, 8.46mmol), K₃PO₄ (18.9g, 88.9mmol) and CuI (806mg, 4.23mmol) were added. The reaction mixture was heated at 150°C for 18h. The reaction mixture was filtered and the filtrate was concentrated *in vacuo.* The residue was suspended in 1M aq. Na₂CO₃ (250mL) and extracted into DCM (2x250mL). The combined organic fractions were dried (MgSO₄) and concentrated *in vacuo.* The residue was purified by column chromatography to give the title compound as a yellow solid (8.58g, 88.6%). LCMS (ES⁺): 229.1 [MH]⁺. HPLC: Rt 4.48min, 98.6% purity.

### INTERMEDIATE 135-138

Intermediates 135-138 were prepared similarly to Intermediate 134, by arylation of 6-azaindole with the appropriate aryl or heteroaryl iodide or bromide; see Table 9 below.

**Table 9: Arylations of 6-azaindole**

| | | | |
|---|---|---|---|
| 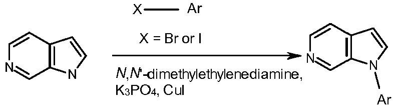 | | | |

| **Ex** | **Structure** | **Name** | **Intermediate(s), Form, Yield, LCMS, HPLC** |
|---|---|---|---|
| 135 | 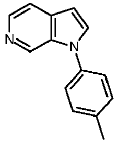 | 1-(4-Methylphenyl)-1H-pyrrolo[2,3-c]pyridine | Green gum |
| | | | Yield 800mg, 45.4% |
| | | | LCMS (ES⁺): 209.1 [MH]⁺ |
| | | | HPLC: Rt 4.65min, 99.7% purity. |
| 136 | 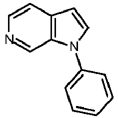 | 1-Phenyl-1H-pyrrolo[2,3-c]pyridine | Green gum |
| | | | Yield 890mg, 54.0% |
| | | | LCMS (ES⁺): 195.1 [MH]⁺ |
| | | | HPLC: Rt 4.19min, 99.6% purity. |
| 137 | 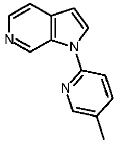 | 5-Methyl-2-{1H-pyrrolo[2,3-c]pyridin-1-yl}pyridine | Light yellow solid |
| | | | Yield 113mg, 6.38% |
| | | | LCMS (ES⁺): 210.1 [MH]⁺ |
| | | | HPLC: Rt 4.45min, 100% purity. |
| 138 | 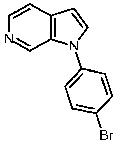 | 1-(4-Bromophenyl)-1H-pyrrolo[2,3-c]pyridine | Green solid |
| | | | Yield 1.14g, 24.7% |
| | | | LCMS (ES⁺): 273.0 [MH]⁺ |
| | | | HPLC: Rt 4.78min, 98.5% purity. |

### INTERMEDIATE 139

### 1-[(4-Iodophenyl)carbonyl]-4-methylpiperazine

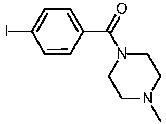

4-lodobenzoic acid (500mg, 2.02mmol) and DMF (50uL) were dissolved in DCM (24mL), oxalyl chloride (182uL, 2.12mmol) was added drop-wise and the reaction mixture was stirred for 30min. DIPEA (421uL, 2.42mmol) and a solution of N-methylpiperazine (222mg, 2.22mmol) in DCM (1mL) were added and the reaction mixture was stirred for 30min, washed with sat. aq. NaHCO₃ (2x75mL), dried (MgSO₄) and concentrated *in vacuo* to give the title compound (660mg, 99.2%) as a yellow solid. LCMS (ES⁺): 331.0 [MH]⁺. HPLC: Rt 4.05min, 99.6% purity.

### INTERMEDIATE 140

### 4-(5-Bromopyrimidin-2-yl)piperazin-2-one

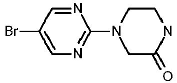

5-Bromo-2-chloropyrimidine (750mg, 3.88mmol), DIPEA (878uL, 5.04mmol) and piperazin-2-one (427mg, 4.26mmol) were dissolved in MeCN (20mL). The reaction mixture was heated at 95°C for 1h then allowed to cool to RT overnight. The resulting solid was collected by filtration, washed with water (2x20mL) to give the title compound (546mg, 54.8%) as a white solid. LCMS (ES⁺): 257.1 and 259.1 [MH]⁺. HPLC: Rt 4.68min, 98.6% purity.

### INTERMEDIATE 141-142

Intermediates 141-142 were prepared similarly to Intermediate 140, by coupling of 5-bromo-2-chloropyrimidine with the appropriate amine; see Table 10 below.

**Table 10: SNAr with 5-bromo-2-chloropyrimidine**

| | | | |
|---|---|---|---|
| 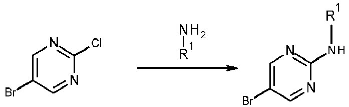 | | | |

| **Ex** | **Structure** | **Name** | **Intermediate(s), Form, Yield, LCMS, HPLC** |
|---|---|---|---|
| 141 | 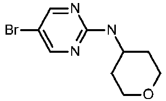 | 5-Bromo-*N*-(oxan-4-yl)pyrimidin-2-amine | White solid |
| | | | Yield 645mg, 48.4% |
| | | | LCMS (ES⁺): 258.0 [MH] |
| 142 | 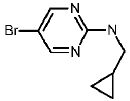 | 5-Bromo-*N-*(cyclopropylmethyl)pyrimidin-2-amine | Light yellow solid |
| | | | Yield 476mg, 80.7% |
| | | | LCMS (ES⁺): 228.1 [MH]⁺ |
| | | | HPLC: Rt 6.48min, 83.9% purity. |

### INTERMEDIATE 143

### 4-(5-Bromo-4-methylpyridin-2-yl)morpholine

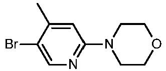

5-Bromo-2-fluoro-4-methylpyridine (1.00g, 5.26mmol) and morpholine (1.38mL, 15.8mmol) were dissolved in MeCN (3.0mL) and the reaction mixture heated by microwave reactor at 140-160°C for 1.5h. The reaction mixture was diluted with EtOAc (40mL), washed with sat. aq. NaHCO₃ (40mL), dried (MgSO₄) and the solvents removed *in vacuo* to give the title compound (1.20g, 88.7%) as a white solid. LCMS (ES⁺): 257.0 [MH]⁺. HPLC: Rt 4.40min, 95.1% purity.

### INTERMEDIATE 144

### 1-Cyclopropyl-4-iodo-1,2-dihydropyridin-2-one

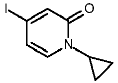

4-lodo-1,2-dihydropyridin-2-one (1.00g, 4.52mmol), copper (II) acetate (879mg, 4.84mmol), 4,4-dipyridyl (756mg, 4.84mmol), cyclopropylboronic acid (875mg, 10.2mmol) and Na₂CO₃ (1.09g, 10.3mmol) in DCE (40mL) was stirred at 70°C for 18h. The reaction was quenched with sat. NH₄Cl (20mL) and water (50mL) and extracted with DCM (2x50mL), dried MgSO₄ and concentrated *in vacuo.* The crude material was purified by column chromatography to give the title compound (585mg, 49.5%) as a yellow oil. LCMS (ES⁺): 262.0 [MH]⁺.

### INTERMEDIATE 145

### 4-[(5-Bromopyridin-2-yl)methyl]morpholine

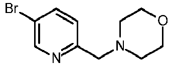

5-Bromo-pyridine-2-carbaldehyde (1.00g, 5.38mmol) and morpholine (464uL, 5.38mmol) were dissolved in DCM (20mL) and NaBH(OAc)₃ (1.71g, 8.06mmol) was added. The reaction mixture was stirred at RT for 4d then heated at 50°C for 7h. Morpholine (464uL, 5.38mmol) and NaBH(OAc)₃ (1.71 g, 8.06mmol) were added and the reaction was heated at 50°C for 16h. Water (50mL) and sat. aq. Na₂CO₃ sol. (50mL) were added and the aqueous fraction was then extracted with DCM (2x100mL) dried (MgSO₄), filtered and the solvent removed *in vacuo.* The crude material was purified by column chromatography to give the title compound (1.31g, 94.8%) as a yellow oil. LCMS (ES⁺): 257.0 [MH]⁺.

### INTERMEDIATE 146

### 4-lodo-1 -methyl-1,2-dihydropyridin-2-one

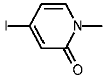

4-lodo-1,2-dihydropyridin-2-one (500mg, 2.26mmol), Mel (296uL, 4.75mmol) and K₂CO₃ (688mg, 4.98mmol) were suspended in MeCN (20mL) and stirred for 18h. The reaction mixture was filtered and the solvents removed *in vacuo.* The residue was purified by column chromatography to give the title compound (411mg, 77.3%) as a white solid. LCMS (ES⁺): 236.0 [MH]⁺. HPLC: Rt 4.48min, 99.6% purity.

### INTERMEDIATE 147

### 1-Ethyl-4-iodo-1,2-dihydropyridin-2-one

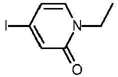

Intermediate 147 was prepared similarly to Intermediate 146, using ethyl iodide instead of methyl iodide, to give the title compound (347mg, 61.6%) as a yellow gum. LCMS (ES⁺): 250.0 [MH]⁺. HPLC: Rt 5.04min, 96.0% purity.

### INTERMEDIATE 148

### 6-Bromo-1-methyl-1,2-dihydropyridin-2-one

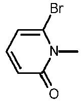

Intermediate 148 was prepared similarly to Intermediate 146, using 2-bromo-6-hydroxypyridine instead of 4-iodo-1,2-dihydropyridin-2-one, to give the title compound (468mg, 86.6%) as an off white solid. LCMS (ES⁺): 188.1 and 190.1 [MH]⁺. HPLC: Rt 4.17min, 98.4% purity.

### EXAMPLE 1

### 3-[3-(4-Chlorophenyl)-3H-imidazo[4,5-c]pyridin-2-yl]pyridine

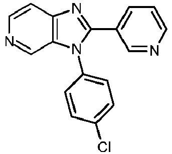

Intermediate 66 (297mg, 0.914mmol) was dissolved in AcOH (5mL) and heated using a microwave reactor at 100°C for 15min, diluted with water (50mL), basified with Na₂CO₃ and extracted into DCM (3x50mL). The combined organic fractions were dried (MgSO₄) and concentrated *in vacuo.* The residue was purified by column chromatography to give the title compound as a white solid (139mg, 49.5%). HRMS (ESI⁺) calcd for [MH]⁺ of C₁₇H₁₁ClN₄ 307.0750 found 307.0748. HPLC: Rt 4.22min, 99.8% purity.

### EXAMPLES 2-43

Examples 2-43 were prepared similarly to Example 1, by cyclisation of Intermediates 67-79, 81-86, 88-93, 95, 100-103, 105-106, 114-121, 124 and 125; see Table 11 below.

**Table 11: Cyclisation of Intermediates 67-79, 81-86, 88-93, 95, 100-103, 105-106, 114-121, 124 and 125**

| | | | |
|---|---|---|---|
| 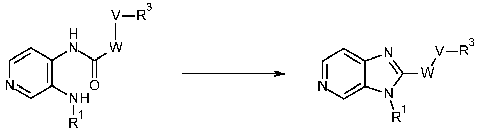 | | | |

| **Ex** | **Structure** | **Name** | **Intermediate(s), Form, Yield, LCMS, HPLC** |
|---|---|---|---|
| 2 | 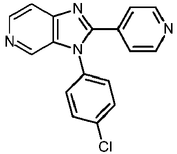 | 4-[3-(4-Chlorophenyl)-3H-imidazo[4,5-c]pyridin-2-yl]pyridine | From Intermediate 67 |
| | | | Light pink solid |
| | | | Yield 109mg, 52.7% |
| | | | HRMS (ESI⁺) calcd for [MH]⁺ of C₁₇H₁₁ClN₄ 307.0750 found 307.0752. |
| | | | HPLC: Rt 3.90min, 99.8% purity |
| 3 | 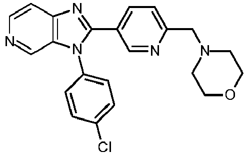 | 4-({5-[3-(4-Chlorophenyl)-3H-imidazo[4,5-c]pyridin-2-yl]pyridin-2-yl}methyl)morpholine | From Intermediate 68 |
| | | | White solid |
| | | | Yield 105mg, 38.6% |
| | | | HRMS (ESI⁺) calcd for [MH]⁺ of C₂₂H₂₀ClN₅O 406.1435 found 406.1428. |
| | | | HPLC: Rt 3.65min, 100% purity |
| 4 | 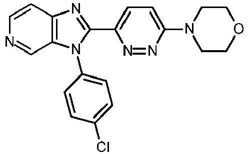 | 4-{6-[3-(4-Chlorophenyl)-3H-imidazo[4,5-c]pyridin-2-yl]pyridazin-3-yl}morpholine | From Intermediate 69 |
| | | | White solid |
| | | | Yield 89.9mg, 18.8% |
| | | | HRMS (ESI⁺) calcd for [MH]⁺ of C₂₀H₁₇ClN₆O 393.1230 found 393.1234. |
| | | | HPLC: Rt 4.71min, 98.4% purity |
| 5 | 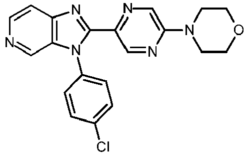 | 4-{5-[3-(4-Chlorophenyl)-3H-imidazo[4,5-c]pyridin-2-yl]pyrazin-2-yl}morpholine | From Intermediate 70 |
| | | | White solid |
| | | | Yield 114mg, 18.9% |
| | | | HRMS (ESI⁺) calcd for [MH]⁺ of C₂₀H₁₇ClN₆O 393.1230 found 393.1234. |
| | | | HPLC: Rt 4.79min, 100% purity |
| 6 | 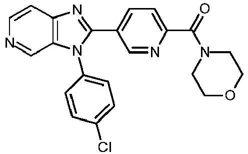 | 4-({5-[3-(4-Chlorophenyl)-3H-imidazo[4,5-c]pyridin-2-yl]pyridin-2-yl}carbonyl)morpholine | From Intermediate 71 |
| | | | White solid |
| | | | Yield 170mg, 40.5% |
| | | | HRMS (ESI⁺) calcd for [MH]⁺ of C₂₂H₁₈ClN₅O₂ 420.1227 found 420.1228. |
| | | | HPLC: Rt 4.03min, 100% purity |
| 7 | 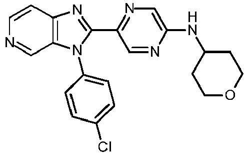 | 5-[3-(4-Chlorophenyl)-3H-imidazo[4,5-c]pyridin-2-yl]-*N-*(oxan-4-yl)pyrazin-2-amine | From Intermediate 72 |
| | | | Yellow solid |
| | | | Yield 50.0mg, 30.0% |
| | | | HRMS (ESI⁺) calcd for [MH]⁺ of C₂₁H₁₉ClN₆O 407.1387 found 407.1380. |
| | | | HPLC: Rt 4.86min, 100% purity |
| 8 | 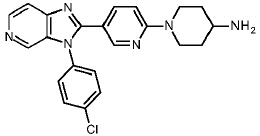 | 1-{5-[3-(4-Chlorophenyl)-3H-imidazo[4,5-c]pyridin-2-yl]pyridin-2-yl}piperidin-4-amine | From Intermediate 73 |
| | | | White solid |
| | | | Yield 24.2mg, 38.7% |
| | | | HRMS (ESI⁺) calcd for [MH]⁺ of C₂₂H₂₁ClN₆ 405.1594 found 405.1591. |
| | | | HPLC: Rt 3.52min, 100% purity |
| 9 | 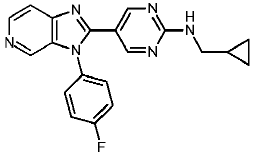 | *N*-(Cyclopropylmethyl)-5-[3-(4-fluorophenyl)-3H-imidazo[4,5-c]pyridin-2-yl]pyrimidin-2-amine | From Intermediate 74 |
| | | | White solid |
| | | | Yield 24.6mg, 8.28% |
| | | | HRMS (ESI⁺) calcd for [MH]⁺ of C₂₀H₁₇FN₆ 361.1577 found 361.1594. |
| | | | HPLC: Rt 5.01min, 97.0% purity |
| 10 | 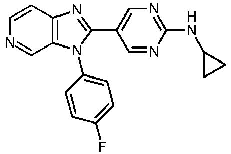 | *N*-Cyclopropyl-5-[3-(4-fluorophenyl)-3H-imidazo[4,5-c]pyridin-2-yl]pyrimidin-2-amine | From Intermediate 75 |
| | | | White solid |
| | | | Yield 30.9mg, 4.93% |
| | | | HRMS (ESI⁺) calcd for [MH]⁺ of C₁₉H₁₅FN₆ 347.1420 found 347.1422. |
| | | | HPLC: Rt 4.46min, 99.6% purity |
| 11 | 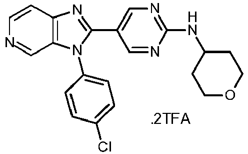 | 5-[3-(4-Chlorophenyl)-3H-imidazo[4,5-c]pyridin-2-yl]-*N-*(oxan-4-yl)pyrimidin-2-amine; bis(trifluoroacetic acid) | From Intermediate 76 |
| | | | White solid |
| | | | Yield 73.8mg, 5.39% |
| | | | HRMS (ESI⁺) calcd for [MH]⁺ of C₂₁H₁₉ClN₆O 407.1387 found 407.1403. |
| | | | HPLC: Rt 4.76min, 100% purity |
| 12 | 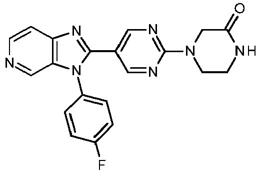 | 4-{5-[3-(4-Fluorophenyl)-3H-imidazo[4,5-c]pyridin-2-yl]pyrimidin-2-yl}piperazin-2-one | From Intermediate 77 |
| | | | White solid |
| | | | Yield 104mg, 9.29% |
| | | | HRMS (ESI⁺) calcd for [MH]⁺ of C₂₀H₁₆FN₇O 390.1479 found 390.1481. |
| | | | HPLC: Rt 4.01min, 100% purity |
| 13 | 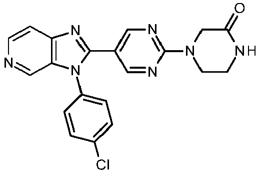 | 4-{5-[3-(4-Chlorophenyl)-3H-imidazo[4,5-c]pyridin-2-yl]pyrimidin-2-yl}piperazin-2-one | From Intermediate 78 |
| | | | White solid |
| | | | Yield 97.1mg, 14.2% |
| | | | HRMS (ESI⁺) calcd for [MH]⁺ of C₂₀H₁₆ClN₇O 406.1183 found 406.1185. |
| | | | HPLC: Rt 4.25min, 99.5% purity |
| 14 | 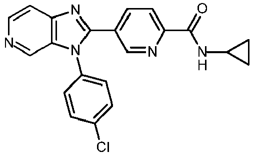 | 5-[3-(4-Chlorophenyl)-3H-imidazo[4,5-c]pyridin-2-yl]-*N-*cyclopropylpyridine-2-carboxamide | From Intermediate 79 |
| | | | White solid |
| | | | Yield 100mg, 13.3% |
| | | | HRMS (ESI⁺) calcd for [MH]⁺ of C₂₁H₁₆ClN₅O 390.1122 found 390.1139. |
| | | | HPLC: Rt 4.81min, 100% purity |
| 15 | 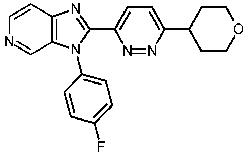 | 3-[3-(4-Fluorophenyl)-3H-imidazo[4,5-c]pyridin-2-yl]-6-(oxan-4-yl)pyridazine | From Intermediate 125 |
| | | | Off white solid |
| | | | Yield 14.0mg, 6.63% |
| | | | HRMS (ESI⁺) calcd for [MH]⁺ of C₂₁H₁₈FN₅O 376.1573 found 376.1575. |
| | | | HPLC: Rt 4.37min, 98.4% purity |
| 16 | 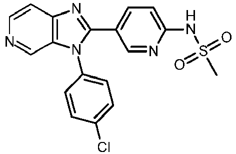 | *N*-{5-[3-(4-Chlorophenyl)-3H-imidazo[4,5-c]pyridin-2-yl]pyridin-2-yl}methanesulfonamide | From Intermediate 81 |
| | | | White solid |
| | | | Yield 163mg, 48.8% |
| | | | HRMS (ESI⁺) calcd for [MH]⁺ of C₁₈H₁₄ClN₅O₂S 400.0635 found 400.0631. |
| | | | HPLC: Rt 4.19min, 100% purity |
| 17 | 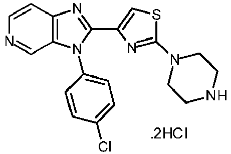 | 1-{4-[3-(4-Chlorophenyl)-3H-imidazo[4,5-c]pyridin-2-yl]-1,3-thiazol-2-yl}piperazine dihydrochloride | From Intermediate 82 |
| | | | Yellow solid |
| | | | Yield 47.3mg, 17.3% * |
| | | | HRMS (ESI⁺) calcd for [MH]⁺ of C₁₉H₁₇ClN₆S 397.1002 found 397.1011. |
| | | | HPLC: Rt 3.44-3.55min, 100% purity |
| 18 | 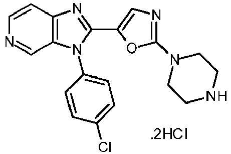 | 1-{5-[3-(4-Chlorophenyl)-3H-imidazo[4,5-c]pyridin-2-yl]-1,3-oxazol-2-yl}piperazine dihydrochloride | From Intermediate 83 |
| | | | Orange solid |
| | | | Yield 58.0mg, 11.8% * |
| | | | HRMS (ESI⁺) calcd for [MH]⁺ of C₁₉H₁₇ClN₆O 381.1230 found 381.1241. |
| | | | HPLC: Rt 3.25min, 100% purity |
| 19 | 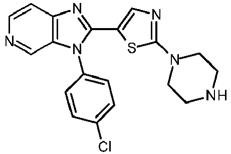 | 1-{5-[3-(4-Chlorophenyl)-3H-imidazo[4,5-c]pyridin-2-yl]-1,3-thiazol-2-yl}piperazine | From Intermediate 84 |
| | | | Off white solid |
| | | | Yield 9.50mg, 2.96%* |
| | | | HRMS (ESI⁺) calcd for [MH]⁺ of C₁₉H₁₇ClN₆S 397.1002 found 397.1008. |
| | | | HPLC: Rt 3.41-3.53min, 99.3% purity |
| 20 | 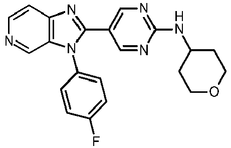 | 5-[3-(4-Fluorophenyl)-3H-imidazo[4,5-c]pyridin-2-yl]-*N-*(oxan-4-yl)pyrimidin-2-amine | From Intermediate 85 |
| | | | White solid |
| | | | Yield 79.0mg, 13.9% |
| | | | HRMS (ESI⁺) calcd for [MH]⁺ of C₂₁H₁₉FN₆O 391.1682 found 391.1693. |
| | | | HPLC: Rt 4.47min, 100% purity |
| 21 | 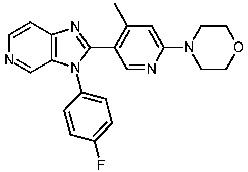 | 4-{5-[3-(4-Fluorophenyl)-3H-imidazo[4,5-c]pyridin-2-yl]-4-methylpyridin-2-yl}morpholine | From Intermediate 86 |
| | | | White solid |
| | | | Yield 15.2mg, 2.17% |
| | | | HRMS (ESI⁺) calcd for [MH]⁺ of C₂₂H₂₀FN₅O 390.1730 found 390.1721. |
| | | | HPLC: Rt 4.09min, 98.2% purity |
| 22 | 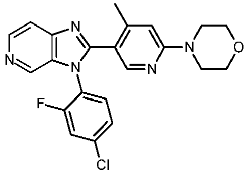 | 4-{5-[3-(4-Chloro-2-fluorophenyl)-3H-imidazo[4,5-c]pyridin-2-yl]-4-methylpyridin-2-yl}morpholine | From Intermediate 88 |
| | | | Yellow solid |
| | | | Yield 16.0mg, 3.59% |
| | | | HRMS (ESI⁺) calcd for [MH]⁺ of C₂₂H₁₉ClFN₅O 424.1340 found 424.134. |
| | | | HPLC: Rt 4.31min, 98.4% purity |
| 23 | 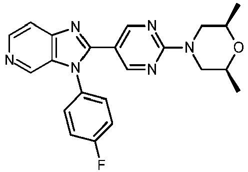 | (2R,6S)-4-{5-[3-(4-Fluorophenyl)-3H-imidazo[4,5-c]pyridin-2-yl]pyrimidin-2-yl}-2,6-dimethylmorpholine | From Intermediate 89 |
| | | | Beige solid |
| | | | Yield 25.8mg, 6.39% |
| | | | HRMS (ESI⁺) calcd for [MH]⁺ of C₂₂H₂₁FN₆O 405.1839 found 405.1843. |
| | | | HPLC: Rt 5.14min, 99.1% purity |
| 24 | 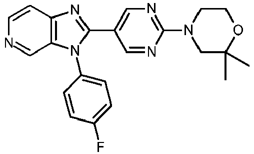 | 4-{5-[3-(4-Fluorophenyl)-3H-imidazo[4,5-c]pyridin-2-yl]pyrimidin-2-yl}-2,2-dimethylmorpholine | From Intermediate 90 |
| | | | White solid |
| | | | Yield 17.1mg, 5.28% |
| | | | HRMS (ESI⁺) calcd for [MH]⁺ of C₂₂H₂₁FN₆O 405.1839 found 405.1852. |
| | | | HPLC: Rt 5.14min, 97.4% purity |
| 25 | 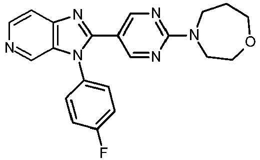 | 4-{5-[3-(4-Fluorophenyl)-3H-imidazo[4,5-c]pyridin-2-yl]pyrimidin-2-yl}-1,4-oxazepane | From Intermediate 91 |
| | | | White solid |
| | | | Yield 415mg, 43.5% |
| | | | HRMS (ESI⁺) calcd for [MH]⁺ of C₂₁H₁₉FN₆O 391.1682 found 391.1682. |
| | | | HPLC: Rt 4.80min, 98.7% purity |
| 26 | 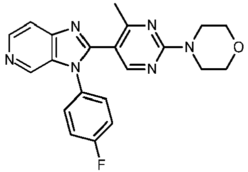 | 4-{5-[3-(4-Fluorophenyl)-3H-imidazo[4,5-c]pyridin-2-yl]-4-methylpyrimidin-2-yl}morpholine | From Intermediate 92 |
| | | | White solid |
| | | | Yield 8.50mg, 1.76% |
| | | | HRMS (ESI⁺) calcd for [MH]⁺ of C₂₁H₁₉FN₆O 391.1682 found 391.1676. |
| | | | HPLC: Rt 4.54min, 97.8% purity |
| 27 | 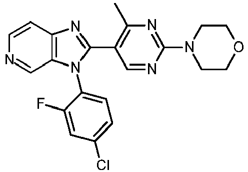 | 4-{5-[3-(4-Chloro-2-fluorophenyl)-3H-imidazo[4,5-c]pyridin-2-yl]-4-methylpyrimidin-2-yl}morpholine | From Intermediate 93 |
| | | | White solid |
| | | | Yield 37.0mg, 8.28% |
| | | | HRMS (ESI⁺) calcd for [MH]⁺ of C₂₁H₁₈ClFN₆O 425.1293 found 425.1296. |
| | | | HPLC: Rt 4.92min, 99.8% purity |
| 28 | 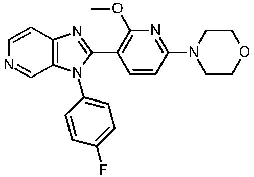 | 4-{5-[3-(4-Fluorophenyl)-3H-imidazo[4,5-c]pyridin-2-yl]-6-methoxypyridin-2-yl}morpholine | From Intermediate 95 |
| | | | Pale yellow solid |
| | | | Yield 47.4mg, 41.6% |
| | | | HRMS (ESI⁺) calcd for [MH]⁺ of C₂₂H₂₀FN₅O₂ 406.1679 found 406.1683. |
| | | | HPLC: Rt 5.02min, 99.5% purity |
| 29 | 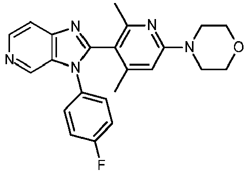 | 4-{5-[3-(4-Fluorophenyl)-3H-imidazo[4,5-c]pyridin-2-yl]-4,6-dimethylpyridin-2-yl}morpholine | From Intermediate 124 |
| | | | Off white solid |
| | | | Yield 15.0mg, 2.00% |
| | | | HRMS (ESI⁺) calcd for [MH]⁺ of C₂₃H₂₂FN₅O 404.1887 found 404.1889. |
| | | | HPLC: Rt 3.74min, 99.5% purity |
| 30 | 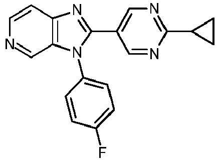 | 2-Cyclopropyl-5-[3-(4-fluorophenyl)-3H-imidazo[4,5-c]pyridin-2-yl]pyrimidine | From Intermediate 100 |
| | | | Off white solid |
| | | | Yield 50.2mg, 17.3% |
| | | | HRMS (ESI⁺) calcd for [MH]⁺ of C₁₉H₁₄FN₅ 332.1311 found 332.1313. |
| | | | HPLC: Rt 4.59min, 99.7% purity |
| 31 | 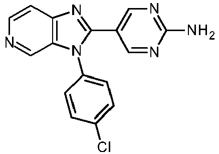 | 5-[3-(4-Chlorophenyl)-3H-imidazo[4,5-c]pyridin-2-yl]pyrimidin-2-amine | From Intermediate 101 |
| | | | Off white solid |
| | | | Yield 14.0mg, 3.16% |
| | | | HRMS (ESI⁺) calcd for [MH]⁺ of C₁₆H₁₁ClN₆ 323.0812 found 323.0815. |
| | | | HPLC: Rt 3.79min, 100% purity |
| 32 | 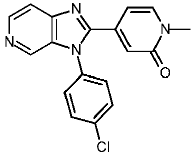 | 4-[3-(4-Chlorophenyl)-3H-imidazo[4,5-c]pyridin-2-yl]-1-methyl-1,2-dihydropyridin-2-one | From Intermediate 102 |
| | | | White solid |
| | | | Yield 109mg, 17.7% |
| | | | HRMS (ESI⁺) calcd for [MH]⁺ of C₁₈H₁₃ClN₄O 337.0856 found 337.0859. |
| | | | HPLC: Rt 4.07min, 98.8% purity |
| 33 | 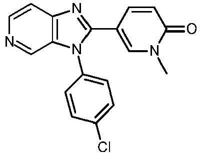 | 5-[3-(4-Chlorophenyl)-3H-imidazo[4,5-c]pyridin-2-yl]-1-methyl-1,2-dihydropyridin-2-one | From Intermediate 103 |
| | | | Off white solid |
| | | | Yield 24.0mg, 7.83% |
| | | | HRMS (ESI⁺) calcd for [MH]⁺ of C₁₈H₁₃ClN₄O 337.0856 found 337.0857. |
| | | | HPLC: Rt 4.14min, 99.6% purity |
| 34 | 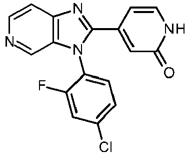 | 4-[3-(4-Chloro-2-fluorophenyl)-3H-imidazo[4,5-c]pyridin-2-yl]-1,2-dihydropyridin-2-one | From Intermediate 105 |
| | | | White solid |
| | | | Yield 15.0mg, 1.05% |
| | | | HRMS (ESI⁺) calcd for [MH]⁺ of C₁₇H₁₀ClFN₄O 341.0605 found 341.0607. |
| | | | HPLC: Rt 3.55min, 100% purity |
| 35 | 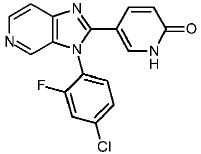 | 5-[3-(4-Chloro-2-fluorophenyl)-3H-imidazo[4,5-c]pyridin-2-yl]-1,2-dihydropyridin-2-one | From Intermediate 106 |
| | | | White solid |
| | | | Yield 41.0mg, 5.72% |
| | | | HRMS (ESI⁺) calcd for [MH]⁺ of C₁₇H₁₀ClFN₄O 341.0605 found 341.0613. |
| | | | HPLC: Rt 3.65min, 100% purity |
| 36 | 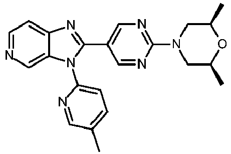 | (2R,6S)-2,6-Dimethyl-4-{5-[3-(5-methylpyridin-2-yl)-3H-imidazo[4,5-c]pyridin-2-yl]pyrimidin-2-yl}morpholine | From Intermediate 114 |
| | | | HRMS (ESI⁺) calcd for [MH]⁺ of C₂₂H₂₃N₇O 402.2042 found 402.2047. |
| | | | Yield 26.0mg, 3.71% |
| | | | White solid |
| | | | HPLC: Rt 4.96min, 98.8% purity |
| 37 | 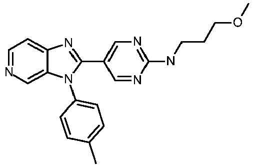 | *N*-(3-Methoxypropyl)-5-[3-(4-methylphenyl)-3H-imidazo[4,5-c]pyridin-2-yl]pyrimidin-2-amine | From Intermediate 115 |
| | | | White solid |
| | | | Yield 65.0mg, 8.65% |
| | | | HRMS (ESI⁺) calcd for [MH]⁺ of C₂₁H₂₂N₆O 375.1933 found 375.1935. |
| | | | HPLC: Rt 4.66min, 99.3% purity |
| 38 | 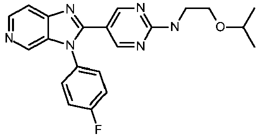 | 5-[3-(4-Fluorophenyl)-3H-imidazo[4,5-c]pyridin-2-yl]-*N-*[2-(propan-2-yloxy)ethyl]pyrimidin-2-amine | From Intermediate 116 |
| | | | White solid |
| | | | Yield 181mg, 23.4% |
| | | | HRMS (ESI⁺) calcd for [MH]⁺ of C₂₁H₂₁FN₆O 393.1839 found 393.1823. |
| | | | HPLC: Rt 4.80 min, 99.4% purity |
| 39 | 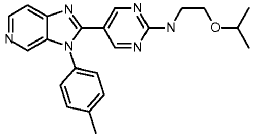 | 5-[3-(4-Methylphenyl)-3H-imidazo[4,5-c]pyridin-2-yl]-*N-*[2-(propan-2-yloxy)ethyl]pyrimidin-2-amine | From Intermediate 117 |
| | | | White solid |
| | | | Yield 55.7mg, 7.14% |
| | | | HRMS (ESI⁺) calcd for [MH]⁺ of C₂₂H₂₄N₆O 389.2090 found 389.2083. |
| | | | HPLC: Rt 5.01min, 100% purity |
| 40 | 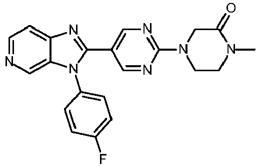 | 4-{5-[3-(4-Fluorophenyl)-3H-imidazo[4,5-c]pyridin-2-yl]pyrimidin-2-yl}-1-methylpiperazin-2-one | From Intermediate 118 |
| | | | White solid |
| | | | Yield 128mg, 21.5% |
| | | | HRMS (ESI⁺) calcd for [MH]⁺ of C₂₁H₁₈FN₇O 404.1635 found 404.1620. |
| | | | HPLC: Rt 4.22min, 100% purity |
| 41 | 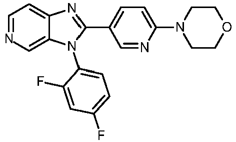 | 4-{5-[3-(2,4-Difluorophenyl)-3H-imidazo[4,5-c]pyridin-2-yl]pyridin-2-yl}morpholine | From Intermediate 119 |
| | | | White solid |
| | | | Yield 41.1mg, 6.60% |
| | | | HRMS (ESI⁺) calcd for [MH]⁺ of C₂₁H₁₇F₂N₅O 394.1479 found 394.1469. |
| | | | HPLC: Rt 4.47min, 99.2% purity |
| 42 | 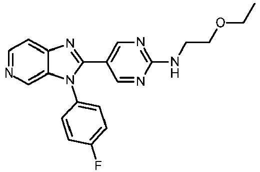 | *N*-(2-Ethoxyethyl)-5-[3-(4-fluorophenyl)-3H-imidazo[4,5-c]pyridin-2-yl]pyrimidin-2-amine | From Intermediate 120 |
| | | | Off white solid |
| | | | Yield 154mg, 27.6% |
| | | | LCMS (ES⁺): 379.0 [MH]⁺ |
| | | | HPLC: Rt 4.70min, 99.1% purity |
| 43 | 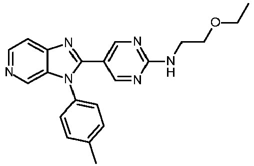 | *N*-(2-Ethoxyethyl)-5-[3-(4-methylphenyl)-3H-imidazo[4,5-c]pyridin-2-yl]pyrimidin-2-amine | From Intermediate 121 |
| | | | White solid |
| | | | Yield 119mg 21.1% |
| | | | LCMS (ES⁺): 375.1 [MH]⁺ |
| | | | HPLC: Rt 4.94min, 99.1% purity |

| | | | |
|---|---|---|---|
| * Boc deprotection under reaction conditions | | | |

### EXAMPLE 44

### 5-[3-(4-Chlorophenyl)-3H-imidazo[4,5-c]pyridin-2-yl]-1H-imidazole

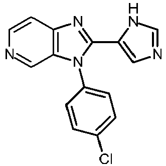

Intermediate 1 (200mg, 0.753mmol) and imidazole-4-carboxaldehyde (86.8mg, 0.903mmol) were dissolved in EtOH (5mL) and Na₂S₂O₄ (524mg, 3.01mmol) was added. The reaction mixture was heated using a microwave reactor at 160°C for 1h, diluted with sat. aq. NaHCO₃ (25mL) and water (25mL), and extracted into DCM (3x50mL). The combined organic fractions were dried (MgSO₄) and concentrated *in vacuo.* The residue was purified by column chromatography to give the title compound as a yellow solid (51.2mg, 23.0%). HRMS (ESI⁺) calcd for [MH]⁺ of C₁₅H₁₀ClN₅ 296.0703 found 296.0709. HPLC: Rt 3.24min, 100% purity.

### EXAMPLES 45-72

Examples 45-72 were prepared similarly to Example 44, by reductive condensation of Intermediates 1-11 with the appropriate aldehyde; see Table 12 below.

**Table 12: Reductive condensations of Intermediates 1-11**

| | | | |
|---|---|---|---|
| 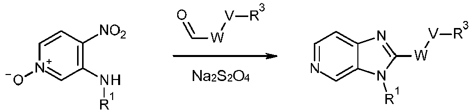 | | | |

| **Ex** | **Structure** | **Name** | **Intermediate(s) used, Form, Yield, LCMS, HPLC** |
|---|---|---|---|
| 45 | 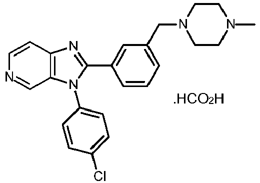 | 1-({3-[3-(4-Chlorophenyl)-3H-imidazo[4,5-c]pyridin-2-yl]phenyl}methyl)-4-methylpiperazine; formic acid | From Intermediate 1 |
| | | | White solid |
| | | | Yield 34.6mg, 9.91% |
| | | | HRMS (ESI⁺) calcd for [MH]⁺ of C₂₄H₂₄ClN₅ 418.1798 found 418.1794. |
| | | | HPLC: Rt 3.51min, 99.0% purity |
| 46 | 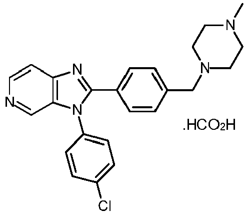 | 1-({4-[3-(4-Chlorophenyl)-3H-imidazo[4,5-c]pyridin-2-yl]phenyl}methyl)-4-methylpiperazine; formic acid | From Intermediate 1 |
| | | | Light yellow solid |
| | | | Yield 42.0mg, 12.0% |
| | | | HRMS (ESI⁺) calcd for [MH]⁺ of C₂₄H₂₄ClN₅ 418.1798 found 418.1813. |
| | | | HPLC: Rt 3.43min, 99.1% purity |
| 47 | 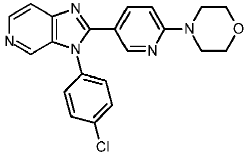 | 4-{5-[3-(4-Chlorophenyl)-3H-imidazo[4,5-c]pyridin-2-yl]pyridin-2-yl}morpholine | From Intermediate 1 |
| | | | White solid |
| | | | Yield 70.9mg, 20.9% |
| | | | HRMS (ESI⁺) calcd for [MH]⁺ of C₂₁H₁₈ClN₅O 392.1278 found 392.1282. |
| | | | HPLC: Rt 4.49min, 100% purity |
| 48 | 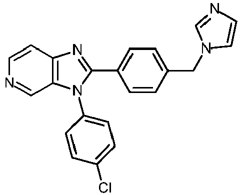 | 1-({4-[3-(4-Chlorophenyl)-3H-imidazo[4,5-c]pyridin-2-yl]phenyl}methyl)-1H-imidazole | From Intermediate 1 |
| | | | Orange gum |
| | | | Yield 39.4mg, 11.4% |
| | | | HRMS (ESI⁺) calcd for [MH]⁺ of C₂₂H₁₆ClN₅ 386.1172 found 386.1174. |
| | | | HPLC: Rt 3.90min, 100% purity |
| 49 | 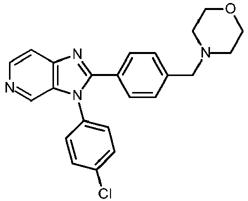 | 4-({4-[3-(4-Chlorophenyl)-3H-imidazo[4,5-c]pyridin-2-yl]phenyl}methyl)morpholine | From Intermediate 1 |
| | | | Yellow solid |
| | | | Yield 9.81mg, 2.84% |
| | | | HRMS (ESI⁺) calcd for [MH]⁺ of C₂₃H₂₁ClN₄O 405.1482 found 405.1478. |
| | | | HPLC: Rt 3.85min, 100% purity |
| 50 | 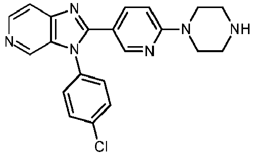 | 1-{5-[3-(4-Chlorophenyl)-3H-imidazo[4,5-c]pyridin-2-yl]pyridin-2-yl}piperazine | From Intermediate 1 |
| | | | Yellow solid |
| | | | Yield 3.10mg, 1.39% * |
| | | | HRMS (ESI⁺) calcd for [MH]⁺ of C₂₁H₁₉ClN₆ 391.1438 found 391.1427. |
| | | | HPLC: Rt 3.51min, 100% purity |
| 51 | 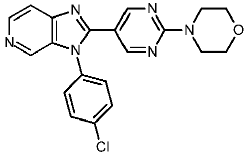 | 4-{5-[3-(4-Chlorophenyl)-3H-imidazo[4,5-c]pyridin-2-yl]pyrimidin-2-yl}morpholine | From Intermediate 1 |
| | | | White solid |
| | | | 2.75mg, 0.73% |
| | | | HRMS (ESI⁺) calcd for [MH]⁺ of C₂₀H₁₇ClN₆O 393.1230 found 393.1234. |
| | | | HPLC: Rt 5.06min, 97.9% purity |
| 52 | 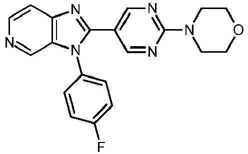 | 4-{5-[3-(4-Fluorophenyl)-3H-imidazo[4,5-c]pyridin-2-yl]pyrimidin-2-yl}morpholine | From Intermediate 2 |
| | | | White solid |
| | | | Yield 166mg, 36.6% |
| | | | HRMS (ESI⁺) calcd for [MH]⁺ of C₂₀H₁₇FN₆O 377.1526 found 377.1514. |
| | | | HPLC: Rt 4.68min, 98.1% purity |
| 53 | 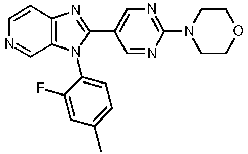 | 4-{5-[3-(2-Fluoro-4-methylphenyl)-3H-imidazo[4,5-c]pyridin-2-yl]pyrimidin-2-yl}morpholine | From Intermediate 3 |
| | | | White solid |
| | | | Yield 40.1mg, 13.5% |
| | | | HRMS (ESI⁺) calcd for [MH]⁺ of C₂₁H₁₉FN₆O 391.1682 found 391.1674. |
| | | | HPLC: Rt 4.77min, 99.3% purity |
| 54 | 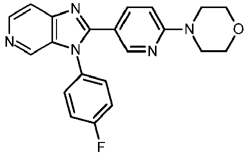 | 4-{5-[3-(4-Fluorophenyl)-3H-imidazo[4,5-c]pyridin-2-yl]pyridin-2-yl}morpholine | From Intermediate 2 |
| | | | White solid |
| | | | Yield 42.1mg, 14.0% |
| | | | HRMS (ESI⁺) calcd for [MH]⁺ of C₂₁H₁₈FN₅O 376.1573 found 376.1560. |
| | | | HPLC: Rt 4.20min, 98.2% purity |
| 55 | 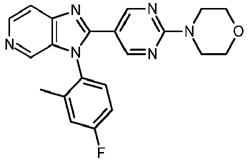 | 4-{5-[3-(4-Fluoro-2-methylphenyl)-3H-imidazo[4,5-c]pyridin-2-yl]pyrimidin-2-yl}morpholine | From Intermediate 4 |
| | | | Pale yellow solid |
| | | | Yield 22.2mg, 3.74% |
| | | | HRMS (ESI⁺) calcd for [MH]⁺ of C₂₁H₁₉FN₆O 391.1682 found 391.1679. |
| | | | HPLC: Rt 4.67min, 98.2% purity |
| 56 | 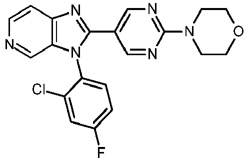 | 4-{5-[3-(2-Chloro-4-fluorophenyl)-3H-imidazo[4,5-c]pyridin-2-yl]pyrimidin-2-yl}morpholine | From Intermediate 5 |
| | | | Off white solid |
| | | | Yield 32.4mg, 4.97% |
| | | | HRMS (ESI⁺) calcd for [MH]⁺ of C₂₀H₁₆ClFN₆O 411.1136 found 411.1127. |
| | | | HPLC: Rt 4.72min, 100% purity |
| 57 | 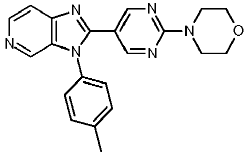 | 4-{5-[3-(4-Methylphenyl)-3H-imidazo[4,5-c]pyridin-2-yl]pyrimidin-2-yl}morpholine | From Intermediate 6 |
| | | | White solid |
| | | | Yield 35.7mg, 5.22% |
| | | | HRMS (ESI⁺) calcd for [MH]⁺ of C₂₁H₂₀N₆O 373.1777 found 373.1763. |
| | | | HPLC: Rt 4.80min, 99.4% purity |
| 58 | 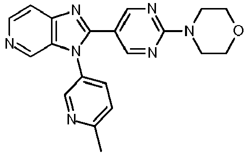 | 4-{5-[3-(6-Methylpyridin-3-yl)-3H-imidazo[4,5-c]pyridin-2-yl]pyrimidin-2-yl}morpholine | From Intermediate 7 |
| | | | Off white solid |
| | | | Yield 39.0mg, 5.14% |
| | | | HRMS (ESI⁺) calcd for [MH]⁺ of C₂₀H₁₉N₇O 374.1729 found 374.1736. |
| | | | HPLC: Rt 3.90min, 99.1% purity |
| 59 | 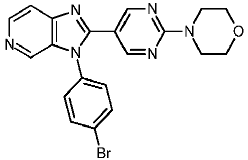 | 4-{5-[3-(4-Bromophenyl)-3H-imidazo[4,5-c]pyridin-2-yl]pyrimidin-2-yl}morpholine | From Intermediate 8 |
| | | | White solid |
| | | | Yield 55.0mg, 7.80% |
| | | | HRMS (ESI⁺) calcd for [MH]⁺ of C₂₀H₁₇BrN₆O 437.0725 found 437.0717. |
| | | | HPLC: Rt 5.15min, 99.0% purity |
| 60 | 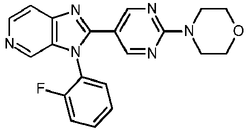 | 4-{5-[3-(2-Fluorophenyl)-3H-imidazo[4,5-c]pyridin-2-yl]pyrimidin-2-yl}morpholine | From Intermediate 9 |
| | | | White solid |
| | | | Yield 18.0mg, 2.38% |
| | | | HRMS (ESI⁺) calcd for [MH]⁺ of C₂₀H₁₇FN₆O 377.1526 found 377.1515. |
| | | | HPLC: Rt 4.54min, 99.4% purity |
| 61 | 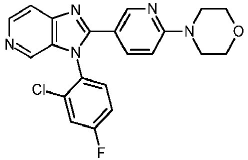 | 4-{5-[3-(2-Chloro-4-fluorophenyl)-3H-imidazo[4,5-c]pyridin-2-yl]pyridin-2-yl}morpholine | From Intermediate 5 |
| | | | Pale yellow solid |
| | | | Yield 23.0mg, 3.54% |
| | | | HRMS (ESI⁺) calcd for [MH]⁺ of C₂₁H₁₇ClFN₅O 410.1184 found 410.1189. |
| | | | HPLC: Rt 4.54min, 99.2% purity |
| 62 | 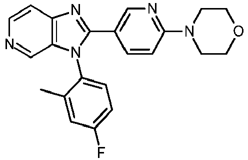 | 4-{5-[3-(4-Fluoro-2-methylphenyl)-3H-imidazo[4,5-c]pyridin-2-yl]pyridin-2-yl}morpholine | From Intermediate 4 |
| | | | Pale yellow solid |
| | | | Yield 21.1mg, 3.57% |
| | | | HRMS (ESI⁺) calcd for [MH]⁺ of C₂₂H₂₀FN₅O 390.1730 found 390.1723. |
| | | | HPLC: Rt 4.50min, 99.3% purity |
| 63 | 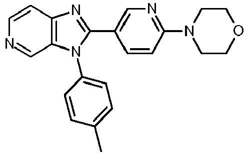 | 4-{5-[3-(4-Methylphenyl)-3H-imidazo[4,5-c]pyridin-2-yl]pyridin-2-yl}morpholine | From Intermediate 6 |
| | | | White solid |
| | | | Yield 72.0mg, 9.51% |
| | | | HRMS (ESI⁺) calcd for [MH]⁺ of C₂₂H₂₁N₅O 372.1824 found 372.1820. |
| | | | HPLC: Rt 4.75min, 100% purity |
| 64 | 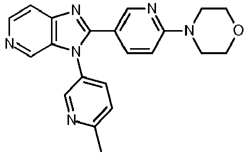 | 4-{5-[3-(6-Methylpyridin-3-yl)-3H-imidazo[4,5-c]pyridin-2-yl]pyridin-2-yl}morpholine | From Intermediate 7 |
| | | | White solid |
| | | | Yield 52.0mg, 7.24% |
| | | | HRMS (ESI⁺) calcd for [MH]⁺ of C₂₁H₂₀N₆O 373.1777 found 373.1768. |
| | | | HPLC: Rt 3.69min, 100% purity |
| 65 | 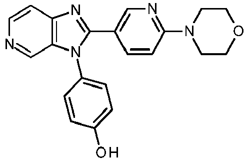 | 4-{2-[6-(Morpholin-4-yl)pyridin-3-yl]-3H-imidazo[4,5-c]pyridin-3-yl}phenol | From Intermediate 10 |
| | | | Off white solid |
| | | | Yield 106mg, 14.0% |
| | | | HRMS (ESI⁺) calcd for [MH]⁺ of C₂₁H₁₉N₅O₂ 374.1617 found 374.1618. |
| | | | HPLC: Rt 3.88min, 99.5% purity |
| 66 | 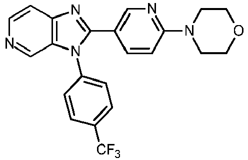 | 4-(5-{3-[4-(Trifluoromethyl)phenyl]-3H-imidazo[4,5-c]pyridin-2-yl}pyridin-2-yl)morpholine | From Intermediate 11 |
| | | | Off white solid |
| | | | Yield 22.0mg, 3.09% |
| | | | HRMS (ESI⁺) calcd for [MH]⁺ of C₂₂H₁₈F₃N₅O 426.1541 found 426.1549. |
| | | | HPLC: Rt 4.88min, 99.5% purity |
| 67 | 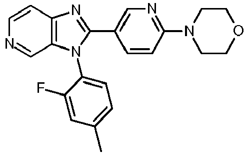 | 4-{5-[3-(2-Fluoro-4-methylphenyl)-3H-imidazo[4,5-c]pyridin-2-yl]pyridin-2-yl}morpholine | From Intermediate 3 |
| | | | Pale yellow solid |
| | | | Yield 78.3mg, 10.6% |
| | | | HRMS (ESI⁺) calcd for [MH]⁺ of C₂₂H₂₀FN₅O 390.1730 found 390.1729. |
| | | | HPLC: Rt 4.66min, 99.7% purity |
| 68 | 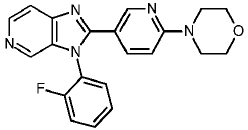 | 4-{5-[3-(2-Fluorophenyl)-3H-imidazo[4,5-c]pyridin-2-yl]pyridin-2-yl}morpholine | From Intermediate 9 |
| | | | White solid |
| | | | Yield 73.0mg, 9.69% |
| | | | HRMS (ESI⁺) calcd for [MH]⁺ of C₂₁H₁₈FN₅O 376.1573 found 376.1584. |
| | | | HPLC: Rt 4.33min, 99.5% purity |
| 69 | 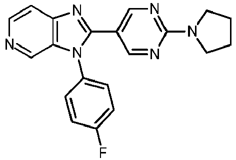 | 5-[3-(4-Fluorophenyl)-3H-imidazo[4,5-c]pyridin-2-yl]-2-(pyrrolidin-1-yl)pyrimidine | From Intermediate 2 |
| | | | White solid |
| | | | Yield 42.2mg, 7.29% |
| | | | HRMS (ESI⁺) calcd for [MH]⁺ of C₂₀H₁₇FN₆ 361.1577 found 361.1584. |
| | | | HPLC: Rt 4.83min, 98.5% purity |
| 70 | 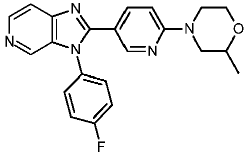 | 4-{5-[3-(4-Fluorophenyl)-3H-imidazo[4,5-c]pyridin-2-yl]pyridin-2-yl}-2-methylmorpholine | From Intermediates 2 and 65 |
| | | | Orange solid |
| | | | Yield 42.7mg, 9.11% |
| | | | HRMS (ESI⁺) calcd for [MH]⁺ of C₂₂H₂₀FN₅O 390.1730 found 390.1726. |
| | | | HPLC: Rt 4.54min, 99.4% purity |
| 71 | 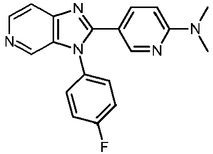 | 5-[3-(4-Fluorophenyl)-3H-imidazo[4,5-c]pyridin-2-yl]-*N*,*N*-dimethylpyridin-2-amine | From Intermediate 2 |
| | | | White solid |
| | | | Yield 84.0mg, 12.6% |
| | | | HRMS (ESI⁺) calcd for [MH]⁺ of C₁₉H₁₆FN₅ 334.1468 found 334.1475. |
| | | | HPLC: Rt 3.72min, 100% purity |
| 72 | 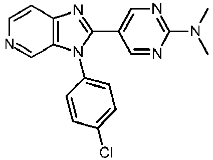 | 5-[3-(4-Chlorophenyl)-3H-imidazo[4,5-c]pyridin-2-yl]-*N*,*N*-dimethylpyrimidin-2-amine | From Intermediates 1 and 64 |
| | | | White solid |
| | | | Yield 12.6mg, 1.91% |
| | | | HRMS (ESI⁺) calcd for [MH]⁺ of C₁₈H₁₅ClN₆ 351.1125 found 351.1125. |
| | | | HPLC: Rt 4.90min, 100% purity |

| | | | |
|---|---|---|---|
| * Additional Boc deprotection step incorporated | | | |

### EXAMPLE 73

### 4-{4-[3-(4-Fluorophenyl)-3H-imidazo[4,5-c]pyridin-2-yl]pyridin-2-yl}morpholine

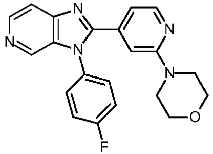

Intermediate 20 (250mg, 1.24mmol), 2-(morpholin-4-yl)pyridine-4-carbaldehyde (238mg, 1.24mmol) and Na₂S₂O₄ (646mg, 3.71 mmol) were suspended in EtOH (2mL) and the reaction mixture was heated at 150°C in a microwave reactor for 45min. The reaction mixture was poured into 1M aq. Na₂CO₃ (25mL) and extracted with DCM (2x25mL). The combined organic fractions were dried (MgSO₄) and concentrated *in vacuo.* The residue was triturated from MeOH (2mL) to give the title compound (101mg, 21.7%) as an off white solid. HRMS (ESI⁺) calcd for [MH]⁺ of C₂₁H₁₈FN₅O 376.1573 found 376.1573. HPLC: Rt 3.89min, 97.8% purity.

### EXAMPLE 74

### 4-{5-[3-(4-Chloro-3-fluorophenyl)-3H-imidazo[4,5-c]pyridin-2-yl]pyrimidin-2-yl}morpholine

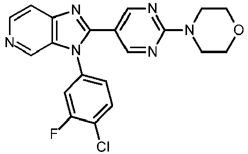

Intermediate 13 (200mg, 0.747mmol), 2-(morpholin-4-yl)pyrimidine-5-carbaldehyde (188mg, 0.971mmol) and Na₂S₂O₄ (520mg, 2.99mmol) were suspended in EtOH (5mL) and the reaction mixture was heated using a microwave reactor at 160°C for 1h. The reaction mixture was diluted with 1M aq. Na₂CO₃ (40mL) and extracted into DCM (2x50mL). The combined organic fractions were dried (MgSO₄) and concentrated *in vacuo.* The residue was purified by column chromatography and by reverse phase HPLC to give the title compound (36.6mg, 11.9%) as an off white solid. HRMS (ESI⁺) calcd for [MH]⁺ of C₂₀H₁₆ClFN₆O 411.1136 found 411.1133. HPLC: Rt 5.09min, 99.7% purity.

### EXAMPLES 75-84

Examples 75-84 were prepared similarly to Example 74, by reductive condensation of Intermediates 14-18 with the appropriate aldehyde; see Table 13 below.

**Table 13: Reductive condensations of Intermediates 14-18**

| | | | |
|---|---|---|---|
| 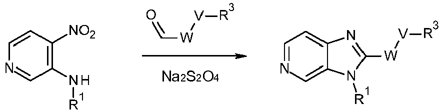 | | | |

| **Ex** | **Structure** | **Name** | **Intermediate(s) used, Form, Yield, LCMS, HPLC** |
|---|---|---|---|
| 75 | 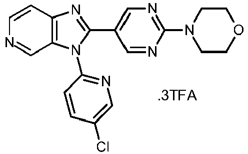 | 4-{5-[3-(5-Chloropyridin-2-yl)-3H-imidazo[4,5-c]pyridin-2-yl]pyrimidin-2-yl}morpholine; tris(trifluoroacetic acid) | From Intermediate 14 White solid |
| | | | Yield 29.0mg, 4.47% |
| | | | HRMS (ESI⁺) calcd for [MH]⁺ of C₁₉H₁₆ClN₇O 394.1183 found 394.1168. |
| | | | HPLC: Rt 4.68min, 97.8% purity |
| 76 | 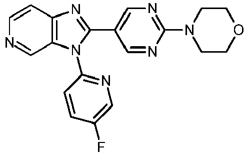 | 4-{5-[3-(5-Fluoropyridin-2-yl)-3H-imidazo[4,5-c]pyridin-2-yl]pyrimidin-2-yl}morpholine | From Intermediate 15 |
| | | | White solid |
| | | | Yield 53.2mg, 7.49% |
| | | | HRMS (ESI⁺) calcd for [MH]⁺ of C₁₉H₁₆FN₇O 378.1479 found 378.1473. |
| | | | HPLC: Rt 4.36min, 98.4% purity |
| 77 | 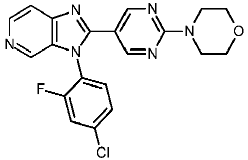 | 4-{5-[3-(4-Chloro-2-fluorophenyl)-3H-imidazo[4,5-c]pyridin-2-yl]pyrimidin-2-yl}morpholine | From Intermediate 16 |
| | | | Yellow solid |
| | | | Yield 186mg, 15.4% |
| | | | HRMS (ESI⁺) calcd for [MH]⁺ of C₂₀H₁₆ClFN₆O 411.1136 found 411.1142. |
| | | | HPLC: Rt 5.09min, 97.6% purity |
| 78 | 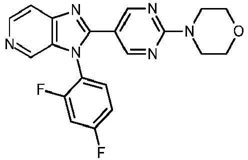 | 4-{5-[3-(2,4-Difluorophenyl)-3H-imidazo[4,5-c]pyridin-2-yl]pyrimidin-2-yl}morpholine | From Intermediate 17 |
| | | | Pale yellow solid |
| | | | Yield 39.2mg, 4.54% |
| | | | HRMS (ESI⁺) calcd for [MH]⁺ of C₂₀H₁₆F₂N₆O 395.1432 found 395.1436. |
| | | | HPLC: Rt 4.76min, 99.6% purity |
| 79 | 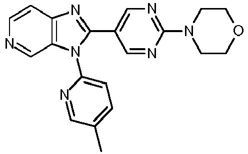 | 4-{5-[3-(5-Methylpyridin-2-yl)-3H-imidazo[4,5-c]pyridin-2-yl]pyrimidin-2-yl}morpholine | From Intermediate 18 |
| | | | Off white solid |
| | | | Yield 46.1mg, 9.47% |
| | | | HRMS (ESI⁺) calcd for [MH]⁺ of C₂₀H₁₉N₇O 374.1729 found 374.1736. |
| | | | HPLC: Rt 4.30min, 99.6% purity |
| 80 | 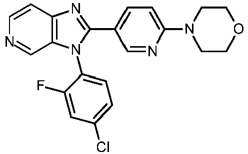 | 4-{5-[3-(4-Chloro-2-fluorophenyl)-3H-imidazo[4,5-c]pyridin-2-yl]pyridin-2-yl}morpholine | From Intermediate 16 |
| | | | White solid |
| | | | Yield 41.0mg, 12.8% |
| | | | HRMS (ESI⁺) calcd for [MH]⁺ of C₂₁H₁₇ClFN₅O 410.1184 found 410.1187. |
| | | | HPLC: Rt 4.58min, 99.6% purity |
| 81 | 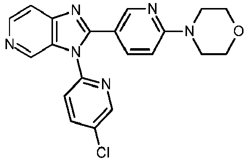 | 4-{5-[3-(5-Chloropyridin-2-yl)-3H-imidazo[4,5-c]pyridin-2-yl]pyridin-2-yl}morpholine | From Intermediate 14 |
| | | | White solid |
| | | | Yield 7.05mg, 2.57% |
| | | | HRMS (ESI⁺) calcd for [MH]⁺ of C₂₀H₁₇ClN₆O 393.1230 found 393.1226. |
| | | | HPLC: Rt 4.45min, 97.9% purity |
| 82 | 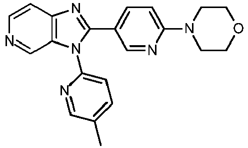 | 4-{5-[3-(5-Methylpyridin-2-yl)-3H-imidazo[4,5-c]pyridin-2-yl]pyridin-2-yl}morpholine | From Intermediate 18 |
| | | | White solid |
| | | | Yield 38.0mg, 7.83% HRMS (ESI⁺) calcd for [MH]⁺ of C₂₁H₂₀N₆O 373.1777 found 373.1787. |
| | | | HPLC: Rt 4.03min, 99.4% purity |
| 83 | 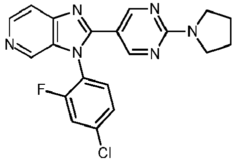 | 5-[3-(4-Chloro-2-fluorophenyl)-3H-imidazo[4,5-c]pyridin-2-yl]-2-(pyrrolidin-1-yl)pyrimidine | From Intermediate 16 |
| | | | White solid |
| | | | Yield 8.20mg, 2.22% |
| | | | HRMS (ESI⁺) calcd for [MH]⁺ of C₂₀H₁₆ClFN₆ 395.1187 found 395.1190. |
| | | | HPLC: Rt 5.09min, 100% purity |
| 84 | 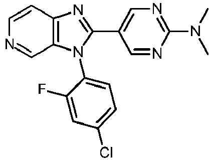 | 5-[3-(4-Chloro-2-fluorophenyl)-3H-imidazo[4,5-c]pyridin-2-yl]-*N*,*N*-dimethylpyrimidin-2-amine | From Intermediates 16 and 64 |
| | | | Light yellow solid |
| | | | Yield 63.7mg, 13.6% HRMS (ESI⁺) calcd for [MH]⁺ of C₁₈H₁₄ClFN₆ |
| | | | 369.1031 found 369.1031. |
| | | | HPLC: Rt 4.93min, 100% purity |

### EXAMPLE 85

### N-(1-{5-[3-(4-Chlorophenyl)-3H-imidazo[4,5-c]pyridin-2-yl]pyridin-2-yl}piperidin-4-yl)acetamide

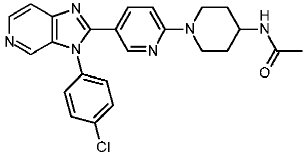

Example 8 (100mg, 0.247mmol), Et₃N (41.2uL, 0.296mmol) and acetyl chloride (19.3uL, 0.272mmol) were dissolved in DCM (10mL) and the reaction mixture was stirred for 2h and concentrated *in vacuo.* The residue was purified by column chromatography and partitioned between DCM (20mL) and sat. aq. NaHCO₃ (20mL). The organic fraction was washed with sat. aq. NaHCO₃ (20mL), dried (MgSO₄) and concentrated *in vacuo* to give the title compound (61.6mg, 55.8%) as a light yellow solid. HRMS (ESI⁺) calcd for [MH]⁺ of C₂₄H₂₃ClN₆O 447.1700 found 447.1701. HPLC: Rt 3.98min, 99.7% purity.

### EXAMPLE 86

### 1-(4-{5-[3-(4-Fluorophenyl)-3H-imidazo[4,5-c]pyridin-2-yl]pyridin-2-yl}piperazin-1-yl)ethan-1-one

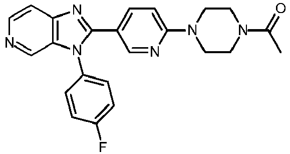

Example 86 was prepared similarly to Example 85, using Intermediate 126 instead of Example 8, to give the title compound (227mg, 38.7%) as a white solid. HRMS (ESI⁺) calcd for [MH]⁺ of C₂₃H₂₁FN₆O 417.1839 found 417.1851. HPLC: Rt 4.26min, 100% purity.

### EXAMPLE 87

### 1-(4-{5-[3-(4-Fluorophenyl)-3H-imidazo[4,5-c]pyridin-2-yl]pyridin-2-yl}-1,4-diazepan-1-yl)ethan-1-one; bis(trifluoroacetic acid)

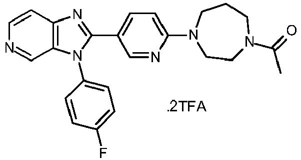

Example 87 was prepared similarly to Example 85, using Intermediate 129 instead of Example 8, to give the title compound (143mg, 41.2%) as a pink gum. HRMS (ESI⁺) calcd for [MH]⁺ of C₂₄H₂₃FN₆O 431.1996 found 431.1997. HPLC: Rt 4.41min, 99.7% purity.

### EXAMPLE 88

### N-(1-{5-[3-(4-Chlrophenyl)-3H-imidazo[4,5-c]pyridin-2-yl]pyridin-2-yl}piperidin-4-yl)methanesulfonamide

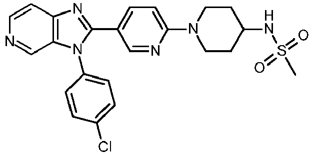

Example 8 (100mg, 0.247mmol), Et₃N (41.2uL, 0.296mmol) and methanesulfonyl chloride (26.8uL, 0.346mmol) were dissolved in DCM (10mL) and the reaction mixture was stirred for 2h, diluted with DCM (20mL), washed with sat. aq. NaHCO₃ (30mL), dried (MgSO₄) and concentrated *in vacuo.* The residue was triturated from MeOH (3mL) and collected by filtration to give the title compound (30.6mg, 25.7%) as a yellow solid. HRMS (ESI⁺) calcd for [MH]⁺ of C₂₃H₂₃ClN₆O₂S 483.1370 found 483.1375. HPLC: Rt 4.18min, 99.4% purity.

### EXAMPLE 89

### 1-{5-[3-(4-Fluorophenyl)-3H-imidazo[4,5-c]pyridin-2-yl]pyridin-2-yl}-4-methanesulfonylpiperazine

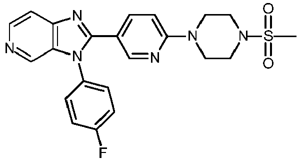

Example 89 was prepared similarly to Example 88, using Intermediate 126 instead of Example 8, to give the title compound (44.5mg, 10.3%) as a yellow solid. HRMS (ESI⁺) calcd for [MH]⁺ of C₂₂H₂₁FN₆O₂S 453.1509 found 453.1522. HPLC: Rt 4.59min, 98.2% purity.

### EXAMPLE 90

### 4-{5-[3-(4-Chlorophenyl)-3H-imidazo[4,5-c]pyridin-2-yl]pyridin-2-yl}piperazine-1-carboxamide dihydrochloride

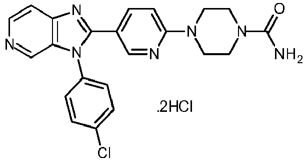

Example 50 trihydrochloride (94.5mg, 0.189mmol) was dissolved in DCM (5mL), and DIPEA (145uL, 0.831mmol) and trimethylsilyl isocyanate (30.7uL, 0.227mmol) were added. The reaction mixture was stirred for 16h, diluted with 1M aq. Na₂CO₃ (25mL) and extracted into DCM (3x25mL). The combined organic fractions were dried (MgSO₄) and concentrated *in vacuo.* The residue was dissolved in 1.25M HCI in EtOH (5mL), stirred for 1h and concentrated *in vacuo.* The residue was purified by reverse phase HPLC to give the title compound (21.4mg, 22.3%) as a yellow solid. HRMS (ESI⁺) calcd for [MH]⁺ of C₂₂H₂₀ClN₇O 434.1496 found 434.1497. HPLC: Rt 4.19min, 98.5% purity.

### EXAMPLES 91-94

Examples 91-94 were prepared similarly to Example 90, by reaction of Examples 8, 18 and Intermediates 126, 129 with trimethylsilyl isocyanate; see Table 14 below.

**Table 14: Reaction of Examples 8, 18 and Intermediates 126, 129 with trimethylsilyl isocyanate**

| | | | |
|---|---|---|---|
| 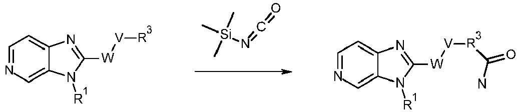 | | | |

| **Ex** | **Structure** | **Name** | **Intermediate(s) used, Form, Yield, LCMS, HPLC** |
|---|---|---|---|
| 91 | 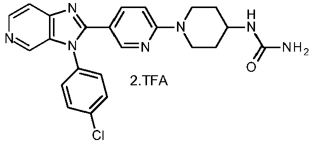 | (1-{5-[3-(4-Chlorophenyl)-3H-imidazo[4,5-c]pyridin-2-yl]pyridin-2-yl}piperidin-4-yl)urea; bis(trifluoroacetic acid) | From Example 8 |
| | | | Yellow solid |
| | | | Yield 36.3mg, 21.7% |
| | | | HRMS (ESI⁺) calcd for [MH]⁺ of C₂₃H₂₂ClN₇O 448.1653 found 448.1656. |
| | | | HPLC: Rt 3.75min, 98.7% purity |
| 92 | 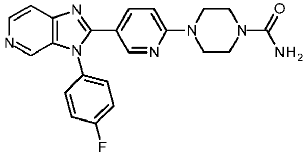 | 4-{5-[3-(4-Fluorophenyl)-3H-imidazo[4,5-c]pyridin-2-yl]pyridin-2-yl}piperazine-1-carboxamide | From Intermediate 126 |
| | | | White solid |
| | | | Yield 44.0mg, 11.1% |
| | | | HRMS (ESI⁺) calcd for [MH]⁺ of C₂₂H₂₀FN₇O 418.1791 found 418.1795. |
| | | | HPLC: Rt 3.88min, 100% purity |
| 93 | 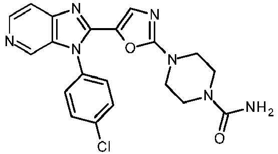 | 4-{5-[3-(4-Chlorophenyl)-3H-imidazo[4,5-c]pyridin-2-yl]-1,3-oxazol-2-yl}piperazine-1-carboxamide | From Example 18 |
| | | | Pale yellow solid |
| | | | Yield 7.20mg, 7.71% |
| | | | HRMS (ESI⁺) calcd for [MH]⁺ of C₂₀H₁₈ClN₇O₂ 424.1289 found 424.1288. |
| | | | HPLC: Rt 4.20min, 99.7% purity |
| 94 | 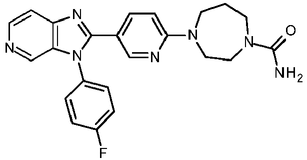 | 4-{5-[3-(4-Fluorophenyl)-3H-imidazo[4,5-c]pyridin-2-yl]pyridin-2-yl}-1,4-diazepane-1-carboxamide | From Intermediate 129 |
| | | | Pink solid |
| | | | Yield 56.7mg, 24.9% |
| | | | HRMS (ESI⁺) calcd for [MH]⁺ of C₂₃H₂₂FN₇O 432.1948 found 432.1955. |
| | | | HPLC: Rt 3.83min, 99.0% purity |

### EXAMPLE 95

### 4-(5-{3-Phenyl-3H-imidazo[4,5-c]pyridin-2-yl}pyrimidin-2-yl)morpholine

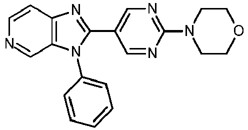

Example 51 (115mg, 0.293mmol) was suspended in EtOH (5mL) and ammonium formate (148mg, 2.34mmol) and 10% Pd/C (50.0mg) were added. The reaction mixture was heated under reflux for 5h, filtered through Celite and concentrated *in vacuo.* The residue was dissolved in DCM (50mL), washed with 1M aq. Na₂CO₃ (50mL) dried (MgSO₄) and concentrated *in vacuo.* The residue was purified by column chromatography to give the title compound (40.2mg, 38.3%) as a white solid. HRMS (ESI⁺) calcd for [MH]⁺ of C₂₀H₁₈N₆O 359.1620 found 359.1613. HPLC: Rt 4.65min, 99.8% purity.

### EXAMPLE 96

### 4-{5-[3-(4-Cyclopropylphenyl)-3H-imidazo[4,5-c]pyridin-2-yl]pyrimidin-2-yl}morpholine

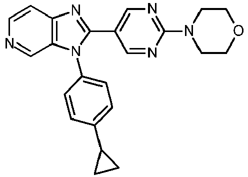

Example 51 (250mg, 0.636mmol), cyclopropylboronic acid (54.7mg, 0.636mmol), Pd(OAc)₂ (14.3mg, 63.6umol), XPhos (30.3mg, 63.6umol) and Cs₂CO₃ (518mg, 1.59mmol) were dissolved in dioxane (1.5mL) and water (1.5mL) and heated in a sealed tube at 100°C for 16h. The reaction mixture was partitioned between DCM (20mL) and water (20mL) and the organic fraction was washed with brine (20mL), dried (MgSO₄) and concentrated *in vacuo.* The residue was purified by reverse phase HPLC to give the title compound (17.1mg, 6.74%) as a white solid. HRMS (ESI⁺) calcd for [MH]⁺ of C₂₃H₂₂N₆O 399.1933 found 399.1938. HPLC: Rt 5.16min, 99.4% purity.

### EXAMPLE 97

### 4-{4-Methyl-5-[3-(4-methylphenyl)-3H-imidazo[4,5-c]pyridin-2-yl]pyridin-2-yl}morpholine

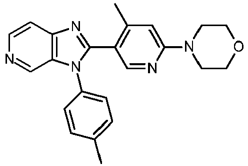

Intermediate 87 (300mg, 1.51mmol) was dissolved in NMP (2mL) and morpholine (783uL, 9.08mmol) and the reaction mixture was heated at 180°C in a microwave reactor for 30min. The reaction mixture was diluted with EtOAc (10mL) and water (10mL). The organic fraction was washed with brine (10mL), dried (MgSO₄) and concentrated *in vacuo.* The residue was purified by reverse phase HPLC to give the title compound (16.4mg, 2.81%) as a colourless gum. HRMS (ESI⁺) calcd for [MH]⁺ of C₂₃H₂₃N₅O 386.1981 found 386.1982. HPLC: Rt 4.17min, 99.3% purity.

### EXAMPLES 98-107

Examples 98-107 were prepared similarly to Example 97, by SnAr and cyclisation of Intermediates 97-98, 106, 109-110, 112, 122 and 123 with the appropriate amine; see Table 15 below.

**Table 15: SnAr and cyclisation of Intermediates 97-98, 106, 109-110, 112, 122 and 123**

| | | | |
|---|---|---|---|
| 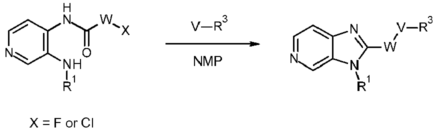 | | | |

| **Ex** | **Structure** | **Name** | **Intermediate(s) used, Form, Yield, LCMS, HPLC** |
|---|---|---|---|
| 98 | 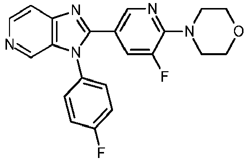 | 4-{3-Fluoro-5-[3-(4-fluorophenyl)-3H-imidazo[4,5-c]pyridin-2-yl]pyridin-2-yl}morpholine | From Intermediate 97 |
| | | | White solid |
| | | | Yield 134mg, 27.7% |
| | | | HRMS (ESI⁺) calcd for [MH]⁺ of C₂₁H₁₇F₂N₅O 394.1479 found 394.1478. |
| | | | HPLC: Rt 4.80min, 99.6% purity |
| 99 | 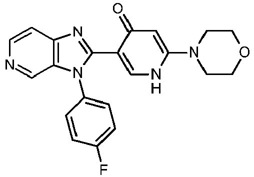 | 5-[3-(4-Fluorophenyl)-3H-imidazo[4,5-c]pyridin-2-yl]-2-(morpholin-4-yl)-1,4-dihydropyridin-4-one | From Intermediate 98 |
| | | | White solid |
| | | | Yield 15.0mg, 1.11% |
| | | | HRMS (ESI⁺) calcd for [MH]⁺ of C₂₁H₁₈FN₅O₂ 392.1523 found 392.1520. |
| | | | HPLC: Rt 3.73min, 100% purity |
| 100 | 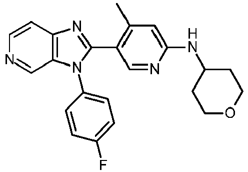 | 5-[3-(4-Fluorophenyl)-3H-imidazo[4,5-c]pyridin-2-yl]-4-methyl-*N*-(oxan-4-yl)pyridin-2-amine | From Intermediate 109 |
| | | | White solid |
| | | | Yield 26.0mg, 8.77% HRMS (ESI⁺) calcd for [MH]⁺ of C₂₃H₂₂FN₅O |
| | | | 404.1887 found 404.1892. |
| | | | HPLC: Rt 3.56min, 100% purity |
| 101 | 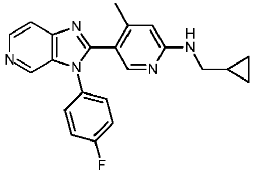 | *N*-(Cyclopropylmethyl)-5-[3-(4-fluorophenyl)-3H-imidazo[4,5-c]pyridin-2-yl]-4-methylpyridin-2-amine | From Intermediate 109 |
| | | | White solid |
| | | | Yield 43.0mg, 15.7% |
| | | | HRMS (ESI⁺) calcd for [MH]⁺ of C₂₂H₂₀FN₅ 374.1781 found 374.1787. |
| | | | HPLC: Rt 3.90min, 100% purity |
| 102 | 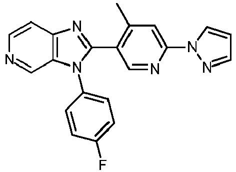 | 5-[3-(4-Fluorophenyl)-3H-imidazo[4,5-c]pyridin-2-yl]-4-methyl-2-(1H-pyrazol-1-yl)pyridine | From Intermediate 109 |
| | | | Off white solid |
| | | | Yield 28.1mg, 12.9% |
| | | | HRMS (ESI⁺) calcd for [MH]⁺ of C₂₁H₁₅FN₆ 371.1420 found 371.1419. |
| | | | HPLC: Rt 4.98min, 99.8% purity |
| 103 | 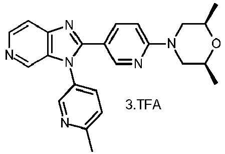 | (2R,6S)-2,6-Dimethyl-4-{5-[3-(6-methylpyridin-3-yl)-3H-imidazo[4,5-c]pyridin-2-yl]pyridin-2-yl}morpholine; tris(trifluoroacetic acid) | From Intermediate 110 |
| | | | Yellow gum |
| | | | Yield 26.0mg, 1.75% |
| | | | HRMS (ESI⁺) calcd for [MH]⁺ of C₂₃H₂₄N₆O 401.2090 found 401.2084. |
| | | | HPLC: Rt 4.27min, 99.1% purity |
| 104 | 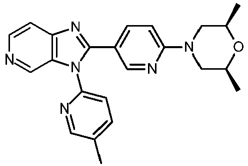 | (2R,6S)-2,6-Dimethyl-4-{5-[3-(5-methylpyridin-2-yl)-3H-imidazo[4,5-c]pyridin-2-yl]pyridin-2-yl}morpholine | From Intermediate 112 |
| | | | White solid |
| | | | Yield 26.0mg, 3.71% |
| | | | HRMS (ESI⁺) calcd for [MH]⁺ of C₂₃H₂₄N₆O 401.2090 found 401.2098. |
| | | | HPLC: Rt 4.66min, 98.9% purity |
| 105 | 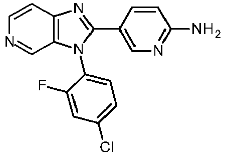 | 5-[3-(4-Chloro-2-fluorophenyl)-3H-imidazo[4,5-c]pyridin-2-yl]pyridin-2-amine | From Intermediate 106 |
| | | | Pale pink solid |
| | | | Yield 24.0mg, 8.49% |
| | | | HRMS (ESI⁺) calcd for [MH]⁺ of C₁₇H₁₁ClFN₅ 340.0765 found 340.0773. |
| | | | HPLC: Rt 3.30min, 100% purity |
| 106 | 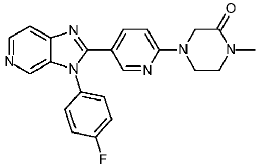 | 4-{5-[3-(4-Fluorophenyl)-3H-imidazo[4,5-c]pyridin-2-yl]pyridin-2-yl}-1-methylpiperazin-2-one | From Intermediate 123 |
| | | | Pale yellow solid |
| | | | Yield 38.5mg, 13.1% |
| | | | HRMS (ESI⁺) calcd for [MH]⁺ of C₂₂H₁₉FN₆O 403.1682 found 403.1684. |
| | | | HPLC: Rt 4.07min, 99.4% purity |
| 107 | 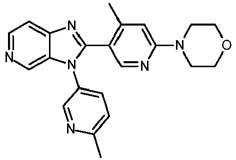 | 4-{4-Methyl-5-[3-(6-methylpyridin-3-yl)-3H-imidazo[4,5-c]pyridin-2-yl]pyridin-2-yl}morpholine | From Intermediate 122 |
| | | | Pale yellow gum |
| | | | Yield 24.0mg, 3.11% |
| | | | LCMS (ES⁺): 387.0 [MH]⁺ |
| | | | HPLC: Rt 3.74min, 98.7% purity |

### EXAMPLE 108

### 4-{5-[3-(4-Fluorophenyl)-3H-imidazo[4,5-c]pyridin-2-yl]-6-methylpyridin-2-yl}morpholine

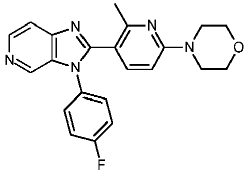

Intermediate 130 (94.0mg, 0.277mmol) was dissolved in DMA (1mL) and morpholine (192uL, 2.22mmol) was added. The reaction mixture was heated at 200°C in a microwave reactor for 30min and partitioned between DCM (20mL) and water (20mL). The organic fraction was washed with water (20mL), dried (MgSO₄) and concentrated *in vacuo.* The residue was purified by reverse phase HPLC to give the title compound (47.0mg, 43.5%) as a white solid. HRMS (ESI⁺) calcd for [MH]⁺ of C₂₂H₂₀FN₅O 390.1730 found 390.1737. HPLC: Rt 3.96min, 99.7% purity.

### EXAMPLES 109-111

Examples 109-111 were prepared similarly to Example 108, by reaction of Intermediates 128, 131 and 133 with the appropriate amine; see Table 16 below.

**Table 16: SNAr of Intermediates 128, 131 and 133 with the appropriate amine**

| | | | |
|---|---|---|---|
| 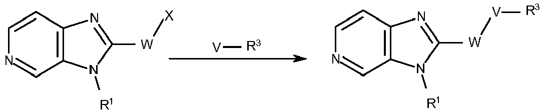 | | | |

| **Ex** | **Structure** | **Name** | **Intermediate(s) used, Form, Yield, LCMS, HPLC** |
|---|---|---|---|
| 109 | 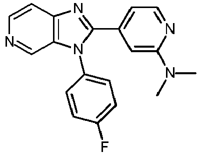 | 4-[3-(4-Fluorophenyl)-3H-imidazo[4,5-c]pyridin-2-yl]-*N*,*N-*dimethylpyridin-2-amine | From Intermediate 131 |
| | | | Yellow solid |
| | | | Yield 110mg, 53.4% |
| | | | HRMS (ESI⁺) calcd for [MH]⁺ of C₁₉H₁₆FN₅ 334.1468 found 334.1476. |
| | | | HPLC: Rt 3.19min, 99.7% purity |
| 110 | 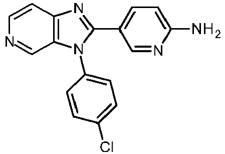 | 5-[3-(4-Chlorophenyl)-3H-imidazo[4,5-c]pyridin-2-yl]pyridin-2-amine | From Intermediate 128 |
| | | | White solid |
| | | | Yield 20.0mg, 26.2% |
| | | | HRMS (ESI⁺) calcd for [MH]⁺ of C₁₇H₁₂ClN₅ 322.0859 found 322.0849. |
| | | | HPLC: Rt 3.45min, 99.7% purity |
| 111 | 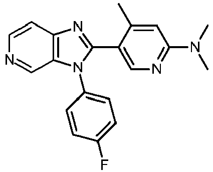 | 5-[3-(4-Fluorophenyl)-3H-imidazo[4,5-c]pyridin-2-yl]-*N*,*N*,4-trimethylpyridin-2-amine | From Intermediate 133 |
| | | | Light yellow solid |
| | | | Yield 24.8mg, 29.2% |
| | | | HRMS (ESI⁺) calcd for [MH]⁺ of C₂₀H₁₈FN₅ 348.1624 found 348.1631. |
| | | | HPLC: Rt 3.44min, 99.1% purity |

### EXAMPLE 112

### 5-[3-(4-Fluorophenyl)-3H-imidazo[4,5-c]pyridin-2-yl]-2-(oxolan-3-yloxy)pyridine

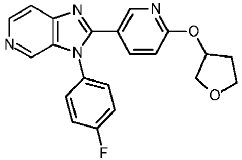

NaH (14.8mg, 60% in mineral oil, 0.370mmol) was suspended in THF (1mL), 3-hydroxytetrahydrofuran (29.7uL, 0.370mmol) was added and the reaction mixture was stirred for 5min. Intermediate 127 (80.0mg, 0.246mmol) was added and the reaction mixture was stirred for 16h, quenched with water (20mL) and diluted with EtOAc (20mL). The organic fraction was washed with brine (20mL), dried (MgSO₄) and concentrated *in vacuo.* The residue was purified by reverse phase HPLC to give the title compound (19.2mg, 20.7%) as a white solid. HRMS (ESI⁺) calcd for [MH]⁺ of C₂₁H₁₇FN₄O₂ 377.1414 found 377.1419. HPLC: Rt: 4.78min, 98.9% purity.

### EXAMPLE 113

### 5-[3-(4-Fluorophenyl)-3H-imidazo[4,5-c]pyridin-2-yl]-2-(oxan-4-yloxy)pyridine

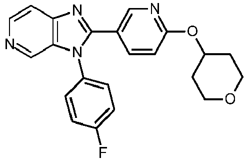

Example 113 was prepared similarly to Example 112, using 4-hydroxytetrahydropyran instead of 3-hydroxytetrahydrofuran, to give the title compound (20.0mg, 20.8%) as a white solid. HRMS (ESI⁺) calcd for [MH]⁺ of C₂₂H₁₉FN₄O₂ 391.1570 found 391.1566. HPLC: Rt 5.04min, 99.7% purity.

### EXAMPLE 114

### 4-[3-(4-Fluorophenyl)-3H-imidazo[4,5-c]pyridin-2-yl]-1-methyl-1,2-dihydropyridin-2-one

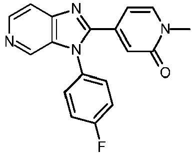

Intermediate 132 (26.0mg, 84.9umol) and Cs₂CO₃ (55.3mg, 0.170mmol) were dissolved in dioxane (0.5mL), Mel (10.6uL, 0.170mmol) was added and the reaction mixture was stirred for 16h, filtered and concentrated *in vacuo.* The residue was purified by reverse phase HPLC to give the title compound (1.41mg, 5.19%) as a white solid. HRMS (ESI⁺) calcd for [MH]⁺ of C₁₈H₁₃FN₄O 321.1151 found 321.1156. HPLC: Rt 3.45min, 98.9% purity.

### EXAMPLE 115

### 1-Cyclopropyl-4-[3-(4-fluorophenyl)-3H-imidazo[4,5-c]pyridin-2-yl]-1,2-dihydropyridin-2-one

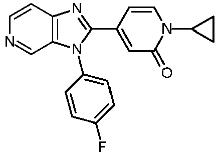

Intermediate 132 (120mg, 0.392mmol), cyclopropylboronic acid (101mg, 1.18mmol), Cu(OAc)₂ (110mg, 0.607mmol), 4,4'-dimethyl-2,2'-bipyridine (72.2mg, 0.392mmol) and Cs₂CO₃ (268mg, 0.823mmol) were suspended in dioxane (2.5mL) and the reaction mixture was stirred at 70°C for 6h. The reaction mixture was partitioned between EtOAc (25mL) and water (25mL) and the organic fraction was washed with brine (25mL), dried (MgSO₄) and concentrated *in vacuo.* The residue was purified by reverse phase HPLC to give the title compound (22.4mg, 16.5%) as an off white solid. HRMS (ESI⁺) calcd for [MH]⁺ of C₂₀H₁₅FN₄O 347.1308 found 347.1309. HPLC: Rt 3.88min, 100% purity.

### EXAMPLE 116

**4-[3-(4-Chloro-2-fluorophenyl)-3H-imidazo[4,5-c]pyridin-2-yl]-1-cyclopropyl-1,2-dihydropyridin-2-one**

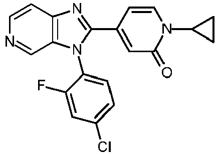

Example 116 was prepared similarly to Example 115, using Example 34 instead of Intermediate 132, to give the title compound (31.0mg, 34.7%) as an off white solid. HRMS (ESI⁺) calcd for [MH]⁺ of C₂₀H₁₄ClFN₄O 381.0918 found 381.0922. HPLC: Rt 4.13min, 100% purity.

### EXAMPLE 117

### N-(2-Methoxyethyl)-N-methyl-5-[3-(4-methylphenyl)-3H-imidazo[4,5-c]pyridin-2-yl]pyrimidin-2-amine

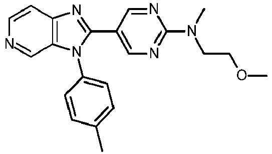

Intermediate 107 (crude) was dissolved in NMP (2mL), Et₃N (633uL, 4.54mmol) was added and the reaction mixture was heated at 180°C in a microwave reactor for 30min. The reaction mixture was partitioned between DCM (15mL) and water (15mL) and the organic fraction was washed with brine (15mL), dried (MgSO₄) and concentrated *in vacuo.* The residue was triturated from MeOH to give the title compound as an off white solid (60.3mg, 10.6%). HRMS (ESI⁺) calcd for [MH]⁺ of C₂₁H₂₂N₆O 375.1933 found 375.1942. HPLC: Rt: 4.99min, 99.3% purity.

### EXAMPLES 118-119

Examples 118-119 were prepared similarly to Example 117, by cyclisation of Intermediates 111 and 113; see Table 17 below.

**Table 17: Cyclisation of Intermediates 111 and 113**

| | | | |
|---|---|---|---|
| 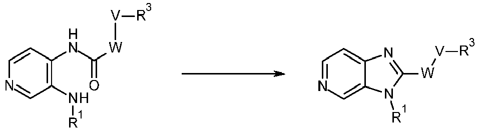 | | | |

| **Ex** | **Structure** | **Name** | **Intermediate(s) used, Form, Yield, LCMS, HPLC** |
|---|---|---|---|
| 118 | 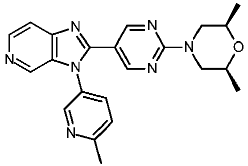 | (2R,6S)-2,6-Dimethyl-4-{5-[3-(6-methylpyridin-3-yl)-3H-imidazo[4,5-c]pyridin-2-yl]pyrimidin-2-yl}morpholine | From Intermediate 111 |
| | | | White solid |
| | | | Yield 58.0mg, 7.23% |
| | | | HRMS (ESI⁺) calcd for [MH]⁺ of C₂₂H₂₃N₇O 402.2042 found 402.2046. |
| | | | HPLC: Rt 4.52min, 97.6% purity |
| 119 | 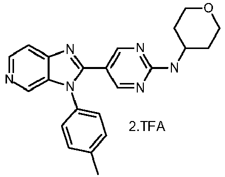 | 5-[3-(4-Methylphenyl)-3H-imidazo[4,5-c]pyridin-2-yl]-*N-*(oxan-4-yl)pyrimidin-2-amine; bis(trifluoroacetic acid) | From Intermediate 113 |
| | | | White solid |
| | | | Yield 12.0mg, 1.55% |
| | | | HRMS (ESI⁺) calcd for [MH]⁺ of C₂₂H₂₂N₆O 387.1933 found 387.1936. |
| | | | HPLC: Rt 4.42min, 99.4% purity |

### REFERENCE EXAMPLE 120

### 4-[1-(4-Chlorophenyl)-1H-pyrrolo[2,3-c]pyridin-2-yl]pyridine

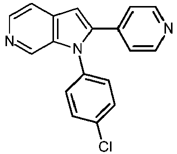

Intermediate 134 (100mg, 0.437mmol) and triisopropyl borate (212uL, 0.918mmol) were dissolved in THF (5mL) and the reaction mixture was cooled to 0°C. LDA (435uL, 2.0M in THF/heptane, 0.875mmol) was added and the reaction mixture was stirred at 0°C for 30min. The reaction was quenched with water (2mL) and diluted with dioxane (3mL). 4-lodopyridine (108mg, 0.525mmol), Pd(PPh₃)₄ (40.4mg, 35.0umol) and a solution of Na₂CO₃ (139mg, 1.31mmol) in water (4mL) were added. The reaction mixture was heated using a microwave reactor at 160°C for 20min. The reaction mixture was partitioned between water (40mL) and EtOAc (40mL), and the organic fraction was dried (MgSO₄) and concentrated *in vacuo.* The residue was purified by reverse phase HPLC to give the title compound as a light yellow solid (21.8mg, 16.3%). HRMS (ESI⁺) calcd for [MH]⁺ of C₁₈H₁₂ClN₃ 306.0798 found 306.0809. HPLC: Rt 3.52min, 99.9% purity.

### REFERENCE EXAMPLES 121-154

Reference Examples 121-154 were prepared similarly to Reference Example 120, by borate formation and Suzuki reaction of Intermediates 134-138 with the appropriate aryl or heteroaryl iodide or bromide; see Table 18 below.

**Table 18: Borate formation and Suzuki reactions of Intermediates 134-138**

| | | | |
|---|---|---|---|
| 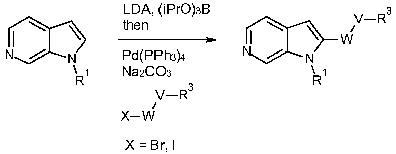 | | | |

| **Ex** | **Structure** | **Name** | **Intermediate(s), Form, Yield, LCMS, HPLC** |
|---|---|---|---|
| 121 | 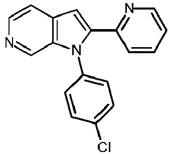 | 2-[1-(4-Chlorophenyl)-1H-pyrrolo[2,3-c]pyridin-2-yl]pyridine | From Intermediate 134 |
| | | | Yellow gum |
| | | | Yield 14.0mg, 6.98% |
| | | | HRMS (ESI⁺) calcd for [MH]⁺ of C₁₈H₁₂ClN₃ 306.0798 found 306.0811. |
| | | | HPLC: Rt 4.82min, 99.1% purity |
| 122 | 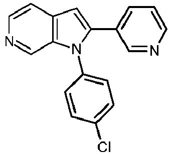 | 3-[1-(4-Chlorophenyl)-1H-pyrrolo[2,3-c]pyridin-2-yl]pyridine | From Intermediate 134 |
| | | | Yellow gum |
| | | | Yield 13.1mg, 9.80% |
| | | | HRMS (ESI⁺) calcd for [MH]⁺ of C₁₈H₁₂ClN₃ 306.0798 found 306.0810. |
| | | | HPLC: Rt 3.95min, 99.1% purity |
| 123 | 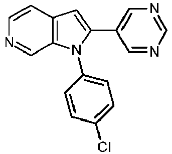 | 5-[1-(4-Chlorophenyl)-1H-pyrrolo[2,3-c]pyridin-2-yl]pyrimidine | From Intermediate 134 |
| | | | White solid |
| | | | Yield 64.9mg, 32.2% |
| | | | HRMS (ESI⁺) calcd for [MH]⁺ of C₁₇H₁₁ClN₄ 307.0750 found 307.0753. |
| | | | HPLC: Rt 4.24min, 99.1% purity |
| 124 | 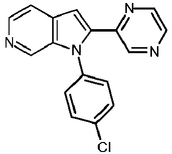 | 2-[1-(4-Chlorophenyl)-1H-pyrrolo[2,3-c]pyridin-2-yl]pyrazine | From Intermediate 134 |
| | | | Yellow gum |
| | | | Yield 28.0mg, 13.9% |
| | | | HRMS (ESI⁺) calcd for [MH]⁺ of C₁₇H₁₁ClN₄ 307.0750 found 307.0764. |
| | | | HPLC: Rt 4.53min, 99.7% purity |
| 125 | 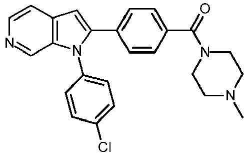 | 1-({4-[1-(4-Chlorophenyl)-1H-pyrrolo[2,3-c]pyridin-2-yl]phenyl}carbonyl)-4-methyl piperazine | From Intermediate 134 and 139 |
| | | | Yellow solid |
| | | | Yield 23.0mg, 8.14% |
| | | | HRMS (ESI⁺) calcd for [MH]⁺ of C₂₅H₂₃ClN₄O 431.1638 found 431.1638. |
| | | | HPLC: Rt 3.77min, 97.9% purity |
| 126 | 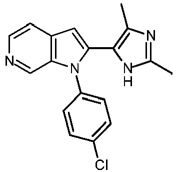 | 5-[1-(4-Chlorophenyl)-1H-pyrrolo[2,3-c]pyridin-2-yl]-2,4-dimethyl-1H-imidazole | From Intermediate 134 |
| | | | Off white solid |
| | | | Yield 14.6mg, 6.90% |
| | | | HRMS (ESI⁺) calcd for [MH]⁺ of C₁₈H₁₅ClN₄ 323.1063 found 323.1067. |
| | | | HPLC: Rt 3.45min, 100% purity |
| 127 | 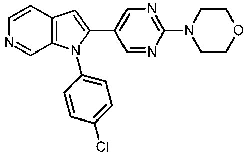 | 4-{5-[1-(4-Chlorophenyl)-1H-pyrrolo[2,3-c]pyridin-2-yl]pyrimidin-2-yl}morpholine | From Intermediate 134 |
| | | | Yellow solid |
| | | | Yield 67.2mg, 19.6% |
| | | | HRMS (ESI⁺) calcd for [MH]⁺ of C₂₁H₁₈ClN₅O 392.1278 found 392.1286. |
| | | | HPLC: Rt 5.12min, 100% purity |
| 128 | 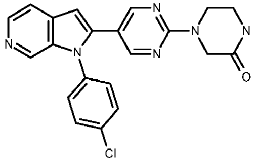 | 4-{5-[1-(4-Chlorophenyl)-1H-pyrrolo[2,3-c]pyridin-2-yl]pyrimidin-2-yl}piperazin-2-one | From Intermediate 134 and 140 |
| | | | White solid |
| | | | Yield 71.5mg, 16.1% |
| | | | HRMS (ESI⁺) calcd for [MH]⁺ of C₂₁H₁₇ClN₆O 405.1230 found 405.1226. |
| | | | HPLC: Rt 4.23min, 98.6% purity. |
| 129 | 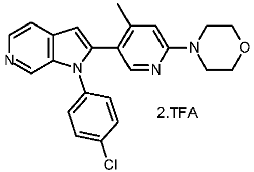 | 4-{5-[1-(4-Chlorophenyl)-1H-pyrrolo[2,3-c]pyridin-2-yl]-4-methylpyridin-2-yl}morpholine; bis(trifluoroacetic acid) | From Intermediate 134 and 143 |
| | | | Colourless gum |
| | | | Yield 6.55mg, 0.95% |
| | | | HRMS (ESI⁺) calcd for [MH]⁺ of C₂₃H₂₁ClN₄O 405.1482 found 405.1494. |
| | | | HPLC: Rt 4.06min, 98.6% purity. |
| 130 | 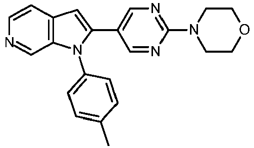 | 4-{5-[1-(4-Methylphenyl)-1H-pyrrolo[2,3-c]pyridin-2-yl]pyrimidin-2-yl}morpholine | From Intermediate 135 |
| | | | White solid |
| | | | Yield 80.0mg, 17.9% |
| | | | HRMS (ESI⁺) calcd for of C₂₂H₂₁N₅O 372.1824 found 372.1828. |
| | | | HPLC: Rt 5.21min, 100% purity. |
| 131 | 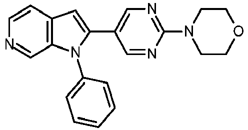 | 4-(5-{1-Phenyl-1H-pyrrolo[2,3-c]pyridin-2-yl}pyrimidin-2-yl)morpholine | From Intermediate 136 |
| | | | White solid |
| | | | Yield 58.5mg, 12.7% |
| | | | HRMS (ESI⁺) calcd for [MH]⁺ of C₂₁H₁₉N₅O 358.1668 found 358.1685. |
| | | | HPLC: Rt 4.90min, 97.4% purity. |
| 132 | 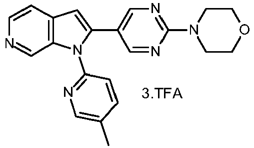 | 4-{5-[1-(5-Methylpyridin-2-yl)-1H-pyrrolo[2,3-c]pyridin-2-yl]pyrimidin-2-yl}morpholine; tris(trifluoroacetic acid) | From Intermediate 137 |
| | | | Yellow solid |
| | | | Yield 3.35mg, 0.87% |
| | | | HRMS (ESI⁺) calcd for [MH]⁺ of C₂₁H₂₀N₆O 373.1777 found 373.1794. |
| | | | HPLC: Rt 4.68min, 99.6% purity. |
| 133 | 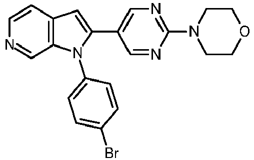 | 4-{5-[1-(4-Bromophenyl)-1H-pyrrolo[2,3-c]pyridin-2-yl]pyrimidin-2-yl}morpholine | From Intermediate 138 |
| | | | Light yellow solid |
| | | | Yield 38.0mg, 9.52% |
| | | | HRMS (ESI⁺) calcd for [MH]⁺ of C₂₁H₁₈BrN₅O 436.0773 found 436.0773. |
| | | | HPLC: Rt 5.32min, 96.2% purity. |
| 134 | 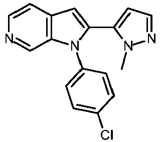 | 5-[1-(4-Chlorophenyl)-1H-pyrrolo[2,3-c]pyridin-2-yl]-1-methyl-1H-pyrazole | From Intermediate 134 |
| | | | Orange solid |
| | | | Yield 133mg, 32.8% |
| | | | HRMS (ESI⁺) calcd for [MH]⁺ of C₁₇H₁₃ClN₄ 309.0907 found 309.0918. |
| | | | HPLC: Rt 4.64min, 98.4% purity. |
| 135 | 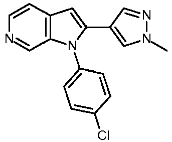 | 4-[1-(4-Chlorophenyl)-1H-pyrrolo[2,3-c]pyridin-2-yl]-1-methyl-1H-pyrazole | From Intermediate 134 |
| | | | Orange solid |
| | | | Yield 153mg, 37.9% |
| | | | HRMS (ESI⁺) calcd for [MH]⁺ of C₁₇H₁₃ClN₄ 309.0907 found 309.0910. |
| | | | HPLC: Rt 4.87min, 99.5% purity. |
| 136 | 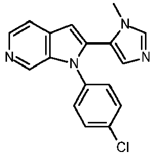 | 5-[1-(4-Chlorophenyl)-1H-pyrrolo[2,3-c]pyridin-2-yl]-1-methyl-1H-imidazole | From Intermediate 134 |
| | | | White solid |
| | | | Yield 63.8mg, 15.8% |
| | | | HRMS (ESI⁺) calcd for [MH]⁺ of C₁₇H₁₃ClN₄ 309.0907 found 309.0914. |
| | | | HPLC: Rt 3.43min, 100% purity. |
| 137 | 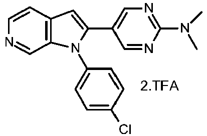 | 5-[1-(4-Chlorophenyl)-1H-pyrrolo[2,3-c]pyridin-2-yl]-*N*,*N-*dimethylpyrimidin-2-amine; bis(trifluoroacetic acid) | From Intermediate 134 |
| | | | Yellow gum |
| | | | Yield 142mg, 18.7% |
| | | | HRMS (ESI⁺) calcd for [MH]⁺ of C₁₉H₁₆ClN₅ 350.1172 found 350.1180. |
| | | | HPLC: Rt 5.46min, 100% purity. |
| 138 | 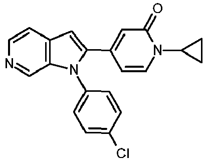 | 4-[1-(4-Chlorophenyl)-1H-pyrrolo[2,3-c]pyridin-2-yl]-1-cyclopropyl-1,2-dihydropyridin-2-one | From Intermediate 134 and 144 |
| | | | Yellow solid |
| | | | Yield 234mg, 36.9% |
| | | | HRMS (ESI⁺) calcd for [MH]⁺ of C₂₁H₁₆ClN₃O 362.1060 found 362.1063. |
| | | | HPLC: Rt 4.76min, 99.0% purity. |
| 139 | 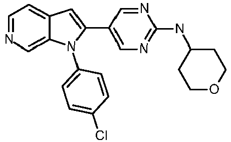 | 5-[1-(4-Chlorophenyl)-1H-pyrrolo[2,3-c]pyridin-2-yl]-*N-*(oxan-4-yl)pyrimidin-2-amine | From Intermediate 134 and 141 |
| | | | White solid |
| | | | Yield 122mg, 22.8% |
| | | | HRMS (ESI⁺) calcd for [MH]⁺ of C₂₂H₂₀ClN₅O 406.1435 found 406.1435. |
| | | | HPLC: Rt 4.78min, 99.3% purity. |
| 140 | 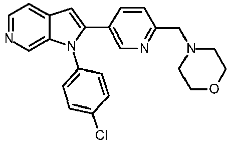 | 4-({5-[1-(4-Chlorophenyl)-1H-pyrrolo[2,3-c]pyridin-2-yl]pyridin-2-yl}methyl)morpholine | From Intermediate 134 and 145 |
| | | | Beige solid |
| | | | Yield 29.7mg, 4.20% |
| | | | HRMS (ESI⁺) calcd for [MH]⁺ of C₂₃H₂₁ClN₄O 405.1482 found 405.1485. |
| | | | HPLC: Rt 3.91min, 98.0% purity. |
| 141 | 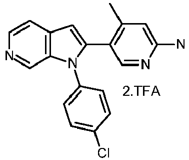 | 5-[1-(4-Chlorophenyl)-1H-pyrrolo[2,3-c]pyridin-2-yl]-4-methylpyridin-2-amine; bis(trifluoroacetic acid) | From Intermediate 134 |
| | | | Colourless gum |
| | | | Yield 1.68mg, 0.34% |
| | | | LCMS (ES⁺): 335.1 [MH]⁺ |
| | | | HPLC: Rt 3.63min, 96.8% purity. |
| 142 | 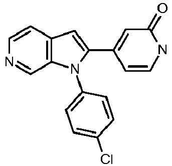 | 4-[1-(4-Chlorophenyl)-1H-pyrrolo[2,3-c]pyridin-2-yl]-1,2-dihydropyridin-2-one | From Intermediate 134 |
| | | | Off white solid |
| | | | Yield 52.0mg, 14.8% |
| | | | HRMS (ESI⁺) calcd for [MH]⁺ of C₁₈H₁₂ClN₃O 322.0747 found 322.0753. |
| | | | HPLC: Rt 3.75min, 97.6% purity. |
| 143 | 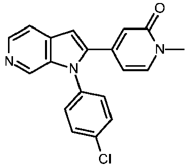 | 4-[1-(4-Chlorophenyl)-1H-pyrrolo[2,3-c]pyridin-2-yl]-1-methyl-1,2-dihydropyridin-2-one | From Intermediate 134 and 146 |
| | | | Off white solid |
| | | | Yield 56.9mg, 15.5% |
| | | | HRMS (ESI⁺) calcd for [MH]⁺ of C₁₉H₁₄ClN₃O 336.0904 found 336.0909. |
| | | | HPLC: Rt 4.07min, 98.6% purity. |
| 144 | 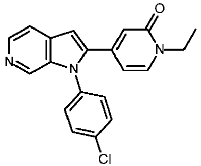 | 4-[1-(4-Chlorophenyl)-1H-pyrrolo[2,3-c]pyridin-2-yl]-1-ethyl-1,2-dihydropyridin-2-one | From Intermediate 134 and 147 |
| | | | Off white solid |
| | | | Yield 59.5mg, 15.6% |
| | | | HRMS (ESI⁺) calcd for [MH]⁺ of C₂₀H₁₆ClN₃O 350.1060 found 350.1065. |
| | | | HPLC: Rt 4.39min, 100% purity. |
| 145 | 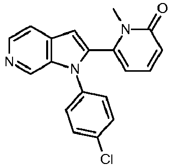 | 6-[1-(4-Chlorophenyl)-1H-pyrrolo[2,3-c]pyridin-2-yl]-1-methyl-1,2-dihydropyridin-2-one | From Intermediate 134 and 148 |
| | | | White solid |
| | | | Yield 82.2mg, 16.0% |
| | | | HRMS (ESI⁺) calcd for [MH]⁺ of C₁₉H₁₄ClN₃O 336.0904 found 336.0914. |
| | | | HPLC: Rt 4.20min, 99.5% purity. |
| 146 | 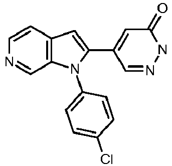 | 5-[1-(4-Chlorophenyl)-1H-pyrrolo[2,3-c]pyridin-2-yl]-2,3-dihydropyridazin-3-one | From Intermediate 134 |
| | | | Yellow solid |
| | | | Yield 35.5mg, 10.1% |
| | | | HRMS (ESI⁺) calcd for [MH]⁺ of C₁₇H₁₁ClN₄O 323.0699 found 323.0700. |
| | | | HPLC: Rt 3.86min, 98.4% purity. |
| 147 | 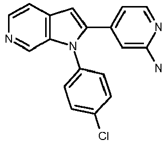 | 4-[1-(4-Chlorophenyl)-1H-pyrrolo[2,3-c]pyridin-2-yl]pyridin-2-amine | From Intermediate 134 |
| | | | Off white solid |
| | | | Yield 103mg, 29.4% |
| | | | HRMS (ESI⁺) calcd for [MH]⁺ of C₁₈H₁₃ClN₄ 321.0907 found 321.0901. |
| | | | HPLC: Rt 3.56min, 100% purity. |
| 148 | 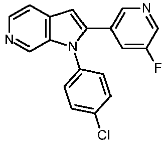 | 3-[1-(4-Chlorophenyl)-1H-pyrrolo[2,3-c]pyridin-2-yl]-5-fluoropyridine | From Intermediate 134 |
| | | | Yellow solid |
| | | | Yield 21.2mg, 6.00% |
| | | | HRMS (ESI⁺) calcd for [MH]⁺ of C₁₈H₁₁ClFN₃ 324.0704 found 324.0714. |
| | | | HPLC: Rt 4.87min, 100% purity. |
| 149 | 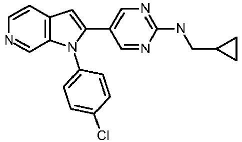 | 5-[1-(4-Chlorophenyl)-1H-pyrrolo[2,3-c]pyridin-2-yl]-*N-*(cyclopropylmethyl)pyrimidin-2-amine | From Intermediate 134 and 142 |
| | | | Off white solid |
| | | | Yield 32.0mg, 7.79% |
| | | | HRMS (ESI⁺) calcd for [MH]⁺ of C₂₁H₁₈ClN₅ 376.1329 found 376.1326. |
| | | | HPLC: Rt 5.38min, 99.1% purity. |
| 150 | 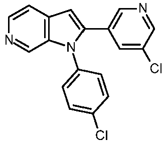 | 3-Chloro-5-[1-(4-chlorophenyl)-1H-pyrrolo[2,3-c]pyridin-2-yl]pyridine | From Intermediate 134 |
| | | | Off white solid |
| | | | Yield 19.2mg, 5.16% |
| | | | HRMS (ESI⁺) calcd for [MH]⁺ of C₁₈H₁₁Cl₂N₃ 340.0408 found 340.0418. |
| | | | HPLC: Rt 5.17min, 100% purity. |
| 151 | 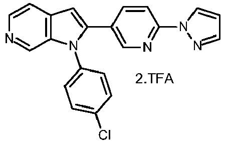 | 5-[1-(4-Chlorophenyl)-1H-pyrrolo[2,3-c]pyridin-2-yl]-2-(1H-pyrazol-1-yl)pyridine; bis(trifluoroacetic acid) | From Intermediate 134 |
| | | | Yellow solid |
| | | | Yield 4.51 mg, 0.69% |
| | | | HRMS (ESI⁺) calcd for [MH]⁺ of C₂₁H₁₄ClN₅ 372.1016 found 372.1025. |
| | | | HPLC: Rt 5.57min, 100% purity. |
| 152 | 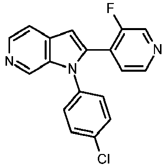 | 4-[1-(4-Chlorophenyl)-1H-pyrrolo[2,3-c]pyridin-2-yl]-3-fluoropyridine | From Intermediate 134 |
| | | | White solid |
| | | | Yield 9.24mg, 2.61% |
| | | | HRMS (ESI⁺) calcd for [MH]⁺ of C₁₈H₁₁ClFN₃ 324.0704 found 324.0710. |
| | | | HPLC: Rt 4.74min, 100% purity. |
| 153 | 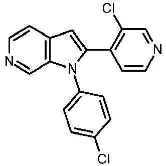 | 3-Chloro-4-[1-(4-chlorophenyl)-1H-pyrrolo[2,3-c]pyridin-2-yl]pyridine | From Intermediate 134 |
| | | | White solid |
| | | | Yield 40.1 mg, 9.29% |
| | | | HRMS (ESI⁺) calcd for [MH]⁺ of C₁₈H₁₁Cl₂N₃ 340.0408 found 340.0421. |
| | | | HPLC: Rt 4.98min, 100% purity. |
| 154 | 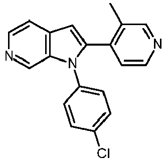 | 4-[1-(4-Chlorophenyl)-1H-pyrrolo[2,3-c]pyridin-2-yl]-3-methylpyridine | From Intermediate 134 |
| | | | White solid |
| | | | Yield 29.5mg, 8.45% |
| | | | HRMS (ESI⁺) calcd for [MH]⁺ of C₁₉H₁₄ClN₃ 320.0954 found 320.0958. |
| | | | HPLC: Rt 3.71min, 100% purity. |

### REFERENCE EXAMPLE 155

### 1-Cyclopropyl-4-{1-phenyl-1H-pyrrolo[2,3-c]pyridin-2-yl}-1,2-dihydropyridin-2-one

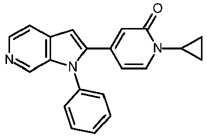

Reference Example 138 (50.0mg, 0.138mmol) was dissolved in MeOH (15mL) and passed through an H-cube (70x4mm 10% Pd/C CatCart, 1.0mL/min, 30°C, 20bar). The solvents were removed *in vacuo* and the residue purified by reverse phase HPLC to give the title compound (0.610mg, 1.35%) as a colourless gum. LCMS (ES⁺): 328.0 [MH]⁺. HPLC: Rt 4.50min, 96.7% purity.

### BIOLOGICAL TESTS

### Biological Assays of the SSAO Enzyme Inhibitors

All primary assays were performed at RT. with purified recombinantly expressed human SSAO. Enzyme was prepared essentially as described in Ohman et al. (Protein Expression and Purification 46 (2006) 321-331). In addition, secondary- and selectivity assays were performed using SSAO prepared from various tissues or purified rat recombinant SSAO. The enzyme activity was assayed with benzylamine as substrate by measuring either benzaldehyde production, using ¹⁴C-labeled substrate, or by utilizing the production of hydrogen peroxide in a horseradish peroxidase (HRP) coupled reaction. Briefly, test compounds were dissolved in dimethyl sulfoxide (DMSO) to a concentration of 10mM. Dose-response measurements were assayed by either creating 1:10 serial dilutions in DMSO to produce a 7 point curve or by making 1:3 serial dilutions in DMSO to produce 11 point curves. The top concentrations were adjusted depending on the potency of the compounds and subsequent dilution in reaction buffer yielded a final DMSO concentration ≤ 2%.

### Hydrogen peroxide detection:

In a horseradish peroxidase (HRP) coupled reaction, hydrogen peroxide oxidation of 10-acetyl-3,7-dihydroxyphenoxazine produced resorufin, which is a highly fluorescent compound (Zhout and Panchuk-Voloshina. Analytical Biochemistry 253 (1997) 169-174; Amplex® Red Hydrogen Peroxide/peroxidase Assay kit, Invitrogen A22188). Enzyme and compounds in 50mM sodium phosphate, pH 7.4 were set to pre-incubate in flat-bottomed microtiter plates for approximately 15min before initiating the reaction by addition of a mixture of HRP, benzylamine and Amplex reagent. Benzylamine concentration was fixed at a concentration corresponding to the Michaelis constant, determined using standard procedures. Fluorescence intensity was then measured at several time points during 1-2h, exciting at 544nm and reading the emission at 590nm. For the human SSAO assay final concentrations of the reagents in the assay wells were: SSAO enzyme 1ug/ml, benzylamine 100uM, Amplex reagent 20uM, HRP 0.1 U/mL and varying concentrations of test compound. The inhibition was measured as % decrease of the signal compared to a control without inhibitor (only diluted DMSO). The background signal from a sample containing no SSAO enzyme was subtracted from all data points. Data was fitted to a four parameter logistic model and IC₅₀ values were calculated using the GraphPad Prism 4 or XLfit 4 programs.

### Aldehyde detection:

SSAO activity was assayed using 14C-labeled benzylamine and analysed by measuring radioactive benzaldehyde. In a white 96-well optiplate (Packard), 20uL of diluted test compound was pre-incubated at room temperature with 20uL SSAO enzyme for approximately 15min with continuous agitation. All dilutions were made with PBS. The reaction was initiated by adding 20uL of the benzylamine substrate solution containing [7-14C] Benzylamine hydrochloride (CFA589, GE Healthcare). The plate was incubated for 1h as above after which the reaction was stopped by acidification (10uL 1M aq HCl). Then 90uL Micro Scint-E solution (Perkin-Elmer) was added to each well and the plate was continuously mixed for 15min. Phase separation occurred instantly and activity was read in a Topcount scintillation counter (Perkin-Elmer). In the final reaction well, the human recombinant SSAO concentration was 10ug/ml. In order to optimize sensitivity, the substrate concentration was decreased as compared to the HRP coupled assay in order to get a higher fraction of radioactive product. In the human SSAO assay, benzylamine concentration was 40uM (0.2uCi/mL). Data was analysed as above.

All of the exemplified compounds of the invention had an IC₅₀ value of between 1nM and 1200nM at SSAO (see Table 19 below).

**Table 19: SSAO inhibitory activity (A: <50nM, B: 50-200nM, C: 200-1200nM)**

| **Compound** | **SSAO IC₅₀ (nM)** | **Compound** | **SSAO IC₅₀ (nM)** | **Compound** | **SSAO IC₅₀ (nM)** |
|---|---|---|---|---|---|
| **1** | C | **53** | C | **105** | C |
| **2** | C | **54** | C | **106** | B |
| **3** | B | **55** | A | **107** | A |
| **4** | A | **56** | C | **108** | C |
| **5** | B | **57** | B | **109** | C |
| **6** | C | **58** | C | **110** | C |
| **7** | B | **59** | B | **111** | C |
| **8** | A | **60** | B | **112** | A |
| **9** | A | **61** | A | **113** | A |
| **10** | B | **62** | B | **114** | C |
| **11** | A | **63** | C | **115** | A |
| **12** | B | **64** | C | **116** | B |
| **13** | A | **65** | A | **117** | C |
| **14** | C | **66** | B | **118** | A |
| **15** | B | **67** | A | **119** | B |
| **16** | C | **68** | A | **120** | C |
| **17** | C | **69** | A | **121** | A |
| **18** | B | **70** | A | **122** | B |
| **19** | B | **71** | C | **123** | A |
| **20** | B | **72** | C | **124** | A |
| **21** | A | **73** | B | **125** | C |
| **22** | A | **74** | C | **126** | A |
| **23** | A | **75** | A | **127** | A |
| **24** | A | **76** | B | **128** | A |
| **25** | A | **77** | C | **129** | A |
| **26** | B | **78** | A | **130** | B |
| **27** | A | **79** | A | **131** | C |
| **28** | B | **80** | B | **132** | B |
| **29** | C | **81** | A | **133** | A |
| **30** | C | **82** | A | **134** | A |
| **31** | C | **83** | A | **135** | A |
| **32** | A | **84** | A | **136** | A |
| **33** | C | **85** | A | **137** | A |
| **34** | B | **86** | A | **138** | A |
| **35** | C | **87** | A | **139** | A |
| **36** | B | **88** | A | **140** | A |
| **37** | A | **89** | A | **141** | B |
| **38** | B | **90** | A | **142** | B |
| **39** | A | **91** | C | **143** | A |
| **40** | B | **92** | A | **144** | B |
| **41** | A | **93** | B | **145** | A |
| **42** | B | **94** | C | **146** | B |
| **43** | A | **95** | B | **147** | A |
| **44** | C | **96** | B | **148** | A |
| **45** | C | **97** | C | **149** | A |
| **46** | A | **98** | B | **150** | B |
| **47** | A | **99** | A | **151** | B |
| **48** | A | **100** | C | **152** | C |
| **49** | A | **101** | B | **153** | B |
| **50** | A | **102** | B | **154** | A |
| **51** | A | **103** | B | **155** | C |
| **52** | A | **104** | A | | |

### HERG ASSAY

Compounds of the invention were tested for inhibition of the human ether a go-go related gene (hERG) K⁺ channel using IonWorks patch clamp electrophysiology. 8 Point concentration-response curves were generated on two occasions using 3-fold serial dilutions from the maximum assay concentration (11uM). Electrophysiological recordings were made from a Chinese Hamster Lung cell line stably expressing the full length hERG channel. Single cell ion currents were measured in the perforated patch clamp configuration (100ug/mL amphoterocin) at room temperature using an IonWorks Quattro instrument. The internal solution contained 140mM KCI, 1mM MgCl₂, 1mM EGTA and 20mM HEPES and was buffered to pH 7.3. The external solution contained 138mM NaCl, 2.7mM KCI, 0.9mM CaCl₂, 0.5mM MgCl₂, 8mM Na₂HPO₄ and 1.5mM KH₂PO₄, and was buffered to pH 7.3. Cells were clamped at a holding potential of 70mV for 30s and then stepped to +40mV for 1s. This was followed by a hyperpolarising step of 1s to 30mV to evoke the hERG tail current. This sequence was repeated 5 times at a frequency of 0.25Hz. Currents were measured from the tail step at the 5^{th} pulse, and referenced to the holding current. Compounds were incubated for 6-7min prior to a second measurement of the hERG signal using an identical pulse train. A minimum of 17 cells were required for each plC50 curve fit. A control compound (quinidine) was used (see Table 20 below).

**Table 20: hERG IC50 (A: >10uM, B: 1-10uM, C: 0.1uM-1uM)**

| **Compound** | **hERG IC50** | **Compound** | **hERG IC50** | **Compound** | **hERG IC50)** |
|---|---|---|---|---|---|
| **2** | A | **57** | A | **98** | A |
| **3** | A | **63** | A | **110** | A |
| **4** | B | **64** | A | **117** | A |
| **9** | B | **67** | B | **120** | B |
| **11** | A | **69** | A | **122** | B |
| **12** | A | **70** | A | **127** | C |
| **13** | A | **71** | A | **128** | A |
| **18** | A | **75** | A | **130** | C |
| **20** | A | **77** | A | **131** | B |
| **21** | A | **78** | A | **132** | C |
| **23** | A | **80** | A | **136** | B |
| **24** | A | **81** | A | **138** | B |
| **46** | A | **82** | A | **139** | C |
| **47** | B | **85** | A | **140** | B |
| **48** | B | **86** | A | **142** | B |
| **50** | A | **88** | B | **147** | B |
| **51** | B | **89** | A | **152** | B |
| **52** | A | **91** | A | **153** | B |
| **53** | A | **92** | A | | |
| **54** | A | **95** | A | | |

## Claims

1. A compound of formula (I) or a pharmaceutically acceptable salt, or N-oxide thereof: 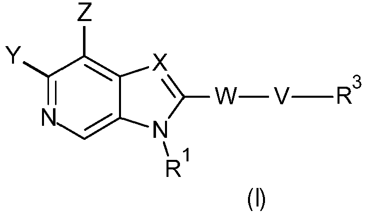 Wherein:
**Y** is selected from hydrogen, hydroxyl, -NH₂, -NH-C₁₋₄-alkyl, -NH-halo-C₁₋₄-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, a 3-7 membered cycloalkyl ring, -OR⁵,-NR⁶C(O)OR⁵, -NR⁶C(O)R⁵, -NR⁶C(O)NR^{4A}R^{4B}, -C(O)NR^{4A}R^{4B}, -C(O)R⁵, -C(O)OR⁵, and -NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is -N=;
**W** is a phenyl ring or a 5 or 6-membered heteroaryl ring selected from pyridinyl, pyridazinyl, pyrazinyl, pyrimidinyl, oxazolyl, thiazolyl or imidazolyl, any of which rings being optionally substituted with one or more substituents selected from halogen, cyano, oxo, C₁₋₄-alkyl, halo-C₁₋₄-alkyl, cyano-C₁₋₄-alkyl, -OR⁵, -NR^{7A}R^{7B},-NR⁶C(O)OR⁵, -NR⁶C(O)R⁵, -NR⁶C(O)NR^{7A}R^{7B}, -C(O)NR^{7A}R^{7B}, -C(O)R⁵, -C(O)OR⁵,-SO₂R⁵, -SO₂NR^{7A}R^{7B} and -NR⁶S(O)₂R⁵;
R^{7A} and R^{7B} are independently hydrogen, C₁₋₄-alkyl or halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is selected from hydrogen, -C₁₋₄-alkyl, -C₁₋₄-alkyl-C₁₋₄-alkoxy or a 3-7 membered heterocyclic ring or 3-7 membered cycloalkyl ring, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵, -NR⁶C(O)NR^{4A}R^{aB},-C(O)NR^{4A}R^{4B}, -C(O)R⁵, -C(O)OR⁵, -SO₂R⁵, -SO₂NR^{4A}R^{4B} and -NR⁶S(O)₂R⁵;
PROVIDED THAT groups -WVR³ and/or R¹ are not: 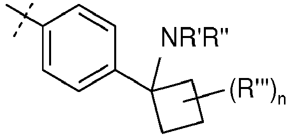 wherein
n is 0, 1, or 2;
R' and R" are independently selected from the group consisting of H, -C₁-C₆alkyl,-(C=O)-C₁-C₆ alkyl and -(C=O)OC(CH₃)₃; and
R''' is H, OH, or C₁-C₆ alkyl.

2. A compound of formula (I) or a pharmaceutically acceptable salt, or N-oxide thereof: 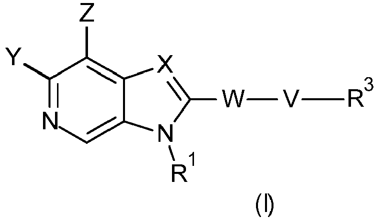 Wherein:
**Y** is selected from hydrogen, hydroxyl, -NH₂, -NH-C₁₋₄-alkyl, -NH-halo-C₁₋₄-alkyl, or -C₁₋₄-alkoxy;
**Z** is selected from hydrogen, halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, or-NHhalo-C₁₋₄-alkyl;
**R¹** is a phenyl ring, or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR⁶C(O)OR⁵, -NR⁶C(O)R⁵,-NR⁶C(O)NR^{4A}R^{4B}, -C(O)NR^{4A}R^{4B}, -C(O)R⁵, -C(O)OR⁵, and -NR⁶S(O)₂R⁵; wherein
R^{4A}, R^{4B} R⁵ and R⁶ are each independently selected from hydrogen, C₁₋₄-alkyl or halo-C₁₋₄-alkyl, or
R^{4A} and R^{4B} together with the nitrogen to which they are attached form a 3-7 membered cyclic amino group, optionally substituted by one or more substituents selected from: halogen, hydroxyl, cyano, C₁₋₄-alkyl, halo-C₁₋₄-alkyl, C₁₋₄-alkoxy, halo-C₁₋₄-alkoxy, -CONH₂, -SO₂NH₂, -NH₂, -NHC₁₋₄-alkyl, -NHhalo-C₁₋₄-alkyl;
**X** is -N=;
**W** is a phenyl ring or a 5 or 6-membered heteroaryl ring, either ring being optionally substituted with one or more substituents selected from halogen, cyano, C₁₋₄-alkyl, halo-C₁₋₄-alkyl, cyano-C₁₋₄-alkyl, -OR⁵, -NR^{7A}R^{7B}, -NR⁶C(O)OR⁵,-NR⁶C(O)R⁵, -NR⁶C(O)NR^{7A}R^{7B}, -C(O)NR^{7A}R^{7B}, -C(O)R⁵, -C(O)OR⁵, -SO₂R⁵,-SO₂NR^{7A}R^{7B} and -NR⁶S(O)₂R⁵;
R^{7A} and R^{7B} are independently hydrogen, C₁₋₄-alkyl or halo-C₁₋₄-alkyl.
**V** is selected from a bond, -O-, -N(R⁶)-, -(C=O)-, -CONR⁶-, -NR⁶C(O)-, or -C₁₋₄-alkylene-, wherein the C₁₋₄-alkylene group is optionally substituted by halogen, and wherein any one of the carbon atoms of the C₁₋₄-alkylene group may be replaced by -O- or -N(R⁶)-;
**R³** is hydrogen, or a 3-7 membered heterocyclic ring, or 3-7 membered cycloalkyl ring selected from cyclopropyl, cyclopentyl or cyclohexyl, or a 5 or 6-membered heteroaryl ring, any one of the rings being optionally substituted with one or more substituents selected from halogen, oxo, hydroxyl, cyano, C₁₋₄-alkyl, halo-C₁₋₄-alkyl, cyano-C₁₋₄-alkyl, -OR⁵, -NR^{4A}R^{4B}, -NR⁶C(O)OR⁵, -NR⁶C(O)R⁵,-NR⁶C(O)NR^{4A}R^{4B} -C(O)NR^{4A}R^{4B}, -C(O)R⁵, -C(O)OR⁵, -SO₂R⁵, -SO₂NR^{4A}R^{4B} and-NR⁶S(O)₂R⁵.

3. A compound according to claim 1 or 2 wherein Y and Z are both hydrogen.

4. A compound according to any one of claims 1 to 3 wherein R¹ is phenyl or 6-membered heteroaryl, optionally substituted with one or more substituents selected from halogen, C₁₋₄-alkyl or halo-C₁₋₄-alkyl.

5. A compound according to any one of claims 1 to 4 wherein W is:
(i) a phenyl ring optionally substituted with one or more substituents as defined in claim 1 or claim 2; or
(ii) a 6-membered heteroaryl ring selected from pyridine, pyridazine, pyrazine, or pyrimidine optionally substituted with one or more substituents as defined in claim 1 or claim 2; or
(iii) a 5-membered heteroaryl ring selected from oxazole, thiazole or imidazole optionally substituted with one or more substituents as defined in claim 1 or claim 2.

6. A compound according to any one of claims 1 to 5 wherein V is selected from the group consisting of a direct bond, -CH₂-, -(CH₂)₂-, or -N(R⁶)CH₂-, and-CH₂-N(R⁶)-.

7. A compound according to claim 1, or any one of claims 3 to 6 when dependent on claim 1, wherein R³ is cyclopropyl, cyclobutyl, cyclopentyl or cyclohexyl optionally substituted as defined in claim 1.

8. A compound according to any one of claims 1 to 5 wherein R³ is a ring substituted with-NR^{4A}R^{4B} wherein R^{4A} and R^{4B}, together with the nitrogen atom to which they are attached join together to form a 4-7 membered heterocyclic ring optionally substituted as defined in claim 1 or claim 2.

9. A compound according to any one of claims 1 to 5 wherein R³ is selected from the group consisting of: 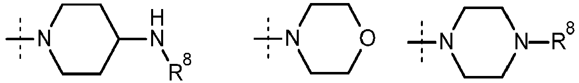 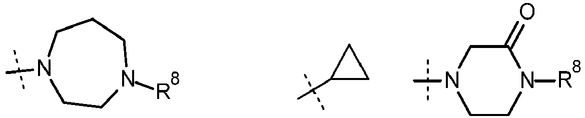 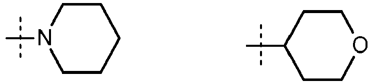 wherein R⁸ is selected from hydrogen, CH₃, -CONH₂, -NHCONH₂, -S(O)₂CH₃,-COCH₃.

10. A compound according to claim 1, wherein the compound is of formula (Xa) 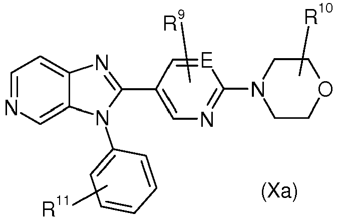
wherein E is -C= or -N=,
R⁹ and R¹⁰ are each independently one or more substituents selected from hydrogen, halogen, cyano, oxo, C₁₋₄-alkyl, -OC₁₋₄-alkyl, or halo-C₁₋₄-alkyl;
R¹¹ is one or more substituents selected from hydrogen, halogen, cyano, cyclopropyl, C₁₋₄-alkyl, or halo-C₁₋₄-alkyl.

11. A compound according to claim 1 or claim 2 selected from:
3-[3-(4-Chlorophenyl)-3H-imidazo[4,5-c]pyridin-2-yl]pyridine (Example 1);
4-[3-(4-Chlorophenyl)-3H-imidazo[4,5-c]pyridin-2-yl]pyridine (Example 2);
4-({5-[3-(4-Chlorophenyl)-3H-imidazo[4,5-c]pyridin-2-yl]pyridin-2-yl}methyl)morpholine (Example 3);
4-{6-[3-(4-Chlorophenyl)-3H-imidazo[4,5-c]pyridin-2-yl]pyridazin-3-yl}morpholine (Example 4);
4-{5-[3-(4-Chlorophenyl)-3H-imidazo[4,5-c]pyridin-2-yl]pyrazin-2-yl}morpholine (Example 5);
4-({5-[3-(4-Chlorophenyl)-3H-imidazo[4,5-c]pyridin-2-yl]pyridin-2-yl}carbonyl)morpholine (Example 6);
5-[3-(4-Chlorophenyl)-3H-imidazo[4,5-c]pyridin-2-yl]-*N*-(oxan-4-yl)pyrazin-2-amine (Example 7);
1-{5-[3-(4-Chlorophenyl)-3H-imidazo[4,5-c]pyridin-2-yl]pyridin-2-yl}piperidin-4-amine (Example 8);
*N*-(Cyclopropylmethyl)-5-[3-(4-fluorophenyl)-3H-imidazo[4,5-c]pyridin-2-yl]pyrimidin-2-amine (Example 9);
*N*-Cyclopropyl-5-[3-(4-fluorophenyl)-3H-imidazo[4,5-c]pyridin-2-yl]pyrimidin-2-amine (Example 10);
5-[3-(4-Chlorophenyl)-3H-imidazo[4,5-c]pyridin-2-yl]-*N*-(oxan-4-yl)pyrimidin-2-amine (Example 11);
4-{5-[3-(4-Fluorophenyl)-3H-imidazo[4,5-c]pyridin-2-yl]pyrimidin-2-yl}piperazin-2-one (Example 12);
4-{5-[3-(4-Chlorophenyl)-3H-imidazo[4,5-c]pyridin-2-yl]pyrimidin-2-yl}piperazin-2-one (Example 13);
5-[3-(4-Chlorophenyl)-3H-imidazo[4,5-c]pyridin-2-yl]-*N*-cyclopropylpyridine-2-carboxamide (Example 14);
3-[3-(4-Fluorophenyl)-3H-imidazo[4,5-c]pyridin-2-yl]-6-(oxan-4-yl)pyridazine (Example 15);
*N*-{5-[3-(4-Chlorophenyl)-3H-imidazo[4,5-c]pyridin-2-yl]pyridin-2-yl}methanesulfonamide (Example 16);
1-{4-[3-(4-Chlorophenyl)-3H-imidazo[4,5-c]pyridin-2-yl]-1,3-thiazol-2-yl}piperazine (Example 17);
1-{5-[3-(4-Chlorophenyl)-3H-imidazo[4,5-c]pyridin-2-yl]-1,3-oxazol-2-yl}piperazine (Example 18);
1-{5-[3-(4-Chlorophenyl)-3H-imidazo[4,5-c]pyridin-2-yl]-1,3-thiazol-2-yl}piperazine (Example 19);
5-[3-(4-Fluorophenyl)-3H-imidazo[4,5-c]pyridin-2-yl]-*N*-(oxan-4-yl)pyrimidin-2-amine (Example 20);
4-{5-[3-(4-Fluorophenyl)-3H-imidazo[4,5-c]pyridin-2-yl]-4-methylpyridin-2-yl}morpholine (Example 21);
4-{5-[3-(4-Chloro-2-fluorophenyl)-3H-imidazo[4,5-c]pyridin-2-yl]-4-methylpyridin-2-yl}morpholine (Example 22);
(2R,6S)-4-{5-[3-(4-Fluorophenyl)-3H-imidazo[4,5-c]pyridin-2-yl]pyrimidin-2-yl}-2,6-dimethylmorpholine (Example 23);
4-{5-[3-(4-Fluorophenyl)-3H-imidazo[4,5-c]pyridin-2-yl]pyrimidin-2-yl}-2,2-dimethylmorpholine (Example 24);
4-{5-[3-(4-Fluorophenyl)-3H-imidazo[4,5-c]pyridin-2-yl]pyrimidin-2-yl}-1,4-oxazepane (Example 25);
4-{5-[3-(4-Fluorophenyl)-3H-imidazo[4,5-c]pyridin-2-yl]-4-methylpyrimidin-2-yl}morpholine (Example 26);
4-{5-[3-(4-Chloro-2-fluorophenyl)-3H-imidazo[4,5-c]pyridin-2-yl]-4-methylpyrimidin-2-yl}morpholine (Example 27);
4-{5-[3-(4-Fluorophenyl)-3H-imidazo[4,5-c]pyridin-2-yl]-6-methoxypyridin-2-yl}morpholine (Example 28);
4-{5-[3-(4-Fluorophenyl)-3H-imidazo[4,5-c]pyridin-2-yl]-4,6-dimethylpyridin-2-yl}morpholine (Example 29);
2-Cyclopropyl-5-[3-(4-fluorophenyl)-3H-imidazo[4,5-c]pyridin-2-yl]pyrimidine (Example 30);
5-[3-(4-Chlorophenyl)-3H-imidazo[4,5-c]pyridin-2-yl]pyrimidin-2-amine (Example 31);
4-[3-(4-Chlorophenyl)-3H-imidazo[4,5-c]pyridin-2-yl]-1-methyl-1,2-dihydropyridin-2-one (Example 32);
5-[3-(4-Chlorophenyl)-3H-imidazo[4,5-c]pyridin-2-yl]-1-methyl-1,2-dihydropyridin-2-one (Example 33);
4-[3-(4-Chloro-2-fluorophenyl)-3H-imidazo[4,5-c]pyridin-2-yl]-1,2-dihydropyridin-2-one (Example 34);
5-[3-(4-Chloro-2-fluorophenyl)-3H-imidazo[4,5-c]pyridin-2-yl]-1,2-dihydropyridin-2-one (Example 35);
(2R,6S)-2,6-Dimethyl-4-{5-[3-(5-methylpyridin-2-yl)-3H-imidazo[4,5-c]pyridin-2-yl]pyrimidin-2-yl}morpholine (Example 36);
*N*-(3-Methoxypropyl)-5-[3-(4-methylphenyl)-3H-imidazo[4,5-c]pyridin-2-yl]pyrimidin-2-amine (Example 37);
5-[3-(4-Fluorophenyl)-3H-imidazo[4,5-c]pyridin-2-yl]-*N*-[2-(propan-2-yloxy)ethyl]pyrimidin-2-amine (Example 38);
5-[3-(4-Methylphenyl)-3H-imidazo[4,5-c]pyridin-2-yl]-*N*-[2-(propan-2-yloxy)ethyl]pyrimidin-2-amine (Example 39);
4-{5-[3-(4-Fluorophenyl)-3H-imidazo[4,5-c]pyridin-2-yl]pyrimidin-2-yl}-1-methylpiperazin-2-one (Example 40);
4-{5-[3-(2,4-Difluorophenyl)-3H-imidazo[4,5-c]pyridin-2-yl]pyridin-2-yl}morpholine (Example 41);
*N*-(2-Ethoxyethyl)-5-[3-(4-fluorophenyl)-3H-imidazo[4,5-c]pyridin-2-yl]pyrimidin-2-amine (Example 42);
*N*-(2-Ethoxyethyl)-5-[3-(4-methylphenyl)-3H-imidazo[4,5-c]pyridin-2-yl]pyrimidin-2-amine (Example 43);
5-[3-(4-Chlorophenyl)-3H-imidazo[4,5-c]pyridin-2-yl]-1H-imidazole (Example 44);
1-({3-[3-(4-Chlorophenyl)-3H-imidazo[4,5-c]pyridin-2-yl]phenyl}methyl)-4-methylpiperazine (Example 45);
1-({4-[3-(4-Chlorophenyl)-3H-imidazo[4,5-c]pyridin-2-yl]phenyl}methyl)-4-methylpiperazine (Example 46);
4-{5-[3-(4-Chlorophenyl)-3H-imidazo[4,5-c]pyridin-2-yl]pyridin-2-yl}morpholine (Example 47);
1-({4-[3-(4-Chlorophenyl)-3H-imidazo[4,5-c]pyridin-2-yl]phenyl}methyl)-1H-imidazole (Example 48);
4-({4-[3-(4-Chlorophenyl)-3H-imidazo[4,5-c]pyridin-2-yl]phenyl}methyl)morpholine (Example 49);
1-{5-[3-(4-Chlorophenyl)-3H-imidazo[4,5-c]pyridin-2-yl]pyridin-2-yl}piperazine (Example 50);
4-{5-[3-(4-Chlorophenyl)-3H-imidazo[4,5-c]pyridin-2-yl]pyrimidin-2-yl}morpholine (Example 51);
4-{5-[3-(4-Fluorophenyl)-3H-imidazo[4,5-c]pyridin-2-yl]pyrimidin-2-yl}morpholine (Example 52);
4-{5-[3-(2-Fluoro-4-methylphenyl)-3H-imidazo[4,5-c]pyridin-2-yl]pyrimidin-2-yl}morpholine (Example 53);
4-{5-[3-(4-Fluorophenyl)-3H-imidazo[4,5-c]pyridin-2-yl]pyridin-2-yl}morpholine (Example 54);
4-{5-[3-(4-Fluoro-2-methylphenyl)-3H-imidazo[4,5-c]pyridin-2-yl]pyrimidin-2-yl}morpholine (Example 55);
4-{5-[3-(2-Chloro-4-fluorophenyl)-3H-imidazo[4,5-c]pyridin-2-yl]pyrimidin-2-yl}morpholine (Example 56);
4-{5-[3-(4-Methylphenyl)-3H-imidazo[4,5-c]pyridin-2-yl]pyrimidin-2-yl}morpholine (Example 57);
4-{5-[3-(6-Methylpyridin-3-yl)-3H-imidazo[4,5-c]pyridin-2-yl]pyrimidin-2-yl}morpholine (Example 58);
4-{5-[3-(4-Bromophenyl)-3H-imidazo[4,5-c]pyridin-2-yl]pyrimidin-2-yl}morpholine (Example 59);
4-{5-[3-(2-Fluorophenyl)-3H-imidazo[4,5-c]pyridin-2-yl]pyrimidin-2-yl}morpholine (Example 60);
4-{5-[3-(2-Chloro-4-fluorophenyl)-3H-imidazo[4,5-c]pyridin-2-yl]pyridin-2-yl}morpholine (Example 61);
4-{5-[3-(4-Fluoro-2-methylphenyl)-3H-imidazo[4,5-c]pyridin-2-yl]pyridin-2-yl}morpholine (Example 62);
4-{5-[3-(4-Methylphenyl)-3H-imidazo[4,5-c]pyridin-2-yl]pyridin-2-yl}morpholine (Example 63);
4-{5-[3-(6-Methylpyridin-3-yl)-3H-imidazo[4,5-c]pyridin-2-yl]pyridin-2-yl}morpholine (Example 64);
4-{2-[6-(Morpholin-4-yl)pyridin-3-yl]-3H-imidazo[4,5-c]pyridin-3-yl}phenol (Example 65);
4-(5-{3-[4-(Trifluoromethyl)phenyl]-3H-imidazo[4,5-c]pyridin-2-yl}pyridin-2-yl)morpholine (Example 66);
4-{5-[3-(2-Fluoro-4-methylphenyl)-3H-imidazo[4,5-c]pyridin-2-yl]pyridin-2-yl}morpholine (Example 67);
4-{5-[3-(2-Fluorophenyl)-3H-imidazo[4,5-c]pyridin-2-yl]pyridin-2-yl}morpholine (Example 68);
5-[3-(4-Fluorophenyl)-3H-imidazo[4,5-c]pyridin-2-yl]-2-(pyrrolidin-1-yl)pyrimidine (Example 69);
4-{5-[3-(4-Fluorophenyl)-3H-imidazo[4,5-c]pyridin-2-yl]pyridin-2-yl}-2-methylmorpholine (Example 70);
5-[3-(4-Fluorophenyl)-3H-imidazo[4,5-c]pyridin-2-yl]-*N*,*N*-dimethylpyridin-2-amine (Example 71);
5-[3-(4-Chlorophenyl)-3H-imidazo[4,5-c]pyridin-2-yl]-*N*,*N*-dimethylpyrimidin-2-amine (Example 72);
4-{4-[3-(4-Fluorophenyl)-3H-imidazo[4,5-c]pyridin-2-yl]pyridin-2-yl}morpholine (Example 73);
4-{5-[3-(4-Chloro-3-fluorophenyl)-3H-imidazo[4,5-c]pyridin-2-yl]pyrimidin-2-yl}morpholine
(Example 74);
4-{5-[3-(5-Chloropyridin-2-yl)-3H-imidazo[4,5-c]pyridin-2-yl]pyrimidin-2-yl}morpholine (Example 75);
4-{5-[3-(5-Fluoropyridin-2-yl)-3H-imidazo[4,5-c]pyridin-2-yl]pyrimidin-2-yl}morpholine (Example 76);
4-{5-[3-(4-Chloro-2-fluorophenyl)-3H-imidazo[4,5-c]pyridin-2-yl]pyrimidin-2-yl}morpholine (Example 77);
4-{5-[3-(2,4-Difluorophenyl)-3H-imidazo[4,5-c]pyridin-2-yl]pyrimidin-2-yl}morpholine (Example 78);
4-{5-[3-(5-Methylpyridin-2-yl)-3H-imidazo[4,5-c]pyridin-2-yl]pyrimidin-2-yl}morpholine (Example 79);
4-{5-[3-(4-Chloro-2-fluorophenyl)-3H-imidazo[4,5-c]pyridin-2-yl]pyridin-2-yl}morpholine (Example 80);
4-{5-[3-(5-Chloropyridin-2-yl)-3H-imidazo[4,5-c]pyridin-2-yl]pyridin-2-yl}morpholine (Example 81);
4-{5-[3-(5-Methylpyridin-2-yl)-3H-imidazo[4,5-c]pyridin-2-yl]pyridin-2-yl}morpholine (Example 82);
5-[3-(4-Chloro-2-fluorophenyl)-3H-imidazo[4,5-c]pyridin-2-yl]-2-(pyrrolidin-1-yl)pyrimidine(Example 83);
5-[3-(4-Chloro-2-fluorophenyl)-3H-imidazo[4,5-c]pyridin-2-yl]-*N*,*N*-dimethylpyrimidin-2-amine(Example 84);
*N*-(1-{5-[3-(4-Chlorophenyl)-3H-imidazo[4,5-c]pyridin-2-yl]pyridin-2-yl}piperidin-4-yl)acetamide (Example 85);
1-(4-{5-[3-(4-Fluorophenyl)-3H-imidazo[4,5-c]pyridin-2-yl]pyridin-2-yl}piperazin-1-yl)ethan-1-one
(Example 86);
1-(4-{5-[3-(4-Fluorophenyl)-3H-imidazo[4,5-c]pyridin-2-yl]pyridin-2-yl}-1,4-diazepan-1-yl)ethan-1-one; bis(trifluoroacetic acid) (Example 87);
*N*-(1-{5-[3-(4-Chlorophenyl)-3H-imidazo[4,5-c]pyridin-2-yl]pyridin-2-yl}piperidin-4-yl)methanesulfonamide
(Example 88);
1-{5-[3-(4-Fluorophenyl)-3H-imidazo[4,5-c]pyridin-2-yl]pyridin-2-yl}-4-methanesulfonylpiperazine
(Example 89);
4-{5-[3-(4-Chlorophenyl)-3H-imidazo[4,5-c]pyridin-2-yl]pyridin-2-yl}piperazine-1-carboxamide (Example 90);
(1-{5-[3-(4-Chlorophenyl)-3H-imidazo[4,5-c]pyridin-2-yl]pyridin-2-yl}piperidin-4-yl)urea (Example 91);
4-{5-[3-(4-Fluorophenyl)-3H-imidazo[4,5-c]pyridin-2-yl]pyridin-2-yl}piperazine-1-carboxamide (Example 92);
4-{5-[3-(4-Chlorophenyl)-3H-imidazo[4,5-c]pyridin-2-yl]-1,3-oxazol-2-yl}piperazine-1-carboxamide (Example 93);
4-{5-[3-(4-Fluorophenyl)-3H-imidazo[4,5-c]pyridin-2-yl]pyridin-2-yl}-1,4-diazepane-1-carboxamide (Example 94);
4-(5-{3-Phenyl-3H-imidazo[4,5-c]pyridin-2-yl}pyrimidin-2-yl)morpholine (Example 95);
4-{5-[3-(4-Cyclopropylphenyl)-3H-imidazo[4,5-c]pyridin-2-yl]pyrimidin-2-yl}morpholine (Example 96);
4-{4-Methyl-5-[3-(4-methylphenyl)-3H-imidazo[4,5-c]pyridin-2-yl]pyridin-2-yl}morpholine (Example 97);
4-{3-Fluoro-5-[3-(4-fluorophenyl)-3H-imidazo[4,5-c]pyridin-2-yl]pyridin-2-yl}morpholine (Example 98);
5-[3-(4-Fluorophenyl)-3H-imidazo[4,5-c]pyridin-2-yl]-2-(morpholin-4-yl)-1,4-dihydropyridin-4-one (Example 99);
5-[3-(4-Fluorophenyl)-3H-imidazo[4,5-c]pyridin-2-yl]-4-methyl-*N*-(oxan-4-yl)pyridin-2-amine (Example 100);
*N*-(Cyclopropylmethyl)-5-[3-(4-fluorophenyl)-3H-imidazo[4,5-c]pyridin-2-yl]-4-methylpyridin-2-amine (Example 101);
5-[3-(4-Fluorophenyl)-3H-imidazo[4,5-c]pyridin-2-yl]-4-methyl-2-(1H-pyrazol-1-yl)pyridine (Example 102);
(2R,6S)-2,6-Dimethyl-4-{5-[3-(6-methylpyridin-3-yl)-3H-imidazo[4,5-c]pyridin-2-yl]pyridin-2-yl}morpholine (Example 103);
(2R,6S)-2,6-Dimethyl-4-{5-[3-(5-methylpyridin-2-yl)-3H-imidazo[4,5-c]pyridin-2-yl]pyridin-2-yl}morpholine (Example 104);
5-[3-(4-Chloro-2-fluorophenyl)-3H-imidazo[4,5-c]pyridin-2-yl]pyridin-2-amine (Example 105);
4-{5-[3-(4-Fluorophenyl)-3H-imidazo[4,5-c]pyridin-2-yl]pyridin-2-yl}-1-methylpiperazin-2-one (Example 106);
4-{4-Methyl-5-[3-(6-methylpyridin-3-yl)-3H-imidazo[4,5-c]pyridin-2-yl]pyridin-2-yl}morpholine (Example 107);
4-{5-[3-(4-Fluorophenyl)-3H-imidazo[4,5-c]pyridin-2-yl]-6-methylpyridin-2-yl}morpholine (Example 108);
4-[3-(4-Fluorophenyl)-3H-imidazo[4,5-c]pyridin-2-yl]-*N*,*N*-dimethylpyridin-2-amine (Example 109);
5-[3-(4-Chlorophenyl)-3H-imidazo[4,5-c]pyridin-2-yl]pyridin-2-amine (Example 110);
5-[3-(4-Fluorophenyl)-3H-imidazo[4,5-c]pyridin-2-yl]-*N*,*N*,4-trimethylpyridin-2-amine (Example 111);
5-[3-(4-Fluorophenyl)-3H-imidazo[4,5-c]pyridin-2-yl]-2-(oxolan-3-yloxy)pyridine (Example 112);
5-[3-(4-Fluorophenyl)-3H-imidazo[4,5-c]pyridin-2-yl]-2-(oxan-4-yloxy)pyridine (Example 113);
4-[3-(4-Fluorophenyl)-3H-imidazo[4,5-c]pyridin-2-yl]-1-methyl-1,2-dihydropyridin-2-one (Example 114);
1-Cyclopropyl-4-[3-(4-fluorophenyl)-3H-imidazo[4,5-c]pyridin-2-yl]-1,2-dihydropyridin-2-one (Example 115);
4-[3-(4-Chloro-2-fluorophenyl)-3H-imidazo[4,5-c]pyridin-2-yl]-1-cyclopropyl-1,2-dihydropyridin-2-one (Example 116);
*N*-(2-Methoxyethyl)-N-methyl-5-[3-(4-methylphenyl)-3H-imidazo[4,5-c]pyridin-2-yl]pyrimidin-2-amine (Example 117);
(2R,6S)-2,6-Dimethyl-4-{5-[3-(6-methylpyridin-3-yl)-3H-imidazo[4,5-c]pyridin-2-yl]pyrimidin-2-yl}morpholine (Example 118);
5-[3-(4-Methylphenyl)-3H-imidazo[4,5-c]pyridin-2-yl]-*N*-(oxan-4-yl)pyrimidin-2-amine (Example 119);
and pharmaceutically acceptable salts thereof.

12. A pharmaceutical composition comprising a compound according to any one of claims 1 to 11, and one or more suitable excipients.

13. A compound according to any one of claims 1 to 11 for use in the treatment of inflammation, an inflammatory disease, an immune or an autoimmune disorder, or inhibition of tumour growth.

14. A compound for use according to claim 13 wherein the inflammation or inflammatory disease or immune or autoimmune disorder is arthritis including rheumatoid arthritis, juvenile rheumatoid arthritis, osteoarthritis and psoriatic arthritis, synovitis, vasculitis, Sjogren's disease, a condition associated with inflammation of the bowel including Crohn's disease, ulcerative colitis, inflammatory bowel disease and irritable bowel syndrome, atherosclerosis, multiple sclerosis, Alzheimer's disease, vascular dementia, Parkinson's disease, cerebral amyloid angiopathy, cerebral autosomal dominant arteriopathy with subcortical infarcts and leukoencephalopathy, a pulmonary inflammatory disease including asthma, chronic obstructive pulmonary disease and acute respiratory distress syndrome, a fibrotic disease including idiopathic pulmonary fibrosis, cardiac fibrosis, liver fibrosis and systemic sclerosis also known as scleroderma, an inflammatory disease of the skin including contact dermatitis, atopic dermatitis and psoriasis, an inflammatory disease of the eye including age related macular degeneration, uveitis and diabetic retinopathy, systemic inflammatory response syndrome, sepsis, an inflammatory and/or autoimmune condition of the liver including autoimmune hepatitis, primary biliary cirrhosis, alcoholic liver disease, sclerosing cholangitis, and autoimmune cholangitis, type I or II diabetes and/or the complications thereof, chronic heart failure, congestive heart failure, an ischemic disease including stroke and ischemia-reperfusion injury or myocardial infarction and/or the complications thereof, or epilepsy.

15. A compound for use according to claim 13 in the treatment of a disease selected from rheumatoid arthritis, osteoarthritis, liver fibrosis, chronic obstructive pulmonary disease, multiple sclerosis, Sjogren's disease, Alzheimer's disease, Parkinson's disease, inflammatory bowel disease, or vascular dementia.

## Patentansprüche

1. Verbindung der Formel (I) oder ein pharmazeutisch verträgliches Salz oder N-Oxid davon: 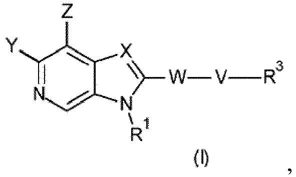 wobei:
**Y** aus Wasserstoff, Hydroxyl, -NH₂, -NH-C₁₋₄-Alkyl, -NH-Halogen-C₁₋₄-Alkyl oder -C₁₋₄-Alkoxy ausgewählt ist;
**Z** aus Folgenden ausgewählt ist: Wasserstoff, Halogen, Hydroxyl, Cyano, C₁₋₄-Alkyl, Halogen-C₁₋₄-Alkyl, C₁₋₄-Alkoxy, Halogen-C₁₋₄-Alkoxy, -CONH₂, -SO₂NH₂, -NH₂, -NH-C₁₋₄-Alkyl oder -NH-Halogen-C₁₋₄-Alkyl;
**R¹** ein Phenylring oder ein 5- oder 6-gliedriger Heteroarylring ist, wobei jeder Ring optional mit einem oder mehreren Substituenten substituiert ist, die aus Folgenden ausgewählt sind: Halogen, Cyano, C₁₋₄-Alkyl, Halogen-C₁₋₄-Alkyl, Cyano-C₁₋₄-Alkyl, einem 3-7-gliedrigen Cycloalkylring, -OR⁵, -NR⁶C(O)OR⁵, -NR⁶C(O)R⁵, -NR⁶C(O)NR^{4A}R^{4B}, -C(O)NR^{4A}R^{4B}, -C(O)R⁵, -C(O)OR⁵ und -NR⁶S(O)₂R⁵; wobei
R^{4A}, R^{4B} R⁵ und R⁶ jeweils unabhängig aus Wasserstoff, C₁₋₄-Alkyl oder Halogen-C₁₋₄-Alkyl ausgewählt sind oder
R^{4A} und R^{4B} zusammen mit dem Stickstoff, an den sie gebunden sind, eine 3-7-gliedrige cyclische Aminogruppe bilden, die optional durch einen oder mehrere Substituenten substituiert ist, die aus Folgenden ausgewählt sind: Halogen, Hydroxyl, Cyano, C₁₋₄-Alkyl, Halogen-C₁₋₄-Alkyl, C₁₋₄-Alkoxy, Halogen-C₁₋₄-Alkoxy, -CONH₂, -SO₂NH₂, -NH₂, -NH-C₁₋₄-Alkyl, -NH-Halogen-C₁₋₄-Alkyl;
**X** -N= ist;
**W** ein Phenylring oder ein 5- oder 6-gliedriger Heteroarylring ist, der aus Folgenden ausgewählt ist: Pyridinyl, Pyridazinyl, Pyrazinyl, Pyrimidinyl, Oxazolyl, Thiazolyl oder Imidazolyl, von denen jedweder Ring optional mit einem oder mehreren Substituenten substituiert ist, die aus Folgenden ausgewählt sind: Halogen, Cyano, Oxo, C₁₋₄-Alkyl, Halogen-C₁₋₄-Alkyl, Cyano-C₁₋₄-Alkyl, -OR⁵, -NR^{7A}R^{7B}, -NR⁶C(O)OR⁵, -NR⁶C(O)R⁵, -NR⁶C(O)NR^{7A}R^{7B}, -C(O)NR^{7A}R^{7B}, -C(O)R⁵, -C(O)OR⁵, -SO₂R⁵, -SO₂NR^{7A}R^{7B} und -NR⁶S(O)₂R⁵;
wobei R^{7A} und R^{7B} unabhängig Wasserstoff, C₁₋₄-Alkyl oder Halogen-C₁₋₄-Alkyl sind;
**V** aus einer Bindung, -O-, -N(R⁶)-, -(C=O)-, -CONR⁶-, -NR⁶C(O)- oder -C₁₋₄-Alkylen ausgewählt ist, wobei die C₁₋₄-Alkylengruppe optional durch Halogen substituiert ist und wobei jedwedes der Kohlenstoffatome der C₁₋₄-Alkylengruppe durch -O- oder -N(R⁶)- ersetzt sein kann;
**R³** aus Folgenden ausgewählt ist: Wasserstoff, -C₁₋₄-Alkyl, -C₁₋₄-Alkyl-C₁₋₄-Alkoxy oder einem 3-7-gliedrigen heterocyclischen Ring oder 3-7-gliedrigen Cycloalkylring oder einem 5- oder 6-gliedrigen Heteroarylring, wobei jedweder der Ringe optional mit einem oder mehreren Substituenten substituiert ist, die aus Folgenden ausgewählt sind: Halogen, Oxo, Hydroxyl, Cyano, C₁₋₄-Alkyl, Halogen-C₁₋₄-Alkyl, Cyano-C₁₋₄-Alkyl, -OR⁵, -NR^{4A}R^{4B}, -NR⁶C(O)OR⁵, -NR⁶C(O)R⁵, -NR⁶C(O)NR^{4A}R^{aB}, -C(O)NR^{4A}R^{4B}, -C(O)R⁵, -C(O)OR⁵, -SO₂R⁵, -SO₂NR^{4A}R^{4B} und -NR⁶S(O)₂R⁵;
MIT DER MAßGABE, DASS Gruppen -WVR³ und/oder R¹ nicht Folgendes sind: 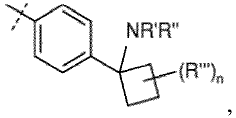 wobei
n 0, 1 oder 2 ist;
R' und R" unabhängig aus der Gruppe ausgewählt sind, die aus H, -C₁-C₆-Alkyl, -(C=O)-C₁-C₆-Alkyl und -(C=O)OC(CH₃)₃ besteht; und
R''' H, OH oder C₁-C₆-Alkyl ist.

2. Verbindung der Formel (I) oder ein pharmazeutisch verträgliches Salz oder N-Oxid davon: 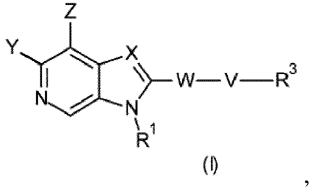 wobei:
**Y** aus Wasserstoff, Hydroxyl, -NH₂, -NH-C₁₋₄-Alkyl, -NH-Halogen-C₁₋₄-Alkyl oder -C₁₋₄-Alkoxy ausgewählt ist;
**Z** aus Folgenden ausgewählt ist: Wasserstoff, Halogen, Hydroxyl, Cyano, C₁₋₄-Alkyl, Halogen-C₁₋₄-Alkyl, C₁₋₄-Alkoxy, Halogen-C₁₋₄-Alkoxy, -CONH₂, -SO₂NH₂, -NH₂, -NH-C₁₋₄-Alkyl oder -NH-Halogen-C₁₋₄-Alkyl;
**R¹** ein Phenylring oder ein 5- oder 6-gliedriger Heteroarylring ist, wobei jeder Ring optional mit einem oder mehreren Substituenten substituiert ist, die aus Folgenden ausgewählt sind: Halogen, Cyano, C₁₋₄-Alkyl, Halogen-C₁₋₄-Alkyl, Cyano-C₁₋₄-Alkyl, -OR⁵, -NR⁶C(O)OR⁵, -NR⁶C(O)R⁵, -NR⁶C(O)NR^{4A}R^{aB}, -C(O)NR^{4A}R^{4B}, -C(O)R⁵, -C(O)OR⁵ und -NR⁶S(O)₂R⁵; wobei
R^{4A}, R^{4B} R⁵ und R⁶ jeweils unabhängig aus Wasserstoff, C₁₋₄-Alkyl oder Halogen-C₁₋₄-Alkyl ausgewählt sind oder
R^{4A} und R^{4B} zusammen mit dem Stickstoff, an den sie gebunden sind, eine 3-7-gliedrige cyclische Aminogruppe bilden, die optional durch einen oder mehrere Substituenten substituiert ist, die aus Folgenden ausgewählt sind: Halogen, Hydroxyl, Cyano, C₁₋₄-Alkyl, Halogen-C₁₋₄-Alkyl, C₁₋₄-Alkoxy, Halogen-C₁₋₄-Alkoxy, -CONH₂, -SO₂NH₂, -NH₂, -NH-C₁₋₄-Alkyl, -NH-Halogen-C₁₋₄-Alkyl;
**X** -N= ist;
**W** ein Phenylring oder ein 5- oder 6-gliedriger Heteroarylring ist, wobei jeder Ring optional mit einem oder mehreren Substituenten substituiert ist, die aus Folgenden ausgewählt sind: Halogen, Cyano, C₁₋₄-Alkyl, Halogen-C₁₋₄-Alkyl, Cyano-C₁₋₄-Alkyl, -OR⁵, -NR^{7A}R^{7B}, -NR⁶C(O)OR⁵, -NR⁶C(O)R⁵, -NR⁶C(O)NR^{7A}R^{7B}, -C(O)NR^{7A}R^{7B}, -C(O)R⁵, -C(O)OR⁵, -SO₂R⁵, -SO₂NR^{7A}R^{7B} und -NR⁶S(O)₂R⁵;
R^{7A} und R^{7B} unabhängig Wasserstoff, C₁₋₄-Alkyl oder Halogen-C₁₋₄-Alkyl sind;
**V** aus einer Bindung, -O-, -N(R⁶)-, -(C=O)-, -CONR⁶-, NR⁶C(O)- oder -C₁₋₄-Alkylen ausgewählt ist, wobei die C₁₋₄-Alkylengruppe optional durch Halogen substituiert ist und wobei jedwedes der Kohlenstoffatome der C₁₋₄-Alkylengruppe durch -O- oder -N(R⁶)- ersetzt sein kann;
**R³** Folgendes ist: Wasserstoff oder ein 3-7-gliedriger heterocyclischer Ring oder 3-7-gliedriger Cycloalkylring, der aus Cyclopropyl, Cyclopentyl oder Cyclohexyl ausgewählt ist, oder ein 5- oder 6-gliedriger Heteroarylring, wobei jedweder der Ringe optional mit einem oder mehreren Substituenten substituiert ist, die aus Folgenden ausgewählt sind: Halogen, Oxo, Hydroxyl, Cyano, C₁₋₄-Alkyl, Halogen-C₁₋₄-Alkyl, Cyano-C₁₋₄-Alkyl, -OR⁵, -NR^{4A}R^{4B}, -NR⁶C(O)OR⁵, -NR⁶C(O)R⁵, -NR⁶C(O)NR^{4A}R^{aB}, -C(O)NR^{4A}R^{4B}, -C(O)R⁵, -C(O)OR⁵, -SO₂R⁵, -SO₂NR^{4A}R^{4B} und -NR⁶S(O)₂R⁵.

3. Verbindung nach Anspruch 1 oder 2, wobei Y und Z beide Wasserstoff sind.

4. Verbindung nach einem der Ansprüche 1 bis 3, wobei R¹ Phenyl oder 6-gliedriges Heteroaryl ist, das optional mit einem oder mehreren Substituenten substituiert ist, die aus Halogen, C₁₋₄-Alkyl oder Halogen-C₁₋₄-Alkyl ausgewählt sind.

5. Verbindung nach einem der Ansprüche 1 bis 4, wobei W Folgendes ist:
(i) ein Phenylring, der optional mit einem oder mehreren Substituenten, wie in Anspruch 1 oder Anspruch 2 definiert, substituiert ist; oder
(ii) ein 6-gliedriger Heteroarylring, der aus Pyridin, Pyridazin, Pyrazin oder Pyrimidin ausgewählt ist, das optional mit einem oder mehreren Substituenten, wie in Anspruch 1 oder Anspruch 2 definiert, substituiert ist; oder
(iii) ein 5-gliedriger Heteroarylring, der aus Oxazol, Thiazol oder Imidazol ausgewählt ist, das optional mit einem oder mehreren Substituenten, wie in Anspruch 1 oder Anspruch 2 definiert, substituiert ist.

6. Verbindung nach einem der Ansprüche 1 bis 5, wobei V aus der Gruppe ausgewählt ist, die aus einer direkten Bindung, -CH₂-, -(CH₂)₂- oder -N(R⁶)CH₂- und -CH₂-N(R⁶)- besteht.

7. Verbindung nach Anspruch 1 oder einem der Ansprüche 3 bis 6, wenn abhängig von Anspruch 1, wobei R³ Cyclopropyl, Cyclobutyl, Cyclopentyl oder Cyclohexyl ist, das, wie in Anspruch 1 definiert, optional substituiert ist.

8. Verbindung nach einem der Ansprüche 1 bis 5, wobei R³ ein Ring ist, der mit -NR^{4A}R^{4B} substituiert ist, wobei sich R^{4A} und R^{4B} zusammen mit dem Stickstoffatom, an das sie gebunden sind, vereinen, um einen 4-7-gliedrigen heterocyclischen Ring zu bilden, der, wie in Anspruch 1 oder Anspruch 2 definiert, optional substituiert ist.

9. Verbindung nach einem der Ansprüche 1 bis 5, wobei R³ aus der Gruppe ausgewählt ist, die aus Folgenden besteht: 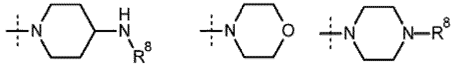 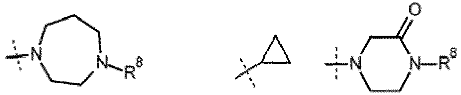 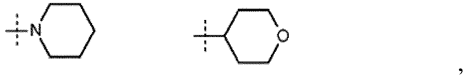 wobei R⁸ aus Wasserstoff, CH₃, -CONH₂, -NHCONH₂, -S(O)₂CH₃, -COCH₃ ausgewählt ist.

10. Verbindung nach Anspruch 1, wobei die Verbindung die Formel (Xa) aufweist: 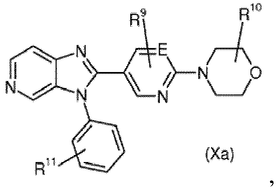
wobei E -C= oder -N= ist,
R⁹ und R¹⁰ jeweils unabhängig ein oder mehrere Substituenten sind, die aus Wasserstoff, Halogen, Cyano, Oxo, C₁₋₄-Alkyl, -O-C₁₋₄-Alkyl oder Halogen-C₁₋₄-Alkyl ausgewählt sind;
R¹¹ ein oder mehrere Substituenten ist, die aus Wasserstoff, Halogen, Cyano, Cyclopropyl, C₁₋₄-Alkyl oder Halogen-C₁₋₄-Alkyl ausgewählt sind.

11. Verbindung nach Anspruch 1 oder Anspruch 2, die aus Folgenden ausgewählt ist:
3-[3-(4-Chlorphenyl)-3H-imidazo[4,5-c]pyridin-2-yl]pyridin (Beispiel 1);
4-[3-(4-Chlorphenyl)-3H-imidazo[4,5-c]pyridin-2-yl]pyridin (Beispiel 2);
4-({5-[3-(4-Chlorphenyl)-3H-imidazo[4,5-c]pyridin-2-yl]pyridin-2-yl}methyl)morpholin (Beispiel 3);
4-{6-[3-(4-Chlorphenyl)-3H-imidazo[4,5-c]pyridin-2-yl]pyridazin-3-yl}morpholin (Beispiel 4);
4-{5-[3-(4-Chlorphenyl)-3H-imidazo[4,5-c]pyridin-2-yl]pyrazin-2-yl}morpholin (Beispiel 5);
4-({5-[3-(4-Chlorphenyl)-3H-imidazo[4,5-c]pyridin-2-yl]pyridin-2-yl}carbonyl)morpholin (Beispiel 6);
5-[3-(4-Chlorphenyl)-3H-imidazo[4,5-c]pyridin-2-yl]-*N*-(oxan-4-yl)pyrazin-2-amin (Beispiel 7);
1-{5-[3-(4-Chlorphenyl)-3H-imidazo[4,5-c]pyridin-2-yl]pyridin-2-yl}piperidin-4-amin (Beispiel 8);
*N*-(Cyclopropylmethyl)-5-[3-(4-fluorphenyl)-3H-imidazo[4,5-c]pyridin-2-yl]pyrimidin-2-amin (Beispiel 9);
*N*-Cyclopropyl-5-[3-(4-fluorphenyl)-3H-imidazo[4,5-c]pyridin-2-yl]pyrimidin-2-amin (Beispiel 10);
5-[3-(4-Chlorphenyl)-3H-imidazo[4,5-c]pyridin-2-yl]-*N*-(oxan-4-yl)pyrimidin-2-amin (Beispiel 11);
4-{5-[3-(4-Fluorphenyl)-3H-imidazo[4,5-c]pyridin-2-yl]pyrimidin-2-yl}piperazin-2-on (Beispiel 12);
4-{5-[3-(4-Chlorphenyl)-3H-imidazo[4,5-c]pyridin-2-yl]pyrimidin-2-yl}piperazin-2-on (Beispiel 13);
5-[3-(4-Chlorphenyl)-3H-imidazo[4,5-c]pyridin-2-yl]-*N*-cyclopropylpyridin-2-carboxamid (Beispiel 14);
3-[3-(4-Fluorphenyl)-3H-imidazo[4,5-c]pyridin-2-yl]-6-(oxan-4-yl)pyridazin (Beispiel 15);
*N*-{5-[3-(4-Chlorphenyl)-3H-imidazo[4,5-c]pyridin-2-yl]pyridin-2-yl}methansulfonamid (Beispiel 16);
1-{4-[3-(4-Chlorphenyl)-3H-imidazo[4,5-c]pyridin-2-yl]-1,3-thiazol-2-yl}piperazin (Beispiel 17);
1-{5-[3-(4-Chlorphenyl)-3H-imidazo[4,5-c]pyridin-2-yl]-1,3-oxazol-2-yl}piperazin (Beispiel 18);
1-{5-[3-(4-Chlorphenyl)-3H-imidazo[4,5-c]pyridin-2-yl]-1,3-thiazol-2-yl}piperazin (Beispiel 19);
5-[3-(4-Fluorphenyl)-3H-imidazo[4,5-c]pyridin-2-yl]-*N*-(oxan-4-yl)pyrimidin-2-amin (Beispiel 20);
4-{5-[3-(4-Fluorphenyl)-3H-imidazo[4,5-c]pyridin-2-yl]-4-methylpyridin-2-yl}morpholin (Beispiel 21);
4-{5-[3-(4-Chlor-2-fluorphenyl)-3H-imidazo[4,5-c]pyridin-2-yl]-4-methylpyridin-2-yl}morpholin (Beispiel 22);
(2R,6S)-4-{5-[3-(4-Fluorphenyl)-3H-imidazo[4,5-c]pyridin-2-yl]pyrimidin-2-yl}-2,6-dimethylmorpholin (Beispiel 23);
4-{5-[3-(4-Fluorphenyl)-3H-imidazo[4,5-c]pyridin-2-yl]pyrimidin-2-yl}-2,2-dimethylmorpholin (Beispiel 24);
4-{5-[3-(4-Fluorphenyl)-3H-imidazo[4,5-c]pyridin-2-yl]pyrimidin-2-yl}-1,4-oxazepan (Beispiel 25);
4-{5-[3-(4-Fluorphenyl)-3H-imidazo[4,5-c]pyridin-2-yl]-4-methylpyrimidin-2-yl}morpholin (Beispiel 26);
4-{5-[3-(4-Chlor-2-fluorphenyl)-3H-imidazo[4,5-c]pyridin-2-yl]-4-methylpyrimidin-2-yl}morpholin (Beispiel 27);
4-{5-[3-(4-Fluorphenyl)-3H-imidazo[4,5-c]pyridin-2-yl]-6-methoxypyridin-2-yl}morpholin (Beispiel 28);
4-{5-[3-(4-Fluorphenyl)-3H-imidazo[4,5-c]pyridin-2-yl]-4,6-dimethylpyridin-2-yl}morpholin (Beispiel 29);
2-Cyclopropyl-5-[3-(4-fluorphenyl)-3H-imidazo[4,5-c]pyridin-2-yl]pyrimidin (Beispiel 30);
5-[3-(4-Chlorphenyl)-3H-imidazo[4,5-c]pyridin-2-yl]pyrimidin-2-amin (Beispiel 31);
4-[3-(4-Chlorphenyl)-3H-imidazo[4,5-c]pyridin-2-yl]-1-methyl-1,2-dihydropyridin-2-on (Beispiel 32);
5-[3-(4-Chlorphenyl)-3H-imidazo[4,5-c]pyridin-2-yl]-1-methyl-1,2-dihydropyridin-2-on (Beispiel 33);
4-[3-(4-Chlor-2-fluorphenyl)-3H-imidazo[4,5-c]pyridin-2-yl]-1,2-dihydropyridin-2-on (Beispiel 34);
5-[3-(4-Chlor-2-fluorphenyl)-3H-imidazo[4,5-c]pyridin-2-yl]-1,2-dihydropyridin-2-on (Beispiel 35);
(2R,6S)-2,6-Dimethyl-4-{5-[3-(5-methylpyridin-2-yl)-3H-imidazo[4,5-c]pyridin-2-yl]pyrimidin-2-yl}morpholin (Beispiel 36);
*N*-(3-Methoxypropyl)-5-[3-(4-methylphenyl)-3H-imidazo[4,5-c]pyridin-2-yl]pyrimidin-2-amin (Beispiel 37);
5-[3-(4-Fluorphenyl)-3H-imidazo[4,5-c]pyridin-2-yl]-*N*-[2-(propan-2-yloxy)ethyl]pyrimidin-2-amin (Beispiel 38);
5-[3-(4-Methylphenyl)-3H-imidazo[4,5-c]pyridin-2-yl]-*N*-[2-(propan-2-yloxy)ethyl]pyrimidin-2-amin (Beispiel 39);
4-{5-[3-(4-Fluorphenyl)-3H-imidazo[4,5-c]pyridin-2-yl]pyrimidin-2-yl}-1-methylpiperazin-2-on (Beispiel 40);
4-{5-[3-(2,4-Difluorphenyl)-3H-imidazo[4,5-c]pyridin-2-yl]pyridin-2-yl}morpholin (Beispiel 41);
*N*-(2-Ethoxyethyl)-5-[3-(4-fluorphenyl)-3H-imidazo[4,5-c]pyridin-2-yl]pyrimidin-2-amin (Beispiel 42);
*N*-(2-Ethoxyethyl)-5-[3-(4-methylphenyl)-3H-imidazo[4,5-c]pyridin-2-yl]pyrimidin-2-amin (Beispiel 43);
5-[3-(4-Chlorphenyl)-3H-imidazo[4,5-c]pyridin-2-yl]-1H-imidazol (Beispiel 44);
1-({3-[3-(4-Chlorphenyl)-3H-imidazo[4,5-c]pyridin-2-yl]phenyl}methyl)-4-methylpiperazin (Beispiel 45);
1-({4-[3-(4-Chlorphenyl)-3H-imidazo[4,5-c]pyridin-2-yl]phenyl}methyl)-4-methylpiperazin (Beispiel 46);
4-{5-[3-(4-Chlorphenyl)-3H-imidazo[4,5-c]pyridin-2-yl]pyridin-2-yl}morpholin (Beispiel 47);
1-({4-[3-(4-Chlorphenyl)-3H-imidazo[4,5-c]pyridin-2-yl]phenyl}methyl)-1H-imidazol (Beispiel 48);
4-({4-[3-(4-Chlorphenyl)-3H-imidazo[4,5-c]pyridin-2-yl]phenyl}methyl)morpholin (Beispiel 49);
1-{5-[3-(4-Chlorphenyl)-3H-imidazo[4,5-c]pyridin-2-yl]pyridin-2-yl}piperazin (Beispiel 50);
4-{5-[3-(4-Chlorphenyl)-3H-imidazo[4,5-c]pyridin-2-yl]pyrimidin-2-yl}morpholin (Beispiel 51);
4-{5-[3-(4-Fluorphenyl)-3H-imidazo[4,5-c]pyridin-2-yl]pyrimidin-2-yl}morpholin (Beispiel 52);
4-{5-[3-(2-Fluor-4-methylphenyl)-3H-imidazo[4,5-c]pyridin-2-yl]pyrimidin-2-yl}morpholin (Beispiel 53);
4-{5-[3-(4-Fluorphenyl)-3H-imidazo[4,5-c]pyridin-2-yl]pyridin-2-yl}morpholin (Beispiel 54);
4-{5-[3-(4-Fluor-2-methylphenyl)-3H-imidazo[4,5-c]pyridin-2-yl]pyrimidin-2-yl}morpholin (Beispiel 55);
4-{5-[3-(2-Chlor-4-fluorphenyl)-3H-imidazo[4,5-c]pyridin-2-yl]pyrimidin-2-yl}morpholin (Beispiel 56);
4-{5-[3-(4-Methylphenyl)-3H-imidazo[4,5-c]pyridin-2-yl]pyrimidin-2-yl}morpholin (Beispiel 57);
4-{5-[3-(6-Methylpyridin-3-yl)-3H-imidazo[4,5-c]pyridin-2-yl]pyrimidin-2-yl}morpholin (Beispiel 58);
4-{5-[3-(4-Bromphenyl)-3H-imidazo[4,5-c]pyridin-2-yl]pyrimidin-2-yl}morpholin (Beispiel 59);
4-{5-[3-(2-Fluorphenyl)-3H-imidazo[4,5-c]pyridin-2-yl]pyrimidin-2-yl}morpholin (Beispiel 60);
4-{5-[3-(2-Chlor-4-fluorphenyl)-3H-imidazo[4,5-c]pyridin-2-yl]pyridin-2-yl}morpholin (Beispiel 61);
4-{5-[3-(4-Fluor-2-methylphenyl)-3H-imidazo[4,5-c]pyridin-2-yl]pyridin-2-yl}morpholin (Beispiel 62);
4-{5-[3-(4-Methylphenyl)-3H-imidazo[4,5-c]pyridin-2-yl]pyridin-2-yl}morpholin (Beispiel 63);
4-{5-[3-(6-Methylpyridin-3-yl)-3H-imidazo[4,5-c]pyridin-2-yl]pyridin-2-yl}morpholin (Beispiel 64);
4-{2-[6-(Morpholin-4-yl)pyridin-3-yl]-3H-imidazo[4,5-c]pyridin-3-yl}phenol (Beispiel 65);
4-(5-{3-[4-(Trifluormethyl)phenyl]-3H-imidazo[4,5-c]pyridin-2-yl}pyridin-2-yl)morpholin (Beispiel 66);
4-{5-[3-(2-Fluor-4-methylphenyl)-3H-imidazo[4,5-c]pyridin-2-yl]pyridin-2-yl}morpholin (Beispiel 67);
4-{5-[3-(2-Fluorphenyl)-3H-imidazo[4,5-c]pyridin-2-yl]pyridin-2-yl}morpholin (Beispiel 68);
5-[3-(4-Fluorphenyl)-3H-imidazo[4,5-c]pyridin-2-yl]-2-(pyrrolidin-1-yl)pyrimidin (Beispiel 69);
4-{5-[3-(4-Fluorphenyl)-3H-imidazo[4,5-c]pyridin-2-yl]pyridin-2-yl}-2-methylmorpholin (Beispiel 70);
5-[3-(4-Fluorphenyl)-3H-imidazo[4,5-c]pyridin-2-yl]-*N*,*N*-dimethylpyridin-2-amin (Beispiel 71);
5-[3-(4-Chlorphenyl)-3H-imidazo[4,5-c]pyridin-2-yl]-*N*,*N*-dimethylpyrimidin-2-amin (Beispiel 72);
4-{4-[3-(4-Fluorphenyl)-3H-imidazo[4,5-c]pyridin-2-yl]pyridin-2-yl}morpholin (Beispiel 73);
4-{5-[3-(4-Chlor-3-fluorphenyl)-3H-imidazo[4,5-c]pyridin-2-yl]pyrimidin-2-yl}morpholin (Beispiel 74);
4-{5-[3-(5-Chlorpyridin-2-yl)-3H-imidazo[4,5-c]pyridin-2-yl]pyrimidin-2-yl}morpholin (Beispiel 75);
4-{5-[3-(5-Fluorpyridin-2-yl)-3H-imidazo[4,5-c]pyridin-2-yl]pyrimidin-2-yl}morpholin (Beispiel 76);
4-{5-[3-(4-Chlor-2-fluorphenyl)-3H-imidazo[4,5-c]pyridin-2-yl]pyrimidin-2-yl}morpholin (Beispiel 77);
4-{5-[3-(2,4-Difluorphenyl)-3H-imidazo[4,5-c]pyridin-2-yl]pyrimidin-2-yl}morpholin (Beispiel 78);
4-{5-[3-(5-Methylpyridin-2-yl)-3H-imidazo[4,5-c]pyridin-2-yl]pyrimidin-2-yl}morpholin (Beispiel 79);
4-{5-[3-(4-Chlor-2-fluorphenyl)-3H-imidazo[4,5-c]pyridin-2-yl]pyridin-2-yl}morpholin (Beispiel 80);
4-{5-[3-(5-Chlorpyridin-2-yl)-3H-imidazo[4,5-c]pyridin-2-yl]pyridin-2-yl}morpholin (Beispiel 81);
4-{5-[3-(5-Methylpyridin-2-yl)-3H-imidazo[4,5-c]pyridin-2-yl]pyridin-2-yl}morpholin (Beispiel 82);
5-[3-(4-Chlor-2-fluorphenyl)-3H-imidazo[4,5-c]pyridin-2-yl]-2-(pyrrolidin-1-yl)pyrimidin (Beispiel 83);
5-[3-(4-Chlor-2-fluorphenyl)-3H-imidazo[4,5-c]pyridin-2-yl]-N,N-dimethylpyrimidin-2-amin (Beispiel 84);
*N*-(1-{5-[3-(4-Chlorphenyl)-3H-imidazo[4,5-c]pyridin-2-yl]pyridin-2-yl}piperidin-4-yl)acetamid (Beispiel 85);
1-(4-{5-[3-(4-Fluorphenyl)-3H-imidazo[4,5-c]pyridin-2-yl]pyridin-2-yl}piperazin-1-yl)ethan-1-on (Beispiel 86);
1-(4-{5-[3-(4-Fluorphenyl)-3H-imidazo[4,5-c]pyridin-2-yl]pyridin-2-yl}-1,4-diazepan-1-yl)ethan-1-on; Bis(trifluoressigsäure) (Beispiel 87);
*N*-(1-{5-[3-(4-Chlorphenyl)-3H-imidazo[4,5-c]pyridin-2-yl]pyridin-2-yl}piperidin-4-yl)methansulfonamid (Beispiel 88);
1-{5-[3-(4-Fluorphenyl)-3H-imidazo[4,5-c]pyridin-2-yl]pyridin-2-yl}-4-methansulfonylpiperazin (Beispiel 89);
4-{5-[3-(4-Chlorphenyl)-3H-imidazo[4,5-c]pyridin-2-yl]pyridin-2-yl}piperazin-1-carboxamid (Beispiel 90);
(1-{5-[3-(4-Chlorphenyl)-3H-imidazo[4,5-c]pyridin-2-yl]pyridin-2-yl}piperidin-4-yl)harnstoff (Beispiel 91);
4-{5-[3-(4-Fluorphenyl)-3H-imidazo[4,5-c]pyridin-2-yl]pyridin-2-yl}piperazin-1-carboxamid (Beispiel 92);
4-{5-[3-(4-Chlorphenyl)-3H-imidazo[4,5-c]pyridin-2-yl]-1,3-oxazol-2-yl}piperazin-1-carboxamid (Beispiel 93);
4-{5-[3-(4-Fluorphenyl)-3H-imidazo[4,5-c]pyridin-2-yl]pyridin-2-yl}-1,4-diazepan-1-carboxamid (Beispiel 94);
4-(5-{3-Phenyl-3H-imidazo[4,5-c]pyridin-2-yl}pyrimidin-2-yl)morpholin (Beispiel 95);
4-{5-[3-(4-Cyclopropylphenyl)-3H-imidazo[4,5-c]pyridin-2-yl]pyrimidin-2-yl}morpholin (Beispiel 96);
4-{4-Methyl-5-[3-(4-methylphenyl)-3H-imidazo[4,5-c]pyridin-2-yl]pyridin-2-yl}morpholin (Beispiel 97);
4-{3-Fluor-5-[3-(4-fluorphenyl)-3H-imidazo[4,5-c]pyridin-2-yl]pyridin-2-yl}morpholin (Beispiel 98);
5-[3-(4-Fluorphenyl)-3H-imidazo[4,5-c]pyridin-2-yl]-2-(morpholin-4-yl)-1,4-dihydropyridin-4-on (Beispiel 99);
5-[3-(4-Fluorphenyl)-3H-imidazo[4,5-c]pyridin-2-yl]-4-methyl-*N*-(oxan-4-yl)pyridin-2-amin (Beispiel 100);
*N*-(Cyclopropylmethyl)-5-[3-(4-fluorphenyl)-3H-imidazo[4,5-c]pyridin-2-yl]-4-methylpyridin-2-amin (Beispiel 101);
5-[3-(4-Fluorphenyl)-3H-imidazo[4,5-c]pyridin-2-yl]-4-methyl-2-(1H-pyrazol-1-yl)pyridin (Beispiel 102);
(2R,6S)-2,6-Dimethyl-4-{5-[3-(6-methylpyridin-3-yl)-3H-imidazo[4,5-c]pyridin-2-yl]pyridin-2-yl}morpholin (Beispiel 103);
(2R,6S)-2,6-Dimethyl-4-{5-[3-(5-methylpyridin-2-yl)-3H-imidazo[4,5-c]pyridin-2-yl]pyridin-2-yl}morpholin (Beispiel 104);
5-[3-(4-Chlor-2-fluorphenyl)-3H-imidazo[4,5-c]pyridin-2-yl]pyridin-2-amin (Beispiel 105);
4-{5-[3-(4-Fluorphenyl)-3H-imidazo[4,5-c]pyridin-2-yl]pyridin-2-yl}-1-methylpiperazin-2-on (Beispiel 106);
4-{4-Methyl-5-[3-(6-methylpyridin-3-yl)-3H-imidazo[4,5-c]pyridin-2-yl]pyridin-2-yl}morpholin (Beispiel 107);
4-{5-[3-(4-Fluorphenyl)-3H-imidazo[4,5-c]pyridin-2-yl]-6-methylpyridin-2-yl}morpholin (Beispiel 108);
4-[3-(4-Fluorphenyl)-3H-imidazo[4,5-c]pyridin-2-yl]-*N,N*-dimethylpyridin-2-amin (Beispiel 109);
5-[3-(4-Chlorphenyl)-3H-imidazo[4,5-c]pyridin-2-yl]pyridin-2-amin (Beispiel 110);
5-[3-(4-Fluorphenyl)-3H-imidazo[4,5-c]pyridin-2-yl]-*N,N*,4-trimethylpyridin-2-amin (Beispiel 111);
5-[3-(4-Fluorphenyl)-3H-imidazo[4,5-c]pyridin-2-yl]-2-(oxolan-3-yloxy)pyridin (Beispiel 112);
5-[3-(4-Fluorphenyl)-3H-imidazo[4,5-c]pyridin-2-yl]-2-(oxan-4-yloxy)pyridin (Beispiel 113);
4-[3-(4-Fluorphenyl)-3H-imidazo[4,5-c]pyridin-2-yl]-1-methyl-1,2-dihydropyridin-2-on (Beispiel 114);
1-Cyclopropyl-4-[3-(4-fluorphenyl)-3H-imidazo[4,5-c]pyridin-2-yl]-1,2-dihydropyridin-2-on (Beispiel 115);
4-[3-(4-Chlor-2-fluorphenyl)-3H-imidazo[4,5-c]pyridin-2-yl]-1-cyclopropyl-1,2-dihydropyridin-2-on (Beispiel 116);
*N*-(2-Methoxyethyl)-*N*-methyl-5-[3-(4-methylphenyl)-3H-imidazo[4,5-c]pyridin-2-yl]pyrimidin-2-amin (Beispiel 117);
(2R,6S)-2,6-Dimethyl-4-{5-[3-(6-methylpyridin-3-yl)-3H-imidazo[4,5-c]pyridin-2-yl]pyrimidin-2-yl}morpholin (Beispiel 118);
5-[3-(4-Methylphenyl)-3H-imidazo[4,5-c]pyridin-2-yl]-N-(oxan-4-yl)pyrimidin-2-amin (Beispiel 119);
und pharmazeutisch verträgliche Salze davon.

12. Pharmazeutische Zusammensetzung, die eine Verbindung nach einem der Ansprüche 1 bis 11 und einen oder mehrere geeignete Hilfsstoffe umfasst.

13. Verbindung nach einem der Ansprüche 1 bis 11 für die Verwendung bei der Behandlung von Entzündung, einer entzündlichen Erkrankung, einer Immun- oder einer Autoimmunstörung oder Hemmung von Tumorwachstum.

14. Verbindung für die Verwendung nach Anspruch 13, wobei die Entzündung oder entzündliche Erkrankung oder Immun- oder Autoimmunstörung Folgendes ist: Arthritis, einschließlich rheumatoider Arthritis, juveniler rheumatoider Arthritis, Osteoarthritis und psoriatischer Arthritis, Synovitis, Vaskulitis, Sjögren-Krankheit, ein Leiden, das mit Entzündung des Darms verbunden ist, einschließlich Crohn-Krankheit, Colitis ulcerosa, entzündlicher Darmerkrankung und Reizdarmsyndrom, Atherosklerose, Multiple Sklerose, Alzheimer-Krankheit, vaskuläre Demenz, Parkinson-Krankheit, zerebrale Amyloidangiopathie, zerebrale autosomal-dominante Arteriopathie mit subkortikalen Infarkten und Leukoenzephalopathie, eine entzündliche Lungenerkrankung, einschließlich Asthma, chronisch obstruktiver Lungenerkrankung und akuten Atemnotsyndroms, eine fibrotische Erkrankung, einschließlich idiopathischer pulmonaler Fibrose, kardialer Fibrose, Leberfibrose und systemischer Sklerose, anderweitig bekannt als Sklerodermie, eine entzündliche Erkrankung der Haut, einschließlich Kontaktdermatitis, atopischer Dermatitis und Psoriasis, eine entzündliche Erkrankung des Auges, einschließlich altersbedingter Makuladegeneration, Uveitis und diabetischer Retinopathie, systemisches inflammatorisches Response-Syndrom, Sepsis, ein entzündliches und/oder autoimmunes Leiden der Leber, einschließlich autoimmuner Hepatitis, primär biliärer Zirrhose, alkoholischer Lebererkrankung, sklerosierende Cholangitis und autoimmune Cholangitis, Typ I oder II Diabetes und/oder die Komplikationen davon, chronische Herzinsuffizienz, kongestive Herzinsuffizienz, eine ischämische Erkrankung, einschließlich Schlaganfall und Ischämie-Reperfusionsschäden oder Myokardinfarkt, und/oder die Komplikationen davon oder Epilepsie.

15. Verbindung für die Verwendung nach Anspruch 13 bei der Behandlung einer Erkrankung, die aus Folgenden ausgewählt ist: rheumatoider Arthritis, Osteoarthritis, Leberfibrose, chronisch obstruktiver Lungenerkrankung, Multipler Sklerose, Sjögren-Krankheit, Alzheimer-Krankheit, Parkinson-Krankheit, entzündlicher Darmerkrankung und vaskulärer Demenz.

## Revendications

1. Composé de la formule (I) ou un sel pharmaceutiquement acceptable, ou un N-oxyde de celui-ci : 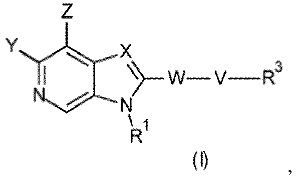 où :
Y est sélectionné parmi hydrogène, hydroxyle, -NH₂, -NH-alkyle C₁₋₄, -NH-halo-alkyle-C₁₋₄ ou -alcoxy-C₁₋₄ ;
Z est sélectionné parmi hydrogène, halogène, hydroxyle, cyano, alkyle-C₁₋₄, halo-alkyle-C₁₋₄, alcoxy-C₁₋₄, halo-alcoxy-C₁₋₄, -CONH₂, -SO₂NH₂, -NH₂, -NHalkyle-C₁₋₄ ou-NHhalo-alkyle-C₁₋₄ ;
R¹ est un cycle phényle, ou un cycle hétéroaryle à 5 ou 6 chaînons, l'un ou l'autre cycle étant optionnellement substitué par un ou plusieurs substituants sélectionnés parmi halogène, cyano, alkyle-C₁₋₄, halo-alkyle-C₁₋₄, cyano-alkyle-C₁₋₄, un cycle cycloalkyle à 3-7 chaînons, -OR⁵, -NR⁶C(O)OR⁵, -NR⁶C(O)R⁵, -NR⁶C(O)NR^{4A}R^{4B}, -C(O)NR^{4A}R^{4B},-C(O)R⁵, -C(O)OR⁵ et -NR⁶S(O)₂R⁵ ; où
R^{4A}, R^{4B} R⁵ et R⁶ sont sélectionnés chacun indépendamment parmi hydrogène, alkyle-C₁₋₄ ou halo-alkyle-C₁₋₄, ou bien
R^{4A} et R^{4B} avec l'azote auquel ils sont attachés forment un groupe amino cyclique à 3-7 chaînons, optionnellement substitué par un ou plusieurs substituants sélectionnés parmi : halogène, hydroxyle, cyano, alkyle-C₁₋₄, halo-alkyle-C₁₋₄, alcoxy-C₁₋₄, halo-alcoxy-C₁₋₄, -CONH₂, -SO₂NH₂, -NH₂, -NHalkyle-C₁₋₄, -NHhalo-alkyle-C₁₋₄ ;
X est -N= ;
W est un cycle phényle ou un cycle hétéroaryle à 5 ou 6 chaînons sélectionné parmi pyridinyle, pyridazinyle, pyrazinyle, pyrimidinyle, oxazolyle, thiazolyle ou imidazolyle, l'un quelconque desquels cycles étant optionnellement substitué par un ou plusieurs substituants sélectionnés parmi halogène, cyano, oxo, alkyle-C₁₋₄, halo-alkyle-C₁₋₄, cyano-alkyle-C₁₋₄, -OR⁵, -NR^{7A}R^{7B}, -NR⁶C(O)OR⁵, -NR⁶(O)R⁵, -NR⁶C(O)NR^{7A}R^{7B}, -C(O)NR^{7A}R^{7B}, -C(O)R⁵, -C(O)OR⁵, -SO₂R⁵, -SO₂NR^{7A}R^{7B} et -NR⁶S(O)₂R⁵ ;
R^{7A} et R^{7B} sont indépendamment un hydrogène, alkyle-C₁₋₄ ou halo-alkyle-C₁₋₄ ;
V est sélectionné parmi une liaison, -O-, -N(R⁶)-, -(C=O)-, -CONR⁶-, -NR⁶C(O)- ou -alkylène-C₁₋₄-, où le groupe alkylène-C₁₋₄ est optionnellement substitué par un halogène, et où l'un quelconque des atomes de carbone du groupe alkylène-C₁₋₄ peut être remplacé par -O- ou -N(R⁶)- ;
R³ est sélectionné parmi hydrogène, -alkyle-C₁₋₄, -alkyle-C₁₋₄-alcoxy-C₁₋₄ ou un cycle hétérocyclique à 3-7 chaînons ou un cycle cycloalkyle à 3-7 chaînons, ou un cycle hétéroaryle à 5 ou 6 chaînons, l'un quelconque des cycles étant optionnellement substitué par un ou plusieurs substituants sélectionnés parmi halogène, oxo, hydroxyle, cyano, alkyle-C₁₋₄, halo-alkyle-C₁₋₄, cyano-alkyle-C₁₋₄, -OR⁵, -NR^{4A}R^{4B}, -NR⁶C(O)OR⁵,-NR⁶C(O)R⁵, -NR⁶C(O)NR^{4A}R^{4B}, -C(O)NR^{4A}R^{4B}, -C(O)R⁵, -C(O)OR⁵, -SO₂R⁵,-SO₂NR^{4A}R^{4B} et -NR⁶S(O)₂R⁵ ;
A CONDITION QUE les groupes -WVR³ et/ou R¹ ne soient pas : 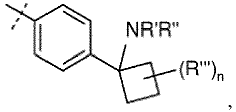 où
n est 0, 1 ou 2 ;
R' et R" sont sélectionnés indépendamment parmi le groupe consistant en H, -alkyle C₁-C₆, -(C=O)-alkyle C₁-C₆ et -(C=O)OC(CH₃)₃ ; et
R'" est H, OH ou alkyle C₁-C₆.

2. Composé de la formule (I) ou un sel pharmaceutiquement acceptable, ou un N-oxyde de celui-ci : 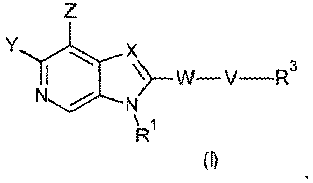 où :
Y est sélectionné parmi hydrogène, hydroxyle, -NH₂, -NH-alkyle C₁₋₄, -NH-halo-alkyle-C₁₋₄ ou -alcoxy-C₁₋₄ ;
Z est sélectionné parmi hydrogène, halogène, hydroxyle, cyano, alkyle-C₁₋₄, halo-alkyle-C₁₋₄, alcoxy-C₁₋₄, halo-alcoxy-C₁₋₄, -CONH₂, -SO₂NH₂, -NH₂, -NHalkyle-C₁₋₄ ou-NHhalo-alkyle-C₁₋₄ ;
R¹ est un cycle phényle, ou un cycle hétéroaryle à 5 ou 6 chaînons, l'un ou l'autre cycle étant optionnellement substitué par un ou plusieurs substituants sélectionnés parmi halogène, cyano, alkyle-C₁₋₄, halo-alkyle-C₁₋₄, cyano-alkyle-C₁₋₄, -OR⁵, -NR⁶C(O)OR⁵,-NR⁶C(O)R⁵, -NR⁶C(O)NR^{4A}R^{4B}, -C(O)NR^{4A}R^{4B}, -C(O)R⁵, -C(O)OR⁵ et -NR⁶S(O)₂R⁵ ; où
R^{4A}, R^{4B} R⁵ et R⁶ sont sélectionnés chacun indépendamment parmi hydrogène, alkyle-C₁₋₄ ou halo-alkyle-C₁₋₄, ou bien
R^{4A} et R^{4B} avec l'azote auquel ils sont attachés forment un groupe amino cyclique à 3-7 chaînons, optionnellement substitué par un ou plusieurs substituants sélectionnés parmi : halogène, hydroxyle, cyano, alkyle-C₁₋₄, halo-alkyle-C₁₋₄, alcoxy-C₁₋₄, halo-alcoxy-C₁₋₄, -CONH₂, -SO₂NH₂, -NH₂, -NHalkyle-C₁₋₄, -NHhalo-alkyle-C₁₋₄ ;
X est -N= ;
W est un cycle phényle ou un cycle hétéroaryle à 5 ou 6 chaînons, l'un ou l'autre cycle étant optionnellement substitué par un ou plusieurs substituants sélectionnés parmi halogène, cyano, alkyle-C₁₋₄, halo-alkyle-C₁₋₄, cyano-alkyle-C₁₋₄, -OR⁵, -NR^{7A}R^{7B},-NR⁶C(O)OR⁵, -NR⁶(O)R⁵, -NR⁶C(O)NR^{7A}R^{7B}, -C(O)NR^{7A}R^{7B}, -C(O)R⁵, -C(O)OR⁵,-SO₂R⁵, -SO₂NR^{7A}R^{7B} et -NR⁶S(O)₂R⁵ ;
R^{7A} et R^{7B} sont indépendamment un hydrogène, alkyle-C₁₋₄ ou halo-alkyle-C₁₋₄ ;
V est sélectionné parmi une liaison, -O-, -N(R⁶)-, -(C=O)-, -CONR⁶-, -NR⁶C(O)- ou -alkylène-C₁₋₄-, où le groupe alkylène-C₁₋₄ est optionnellement substitué par un halogène, et où l'un quelconque des atomes de carbone du groupe alkylène-C₁₋₄ peut être remplacé par -O- ou -N(R⁶)- ;
R³ est un hydrogène, ou un cycle hétérocyclique à 3-7 chaînons, ou un cycle cycloalkyle à 3-7 chaînons sélectionné parmi cyclopropyle, cyclopentyle ou cyclohexyle, ou un cycle hétéroaryle à 5 ou 6 chaînons, l'un quelconque des cycles étant optionnellement substitué par un ou plusieurs substituants sélectionnés parmi halogène, oxo, hydroxyle, cyano, alkyle-C₁₋₄, halo-alkyle-C₁₋₄, cyano-alkyle-C₁₋₄, -OR⁵, -NR^{4A}R^{4B},-NR⁶C(O)OR⁵, -NR⁶C(O)R⁵, -NR⁶C(O)NR^{4A}R^{4B}, -C(O)NR^{4A}R^{4B}, -C(O)R⁵, -C(O)OR⁵,-SO₂R⁵, -SO₂NR^{4A}R^{4B} et -NR⁶S(O)₂R⁵.

3. Composé selon la revendication 1 ou 2, dans lequel Y et Z sont tous les deux un hydrogène.

4. Composé selon l'une quelconque des revendications 1 à 3, dans lequel R¹ est un phényle ou un hétéroaryle à 6 chaînons, optionnellement substitué par un ou plusieurs substituants sélectionnés parmi halogène, alkyle-C₁₋₄ ou halo-alkyle-C₁₋₄.

5. Composé selon l'une quelconque des revendications 1 à 4 dans lequel W est :
(i) un cycle phényle optionnellement substitué par un ou plusieurs substituants comme il est défini dans la revendication 1 ou la revendication 2 ; ou bien
(ii) un cycle hétéroaryle à 6 chaînons sélectionné parmi pyridine, pyridazine, pyrazine ou pyrimidine optionnellement substitué par un ou plusieurs substituants comme il est défini dans la revendication 1 ou la revendication 2 ; ou bien
(iii) un cycle hétéroaryle à 5 chaînons sélectionné parmi oxazole, thiazole ou imidazole optionnellement substitué par un ou plusieurs substituants comme il est défini dans la revendication 1 ou la revendication 2.

6. Composé selon l'une quelconque des revendications 1 à 5, dans lequel V est sélectionné parmi le groupe consistant en une liaison directe, -CH₂-, -(CH₂)₂- ou-N(R⁶)CH₂- et -CH₂-N(R⁶)-.

7. Composé selon la revendication 1, ou selon l'une quelconque des revendications 3 à 6 lorsque dépendantes de la revendication 1, dans lequel R³ est un cyclopropyle, cyclobutyle, cyclopentyle ou cyclohexyle optionnellement substitué comme il est défini dans la revendication 1.

8. Composé selon l'une quelconque des revendications 1 à 5, dans lequel R³ est un cycle substitué par -NR^{4A}R^{4B} où R^{4A} et R^{4B}, avec l'atome d'azote auquel ils sont attachés, se joignent pour former un cycle hétérocyclique à 4-7 chaînons optionnellement substitué comme il est défini dans la revendication 1 ou dans la revendication 2.

9. Composé selon l'une quelconque des revendications 1 à 5, dans lequel R³ est sélectionné parmi le groupe consistant en : 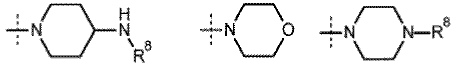 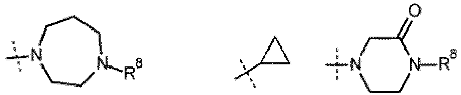 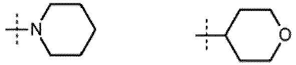 où R⁸ est sélectionné parmi hydrogène, CH₃, -CONH₂, -NHCONH₂, -S(O)₂CH₃, -COCH₃.

10. Composé selon la revendication 1, où le composé est de la formule (Xa) 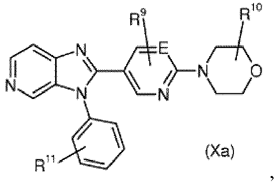
où E est -C= ou -N=,
R⁹ et R¹⁰ sont chacun indépendamment un ou plusieurs substituants sélectionnés parmi hydrogène, halogène, cyano, oxo, alkyle-C₁₋₄, -Oalkyle-C₁₋₄ ou halo-alkyle-C₁₋₄ ;
R¹¹ est un ou plusieurs substituants sélectionnés parmi hydrogène, halogène, cyano, cyclopropyle, alkyle-C₁₋₄ ou halo-alkyle-C₁₋₄.

11. Composé selon la revendication 1 ou la revendication 2 sélectionné parmi :
3-[3-(4-chlorophényl)-3H-imidazo[4,5-c]pyridin-2-yl]pyridine (Exemple 1) ;
4-[3-(4-chlorophényl)-3H-imidazo[4,5-c]pyridin-2-yl]pyridine (Exemple 2) ;
4-({5-[3-(4-chlorophényl)-3H-imidazo[4,5-c]pyridin-2-yl]pyridin-2-yl}méthyl)morpholine (Exemple 3) ;
4{6-[3-(4-chlorophényl)-3H-imidazo[4,5-c]pyridin-2-yl]pyridazin-3-yl}morpholine (Exemple 4) ;
4-{5-[3-(4-chlorophényl)-3H-imidazo[4,5-c]pyridin-2-yl]pyrazin-2-yl}morpholine (Exemple 5) ;
4-({5-[3-(4-chlorophényl)-3H-imidazo[4,5-c]pyridin-2-yl]pyridin-2-yl}carbonyl)morpholine (Exemple 6) ;
5-[3-(4-chlorophényl)-3H-imidazo[4,5-c]pyridin-2-yl]-*N*-(oxan-4-yl)pyrazin-2-amine (Exemple 7) ;
1-{5-[3-(4-chlorophényl)-3H-imidazo[4,5-c]pyridin-2-yl]pyridin-2-yl}pipéridin-4-amine (Exemple 8) ;
N-(cyclopropylméthyl)-5-[3-(4-fluorophényl)-3H-imidazo[4,5-c]pyridin-2-yl]pyrimidin-2-amine (Exemple 9) ;
*N*-cyclopropyl-5-[3-(4-fluorophényl)-3H-imidazo[4,5-c]pyridin-2-yl]pyrimidin-2-amine (Exemple 10) ;
5-[3-(4-chlorophényl)-3H-imidazo[4,5-c]pyridin-2-yl]-N-(oxan-4-yl)pyrimidin-2-amine (Exemple 11) ;
4-{5-[3-(4-fluorophényl)-3H-imidazo[4,5-c]pyridin-2-yl]pyrimidin-2-yl}pipérazin-2-one (Exemple 12) ;
4-{5-[3-(4-chlorophényl)-3H-imidazo[4,5-c]pyridin-2-yl]pyrimidin-2-yl}pipérazin-2-one (Exemple 13) ;
5-[3-(4-chlorophényl)-3H-imidazo[4,5-c]pyridin-2-yl]-*N*-cyclopropylpyridine-2-carboxamide (Exemple 14) ;
3-[3-(4-fluorophényl)-3H-imidazo[4,5-c]pyridin-2-yl]-6-(oxan-4-yl)pyridazine (Exemple 15) ;
*N*-{5-[3-(4-chlorophényl)-3H-imidazo[4,5-c]pyridin-2-yl]pyridin-2-yl}méthanesulfonamide (Exemple 16) ;
1-{4-[3-(4-chlorophényl)-3H-imidazo[4,5-c]pyridin-2-yl]-1,3-thiazol-2-yl}pipérazine (Exemple 17) ;
1-{5-[3-(4-chlorophényl)-3H-imidazo[4,5-c]pyridin-2-yl]-1,3-oxazol-2-yl}pipérazine (Exemple 18) ;
1-{5-[3-(4-chlorophényl)-3H-imidazo[4,5-c]pyridin-2-yl]-1,3-thiazol-2-yl}pipérazine (Exemple 19) ;
5-[3-(4-fluorophényl)-3H-imidazo[4,5-c]pyridin-2-yl]-*N*-(oxan-4-yl)pyrimidin-2-amine (Exemple 20) ;
4-{5-[3-(4-fluorophényl)-3H-imidazo[4,5-c]pyridin-2-yl]-4-méthylpyridin-2-yl}morpholine (Exemple 21) ;
4-{5-[3-(4-chloro-2-fluorophényl)-3H-imidazo[4,5-c]pyridin-2-yl]-4-méthylpyridin-2-yl}morpholine (Exemple 22) ;
(2R,6S)-4-{5-[3-(4-fluorophényl)-3H-imidazo[4,5-c]pyridin-2-yl]pyrimidin-2-yl}-2,6-diméthylmorpholine (Exemple 23) ;
4-{5-[3-(4-fluorophényl)-3H-imidazo[4,5-c]pyridin-2-yl]pyrimidin-2-yl}-2,2-diméthylmorpholine (Exemple 24) ;
4-{5-[3-(4-fluorophényl)-3H-imidazo[4,5-c]pyridin-2-yl]pyrimidin-2-yl}-1,4-oxazépane (Exemple 25) ;
4-{5-[3-(4-fluorophényl)-3H-imidazo[4,5-c]pyridin-2-yl]-4-méthylpyridin-2-yl}morpholine (Exemple 26) ;
4-{5-[3-(4-chloro-2-fluorophényl)-3H-imidazo[4,5-c]pyridin-2-yl]-4-méthylpyrimidin-2-yl} morpholine (Exemple 27) ;
4-{5-[3-(4-fluorophényl)-3H-imidazo[4,5-c]pyridin-2-yl]-4-méthoxypyridin-2-yl}morpholine (Exemple 28) ;
4-{5-[3-(4-fluorophényl)-3H-imidazo[4,5-c]pyridin-2-yl]-4,6-diméthylpyridin-2-yl}morpholine (Exemple 29) ;
2-cyclopropyl-5-[3-(4-fluorophényl)-3H-imidazo[4,5-c]pyridin-2-yl]pyrimidine (Exemple 30) ;
5-[3-(4-chlorophényl)-3H-imidazo[4,5-c]pyridin-2-yl]pyrimidin-2-amine (Exemple 31) ;
4-[3-(4-chlorophényl)-3H-imidazo[4,5-c]pyridin-2-yl]-1-méthyl-1,2-dihydropyridin-2-one (Exemple 32) ;
5-[3-(4-chlorophényl)-3H-imidazo[4,5-c]pyridin-2-yl]-1-méthyl-1,2-dihydropyridin-2-one (Exemple 33) ;
4-[3-(4-chloro-2-fluorophényl)-3H-imidazo[4,5-c]pyridin-2-yl]-1,2-dihydropyridin-2-one (Exemple 34) ;
5-[3-(4-chloro-2-fluorophényl)-3H-imidazo[4,5-c]pyridin-2-yl]-1,2-dihydropyridin-2-one (Exemple 35) ;
(2R,6S)-2,6-diméthyl-4-{5-[3-(5-méthylpyridin-2-yl)-3H-imidazo[4,5-c]pyridin-2-yl]pyrimidin-2-yl}morpholine (Exemple 36) ;
*N*-(3-méthoxypropyl)-5-[3-(4-méthylphényl)-3H-imidazo[4,5-c]pyridin-2-yl]pyrimidin-2-amine (Exemple 37) ;
5-[3-(4-fluorophényl)-3H-imidazo[4,5-c]pyridin-2-yl]-*N*-[2-(propan-2-yloxy)éthyl]pyrimidin-2-amine (Exemple 38) ;
5-[3-(4-méthylphényl)-3H-imidazo[4,5-c]pyridin-2-yl]-N-[2-(propan-2-yloxy)éthyl]pyrimidin-2-amine (Exemple 39) ;
4-{5-[3-(4-fluorophényl)-3H-imidazo[4,5-c]pyridin-2-yl]pyrimidin-2-yl}-1-méthylpipérazin-2-one (Exemple 40) ;
4-{5-[3-(2,4-difluorophényl)-3H-imidazo[4,5-c]pyridin-2-yl]pyridin-2-yl}morpholine (Exemple 41) ;
*N*-(2-éthoxyéthyl)-5-[3-(4-fluorophényl)-3H-imidazo[4,5-c]pyridin-2-yl]pyrimidin-2-amine (Exemple 42) ;
*N*-(2-éthoxyéthyl)-5-[3-(4-méthylphényl)-3H-imidazo[4,5-c]pyridin-2-yl]pyrimidin-2-amine (Exemple 43) ;
5-[3-(4-chlorophényl)-3H-imidazo[4,5-c]pyridin-2-yl]-1H-imidazole (Exemple 44) ;
1({3-[3-(4-chlorophényl)-3H-imidazo[4,5-c]pyridin-2-yl]phényl}méthyl)-4-méthylpipérazine (Exemple 45) ;
1({4-[3-(4-chlorophényl)-3H-imidazo[4,5-c]pyridin-2-yl]phényl}méthyl)-4-méthylpipérazine (Exemple 46) ;
4-{5-[3-(2,4-chlorophényl)-3H-imidazo[4,5-c]pyridin-2-yl]pyridin-2-yl}morpholine (Exemple 47) ;
1({4-[3-(4-chlorophényl)-3H-imidazo[4,5-c]pyridin-2-yl]phényl}méthyl)-1H-imidazole (Exemple 48) ;
4({4-[3-(4-chlorophényl)-3H-imidazo[4,5-c]pyridin-2-yl]phényl}méthyl)morpholine (Exemple 49) ;
1-{5-[3-(4-chlorophényl)-3H-imidazo[4,5-c]pyridin-2-yl]pyridin-2-yl}pipérazine (Exemple 50) ;
4-{5-[3-(2,4-chlorophényl)-3H-imidazo[4,5-c]pyridin-2-yl]pyrimidin-2-yl}morpholine (Exemple 51) ;
4-{5-[3-(2,4-fluorophényl)-3H-imidazo[4,5-c]pyridin-2-yl]pyrimidin-2-yl}morpholine (Exemple 52) ;
4-{5-[3-(2-fluoro-4-méthylphényl)-3H-imidazo[4,5-c]pyridin-2-yl]pyrimidin-2-yl}morpholine (Exemple 53) ;
4-{5-[3-(4-fluorophényl)-3H-imidazo[4,5-c]pyridin-2-yl]pyridin-2-yl}morpholine (Exemple 54) ;
4-{5-[3-(4-fluoro-2-méthylphényl)-3H-imidazo[4,5-c]pyridin-2-yl]pyrimidin-2-yl}morpholine (Exemple 55) ;
4-{5-[3-(2-chloro-4-fluorophényl)-3H-imidazo[4,5-c]pyridin-2-yl]pyrimidin-2-yl}morpholine (Exemple 56) ;
4-{5-[3-(4-méthylphényl)-3H-imidazo[4,5-c]pyridin-2-yl]pyrimidin-2-yl}morpholine (Exemple 57) ;
4-{5-[3-(6-méthylpyridin-3-yl)-3H-imidazo[4,5-c]pyridin-2-yl]pyrimidin-2-yl}morpholine (Exemple 58) ;
4-{5-[3-(4-bromophényl)-3H-imidazo[4,5-c]pyridin-2-yl]pyrimidin-2-yl}morpholine (Exemple 59) ;
4-{5-[3-(2-fluorophényl)-3H-imidazo[4,5-c]pyridin-2-yl]pyrimidin-2-yl}morpholine (Exemple 60) ;
4-{5-[3-(2-chloro-4-fluorophényl)-3H-imidazo[4,5-c]pyridin-2-yl]pyridin-2-yl} morpholine (Exemple 61) ;
4-{5-[3-(4-fluoro-2-méthylphényl)-3H-imidazo[4,5-c]pyridin-2-yl]pyridin-2-yl}morpholine (Exemple 62) ;
4-{5-[3-(4-méthylphényl)-3H-imidazo[4,5-c]pyridin-2-yl]pyridin-2-yl}morpholine (Exemple 63) ;
4-{5-[3-(6-méthylpyridin-3-yl)-3H-imidazo[4,5-c]pyridin-2-yl]pyridin-2-yl}morpholine (Exemple 64) ;
4-{2-[6-(morpholin-4-yl)pyridin-3-yl]-3H-imidazo[4,5-c]pyridin-3-yl}phénol (Exemple 65) ;
4-(5-{3-[4-(trifluorométhyl)phényl]-3H-imidazo[4,5-c]pyridin-2-yl}pyridin-2-yl)morpholine (Exemple 66) ;
4-{5-[3-(2-fluoro-4-méthylphényl)-3H-imidazo[4,5-c]pyridin-2-yl]pyridin-2-yl} morpholine (Exemple 67) ;
4-{5-[3-(2-fluorophényl)-3H-imidazo[4,5-c]pyridin-2-yl]pyridin-2-yl}morpholine (Exemple 68) ;
5-[3-(4-fluorophényl)-3H-imidazo[4,5-c]pyridin-2-yl]-2-(pyrrolidin-1-yl)pyrimidine (Exemple 69) ;
4-{5-[3-(4-fluorophényl)-3H-imidazo[4,5-c]pyridin-2-yl]pyridin-2-yl}méthylmorpholine (Exemple 70) ;
5-[3-(4-fluorophényl)-3H-imidazo[4,5-c]pyridin-2-yl]-*N,N*-diméthylpyridin-2-amine (Exemple 71) ;
5-[3-(4-chlorophényl)-3H-imidazo[4,5-c]pyridin-2-yl]-*N,N*-diméthylpyrimidin-2-amine (Exemple 72) ;
4-{4-[3-(4-fluorophényl)-3H-imidazo[4,5-c]pyridin-2-yl]pyridin-2-yl}morpholine (Exemple 73) ;
4-{5-[3-(4-chloro-3-fluorophényl)-3H-imidazo[4,5-c]pyridin-2-yl]pyrimidin-2-yl}morpholine (Exemple 74) ;
4-{5-[3-(4-chloropyridin-2-yl)-3H-imidazo[4,5-c]pyridin-2-yl]pyrimidin-2-yl}morpholine (Exemple 75) ;
4-{5-[3-(5-fluoropyridin-2-yl)-3H-imidazo[4,5-c]pyridin-2-yl]pyrimidin-2-yl}morpholine (Exemple 76) ;
4-{5-[3-(4-chloro-2-fluorophényl)-3H-imidazo[4,5-c]pyridin-2-yl]pyrimidin-2-yl}morpholine (Exemple 77) ;
4-{5-[3-(2,4-difluorophényl)-3H-imidazo[4,5-c]pyridin-2-yl]pyrimidin-2-yl}morpholine (Exemple 78) ;
4-{5-[3-(5-méthylpyridin-2-yl)-3H-imidazo[4,5-c]pyridin-2-yl]pyrimidin-2-yl}morpholine (Exemple 79) ;
4-{5-[3-(4-chloro-2-fluorophényl)-3H-imidazo[4,5-c]pyridin-2-yl]pyridin-2-yl}morpholine (Exemple 80) ;
4-{5-[3-(4-chloropyridin-2-yl)-3H-imidazo[4,5-c]pyridin-2-yl]pyridin-2-yl}morpholine (Exemple 81) ;
4-{5-[3-(5-méthylpyridin-2-yl)-3H-imidazo[4,5-c]pyridin-2-yl]pyridin-2-yl}morpholine (Exemple 82) ;
5-[3-(4-chloro-2-fluorophényl)-3H-imidazo[4,5-c]pyridin-2-yl]-2-(pyrrolidin-1-yl)pyrimidine (Exemple 83) ;
5-[3-(4-chloro-2-fluorophényl)-3H-imidazo[4,5-c]pyridin-2-yl]-*N,N*-diméthylpyrimidin-2-amine (Exemple 84) ;
*N*-(1-{5-[3-(4-chlorophényl)-3H-imidazo[4,5-c]pyridin-2-yl]pyridin-2-yl}pipéridin-4-yl)acétamide (Exemple 85) ;
1-(4-{5-[3-(4-fluorophényl)-3H-imidazo[4,5-c]pyridin-2-yl]pyridin-2-yl}pipérazin-1-yl)éthan-1-one (Exemple 86) ;
1-(4-{5-[3-(4-fluorophényl)-3H-imidazo[4,5-c]pyridin-2-yl]pyridin-2-yl}-1,4-diazépan-1-yl)éthan-1-one ; acide bis-trifluoroacétique (Exemple 87) ;
*N*-(1-{5-[3-(4-chlorophényl)-3H-imidazo[4,5-c]pyridin-2-yl]pyridin-2-yl}pipéridin-4-yl)méthanesulfonamide (Exemple 88) ;
1-{5-[3-(4-fluorophényl)-3H-imidazo[4,5-c]pyridin-2-yl]pyridin-2-yl}-4-méthanesulfonylpipérazine (Exemple 89) ;
4-{5-[3-(4-chlorophényl)-3H-imidazo[4,5-c]pyridin-2-yl]pyridin-2-yl}pipérazine-1-carboxamide (Exemple 90) ;
(1-{5-[3-(4-chlorophényl)-3H-imidazo[4,5-c]pyridin-2-yl]pyridin-2-yl}pipéridin-4-yl)urée (Exemple 91) ;
4-{5-[3-(4-fluorophényl)-3H-imidazo[4,5-c]pyridin-2-yl]pyridin-2-yl}pipérazine-1-carboxamide (Exemple 92) ;
4-{5-[3-(4-chlorophényl)-3H-imidazo[4,5-c]pyridin-2-yl]-1,3-oxazol-2-yl}pipérazine-1-carboxamide (Exemple 93) ;
4-{5-[3-(4-fluorophényl)-3H-imidazo[4,5-c]pyridin-2-yl]pyridin-2-yl}-1,4-diazépane-1-carboxamide (Exemple 94) ;
4-(5-{3-phényl-3H-imidazo[4,5-c]pyridin-2-yl}pyrimidin-2-yl)morpholine (Exemple 95) ;
4-{5-[3-(4-cyclopropylphényl)-3H-imidazo[4,5-c]pyridin-2-yl]pyrimidin-2-yl}morpholine (Exemple 96) ;
4-{4-méthyl-5-[3-(4-méthylphényl)-3H-imidazo[4,5-c]pyridin-2-yl]pyridin-2-yl}morpholine (Exemple 97) ;
4-{3-fluoro-5-[3-(4-fluorophényl)-3H-imidazo[4,5-c]pyridin-2-yl]pyridin-2-yl}morpholine (Exemple 98) ;
5-[3-(4-fluorophényl)-3H-imidazo[4,5-c]pyridin-2-yl]-2-(morpholin-4-yl)-1,4-dihydropyridin-4-one (Exemple 99) ;
5-[3-(4-fluorophényl)-3H-imidazo[4,5-c]pyridin-2-yl]-4-méthyl-*N*-(oxan-4-yl)pyridin-2-amine (Exemple 100) ;
*N*-(cyclopropylméthyl)-5-[3-(4-fluorophényl)-3H-imidazo[4,5-c]pyridin-2-yl]-4-méthylpyridin-2-amine (Exemple 101) ;
5-[3-(4-fluorophényl)-3H-imidazo[4,5-c]pyridin-2-yl]-4-méthyl-2-(1H-pyrazol-1-yl)pyridine (Exemple 102) ;
(2R,6S)-2,6-diméthyl-4-{5-[3-(6-méthylpyridin-3-yl)-3H-imidazo[4,5-c]pyridin-2-yl]pyridin-2-yl}morpholine (Exemple 103) ;
(2R,6S)-2,6-diméthyl-4-{5-[3-(5-méthylpyridin-2-yl)-3H-imidazo[4,5-c]pyridin-2-yl]pyridin-2-yl}morpholine (Exemple 104) ;
5-[3-(4-chloro-2-fluorophényl)-3H-imidazo[4,5-c]pyridin-2-yl]pyridin-2-amine (Exemple 105) ;
4-{5-[3-(4-fluorophényl)-3H-imidazo[4,5-c]pyridin-2-yl]pyridin-2-yl}-1-méthylpipérazin-2-one (Exemple 106) ;
4-{4-méthyl-5-[3-(6-méthylpyridin-3-yl)-3H-imidazo[4,5-c]pyridin-2-yl]pyridin-2-yl}morpholine (Exemple 107) ;
4-{5-[3-(4-fluorophényl)-3H-imidazo[4,5-c]pyridin-2-yl]-6-méthylpyridin-2-yl}morpholine (Exemple 108) ;
4-[3-(4-fluorophényl)-3H-imidazo[4,5-c]pyridin-2-yl]-*N,N*-diméthylpyridin-2-amine (Exemple 109) ;
5-[3-(4-chlorophényl)-3H-imidazo[4,5-c]pyridin-2-yl]pyridin-2-amine (Exemple 110) ;
5-[3-(4-fluorophényl)-3H-imidazo[4,5-c]pyridin-2-yl]-*N,N*-triméthylpyridin-2-amine (Exemple 111) ;
5-[3-(4-fluorophényl)-3H-imidazo[4,5-c]pyridin-2-yl]-2-(oxolan-3-yloxy)pyridine (Exemple 112) ;
5-[3-(4-fluorophényl)-3H-imidazo[4,5-c]pyridin-2-yl]-2-(oxan-4-yloxy)pyridine (Exemple 113) ;
4-[3-(4-fluorophényl)-3H-imidazo[4,5-c]pyridin-2-yl]-1-méthyl-1,2-dihydropyridin-2-one (Exemple 114) ;
1-cyclopropyl-4-[3-(4-fluorophényl)-3H-imidazo[4,5-c]pyridin-2-yl]-1,2-dihydropyridin-2-one (Exemple 115) ;
4-[3-(4-chloro-2-fluorophényl)-3H-imidazo[4,5-c]pyridin-2-yl]-1-cyclopropyl-1,2-dihydropyridin-2-one (Exemple 116) ;
*N*-(2-méthoxyéthyl)-*N*-méthyl-5-[3-(4-méthylphényl)-3H-imidazo[4,5-c]pyridin-2-yl]pyrimidin-2-amine (Exemple 117) ;
(2R,6S)-2,6-diméthyl-4-{5-[3-(6-méthylpyridin-3-yl)-3H-imidazo[4,5-c]pyridin-2-yl]pyrimidin-2-yl}morpholine (Exemple 118) ;
5-[3-(4-méthylphényl)-3H-imidazo[4,5-c]pyridin-2-yl]-N-(oxan-4-yl)pyridimidin-2-amine (Exemple 119) ;
ou un sel pharmaceutiquement acceptable de ceux-ci.

12. Composition pharmaceutique comprenant un composé selon l'une quelconque des revendications 1 à 11, et un ou plusieurs excipients appropriés.

13. Composé selon l'une quelconque des revendications 1 à 11 à utiliser dans le traitement d'une inflammation, d'une maladie inflammatoire, d'un trouble immun ou auto-immun, ou dans l'inhibition d'une croissance tumorale.

14. Composé à utiliser selon la revendication 13, où l'inflammation ou la maladie inflammatoire ou le trouble immun ou auto-immun est l'arthrite y compris la polyarthrite rhumatoïde, la polyarthrite rhumatoïde juvénile, l'ostéoarthrite et l'arthrite psoriasique, la synovite, la vascularite, le syndrome de Sjögren, une condition associée à une inflammation de l'intestin y compris la maladie de Crohn, la colite ulcéreuse, la maladie inflammatoire de l'intestin et le syndrome de l'intestin irritable, l'athérosclérose, la sclérose en plaques, la maladie d'Alzheimer, la démence vasculaire, la maladie de Parkinson, une angiopathie amyloïde cérébrale, une artériopathie cérébrale autosomique dominante avec infarctus sous-corticaux et leucoencéphalopathie, une maladie pulmonaire inflammatoire y compris l'asthme, une maladie pulmonaire obstructive chronique et le syndrome de détresse respiratoire aiguë, une maladie de type fibrose y compris la fibrose pulmonaire idiopathique, la fibrose cardiaque, la fibrose du foie et la sclérose systémique appelée aussi sclérodermie, une maladie inflammatoire de la peau y compris la dermatite de contact, la dermatite atopique et le psoriasis, une maladie inflammatoire des yeux y compris une dégénérescence maculaire liée à l'âge, l'uvéite et une rétinopathie diabétique, le syndrome de réponse inflammatoire systémique, le sepsis, une condition inflammatoire et/ou auto-immune du foie y compris l'hépatite auto-immune, la cirrhose biliaire primitive, une maladie hépatique alcoolique, la cholangite sclérosante et la cholangite auto-immune, le diabète de type I ou II et/ou ses complications, une défaillance cardiaque chronique, une défaillance cardiaque congestive, une maladie ischémique y compris un accident vasculaire cérébral et une lésion d'ischémie-reperfusion ou un infarctus du myocarde et/ou ses complications, ou l'épilepsie.

15. Composé à utiliser selon la revendication 13 dans le traitement d'une maladie sélectionnée parmi la polyarthrite rhumatoïde, l'ostéoarthrite, une fibrose du foie, une maladie pulmonaire obstructive chronique, la sclérose en plaques, la maladie de Sjögren, la maladie d'Alzheimer, la maladie de Parkinson, une maladie inflammatoire de l'intestin ou la démence vasculaire.
